# EUROPÄISCHE PATENTSCHRIFT

(11) **EP 3 577 114 B1**
(45) Veröffentlichungstag und Bekanntmachung des Hinweises auf die Patenterteilung: **10.11.2021**
(21) Anmeldenummer: 18704201.5
(22) Anmeldetag: 05.02.2018
(51) Int. Cl.: C07D 471/04

(54) **VERFAHREN ZUR HERSTELLUNG HALOGENIERTER IMIDAZOPYRIDINDERIVATE**
METHOD FOR THE PREPARATION OF HALOGENATED IMIDAZOPYRIDINE DERIVATIVES
PROCÉDÉ DE FABRICATION DE DÉRIVÉS D'IMIDAZOPYRIDINE HALOGÉNÉE

(30) Priorität: 06.02.2017 EP 17154787
(43) Veröffentlichungstag der Anmeldung: 11.12.2019
(73) Patentinhaber: Bayer Aktiengesellschaft, 51373 Leverkusen (DE); Bayer CropScience Aktiengesellschaft, 40789 Monheim am Rhein (DE)
(72) Erfinder: MOSRIN, Marc, 50935 Köln (DE); FISCHER, Rüdiger, 50259 Pulheim (DE); HAGER, Dominik, 40789 Monheim (DE); HOFFMEISTER, Laura, 40593 Düsseldorf (DE); KAUSCH-BUSIES, Nina, 51467 Bergisch Gladbach (DE); WILCKE, David, 40235 Düsseldorf (DE); WILLOT, Matthieu, 40215 Düsseldorf (DE); ILG, Kerstin, 50670 Köln (DE)
(74) Vertreter: BIP Patents
(86) Internationale Anmeldenummer: PCT/EP2018/052784
(87) Internationale Veröffentlichungsnummer: WO 2018/141955

(56) Entgegenhaltungen:
- WO-A1-2015/121136
- WO-A1-2016/046071
- WO-A1-2016/058928
- MARC MOSRIN ET AL: "TMPZnCl.LiCl: A New Active Selective Base for the Directed Zincation of Sensitive Aromatics and Heteroaromatics", ORGANIC LETTERS, Bd. 11, Nr. 8, 16. April 2009 (2009-04-16) , Seiten 1837-1840, XP055019579, ISSN: 1523-7060, DOI: 10.1021/ol900342a

## Beschreibung

Die vorliegende Erfindung betrifft ein Verfahren zur Herstellung von halogenierten Imidazopyridinderivaten der Formel (II) 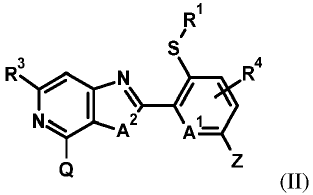 ausgehend von Verbindungen der Formel (I) 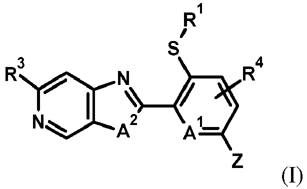 über Zwischenstufen der Formel (IVa) oder (IVb) 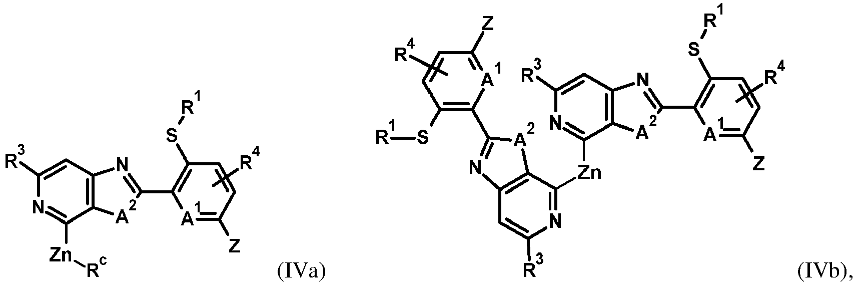 in denen die in den Formeln (I), (II), (IVa) und (IVb) angegebenen Strukturelemente die nachstehend angegebenen Bedeutungen haben.

Ferner betrifft die Erfindung derartige halogenierte Imidazopyridinderivate, sowie Zwischenstufen und die Umsetzungsprodukte von Verbindungen der Formel (II) zu Verbindungen der Formel (III) 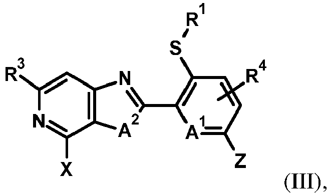 in welcher die angegebenen Strukturelemente die nachstehend angegebenen Bedeutungen haben.

Halogenierte Imidazopyyridinderivate der Formel (II) haben große technische Bedeutung für die pharmazeutische und die agrochemische Industrie und sind beispielsweise wichtige Zwischenprodukte unter anderem bei der Herstellung von Verbindungen, die beispielsweise als Pestizide wirksam sind.

Imidazopyridine zur Verwendung als Pestizide und Verfahren zu deren Herstellung sind beispielsweise beschrieben in WO 2010/125985, WO 2012/074135, WO 2012/086848, WO 2013/018928, WO 2013/191113, WO 2015/000715, WO 2016/124563, WO 2016/124557, WO 2015/121136, WO 2015/133603, WO 2015/198859, WO 2015/002211, WO 2015/071180, WO 2016/023954, WO 2016/020286, WO 2016/046071, WO 2016/058928; WO 2016/116338, WO 2016/107831, WO 2016/129684, WO 2017/055185, WO 2017/068599 und WO 2016/125621. Im Stand der Technik werden jedoch keine konkreten Verbindungen der Formel (II) oder (III) oder Verfahren zu deren Herstellung offenbart.

Insbesondere stellt die regioselektive Einführung der Substituenten Q bzw. X an Verbindungen der Formel (I) eine große Herausforderung dar. Besonders schwierig gestaltet sich dies durch die beiden chemisch sehr ähnlichen Ringsysteme die in den Verbindungen der Formel (I) aneinander gekoppelt sind. In der Literatur ist die Metallierung und folgende Halogenierung von solchen Imidazopyridinen auf dem Pyridin Ring bisher nicht beschrieben.

Bekannt sind aus dem Stand der Technik lediglich Syntheseverfahren zur Herstellung von ungekoppelten, halogenierten Pyridinderivaten. Diese bisher im Stand der Technik beschriebenen, chemischen Syntheseverfahren bedienen sich jedoch zudem sehr häufig Methoden, die aus industrieller Sicht wirtschaftlich nicht zu realisieren sind und/oder andere Nachteile aufweisen.

Nachteilig sind insbesondere bei Lithium- und Magnesiumbasen die geringen chemischen Ausbeuten, die Durchführung bei sehr niedrigen Temperaturen sowie die schwierige Regio- und Chemo-Selektivität der Deprotoniereung aufgrund der hohen Reaktivität von diesen Reagenzien. Manchmal ist eine Transmetallierung mit Zinksalzen wie zum Beispiel Zinkchlorid notwendig, um weitere selektive Reaktionen wie Negishi-Kreuzkupplungen durchzuführen, wie in Organic Letters 2008 (10), S. 2497ff beschrieben. Die Herstellung ist daher sehr teuer (viele Salze entstehen) und nicht für großtechnische kommerzielle Verfahren geeignet.

Im Hinblick auf die vorstehend geschilderten Nachteile besteht dringend Bedarf für ein vereinfachtes, technisch und ökonomisch durchführbares Verfahren zur Herstellung von halogenierten Imidazopyridinderivaten, insbesondere von halogenierten oder substituierten Imidazopyridinderivaten der Formel (II) oder (III). Die mit diesem angestrebten Verfahren erhältlichen halogenierten Imidazopyridinderivate sollen dabei vorzugsweise mit guter Ausbeute, hoher Reinheit und in ökonomischer Weise erhalten werden.

Überraschenderweise wurde gefunden, dass in einem Verfahren unter Verwendung einer Zink-metallorganischen Base halogenierte Imidazopyridinderivate der Formel (II) vorteilhaft, insbesondere auch mit hoher Regio- und Chemoselektivität und guter Ausbeute hergestellt werden können.

Gegenstand der vorliegenden Erfindung ist demnach ein Verfahren zur Herstellung von Verbindungen der Formel (II) 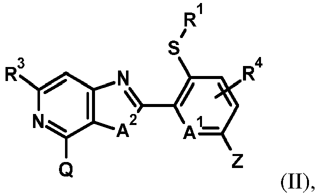 in welcher (Ausgestaltung 1)
- Q und Z: unabhängig voneinander für Halogen stehen,
- A¹: für Stickstoff, =N⁺-O⁻ oder =C-R⁵ steht,
- A²: für ―N-R⁶, Sauerstoff oder Schwefel steht,
- R¹: für (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Halogenalkoxy-(C₁-C₆)alkyl, (C₂-C₆)Alkenyl, (C₂-C₆)Alkenyloxy-(C₁-C₆)alkyl, (C₂-C₆)Halogenalkenyloxy-(C₁-C₆)alkyl, (C₂-C₆)Halogenalkenyl, (C₂-C₆)Cyanoalkenyl, (C₂-C₆)Alkinyl, (C₂-C₆)Alkinyloxy-(C₁-C₆)alkyl, (C₂-C₆)Halogenalkinyloxy-(C₁-C₆)alkyl, (C₂-C₆)Halogenalkinyl, (C₂-C₆)Cyanoalkinyl, (C₃-C₈)Cycloalkyl, (C₃-C₆)Halogencycloalkyl, (C₃-C₆)Cyanocycloalkyl, (C₃-C₈)Cycloalkyl-(C₃-C₈)Cycloalkyl, (C₁-C₆)Alkyl-(C₃-C₈)Cycloalkyl, Halogen(C₃-C₈)cycloalkyl, Amino, (C₁-C₆)Alkylamino, Di-(C₁-C₆)alkyl-amino, (C₃-C₈)Cycloalkylamino, (C₁-C₆)Alkylcarbonyl-amino, (C₁-C₆)Alkylthio-(C₁-C₆)alkyl, (C₁-C₆)Halogenalkylthio-(C₁-C₆)alkyl, (C₁-C₆)Alkylsulfinyl-(C₁-C₆)alkyl, (C₁-C₆)Halogenalkylsulfinyl-(C₁-C₆)alkyl, (C₁-C₆)Alkylsulfonyl-(C₁-C₆)alkyl, (C₁-C₆)Halogenalkylsulfonyl-(C₁-C₆)alkyl, (C₁-C₆)Alkoxy-(C₁-C₆)alkylthio-(C₁-C₆)alkyl, (C₁-C₆)Alkoxy-(C₁-C₆)alkylsulfinyl-(C₁-C₆)alkyl, (C₁-C₆)Alkoxy-(C₁-C₆)alkylsulfonyl-(C₁-C₆)alkyl, (C₁-C₆)Alkylcarbonyl-(C₁-C₆)alkyl, (C₁-C₆)Halogenalkylcarbonyl-(C₁-C₆)alkyl, (C₁-C₆)Alkoxycarbonyl-(C₁-C₆)alkyl, (C₁-C₆)Halogenalkoxycarbonyl-(C₁-C₆)alkyl, (C₁-C₆)Alkylsulfonylamino, Aminosulfonyl-(C₁-C₆)alkyl, (C₁-C₆)Alkylaminosulfonyl-(C₁-C₆)alkyl, Di-(C₁-C₆)alkyl-aminosulfonyl-(C₁-C₆)alkyl,
oder für jeweils gegebenenfalls einfach oder mehrfach, gleich oder verschieden durch Aryl, Hetaryl oder Heterocyclyl substituiertes (C₁-C₆)Alkyl, (C₁-C₆)Alkoxy, (C₂-C₆)Alkenyl, (C₂-C₆)Alkinyl, (C₃-C₈)Cycloalkyl steht, wobei Aryl, Hetaryl oder Heterocyclyl jeweils gegebenenfalls einfach oder mehrfach, gleich oder verschieden durch Halogen, Cyano, Nitro, Hydroxy, Amino, Carboxy, Carbamoyl, Aminosulfonyl, (C₁-C₆)Alkyl, (C₃-C₆)Cycloalkyl, (C₁-C₆)Alkoxy, (C₁-C₆)Halogenalkyl, (C₁-C₆)Halogenalkoxy, (C₁-C₆)Alkylthio, (C₁-C₆)Alkyl-sulfinyl, (C₁-C₆)Alkylsulfonyl, (C₁-C₆)Alkylsulfimino, (C₁-C₆)Alkylsulfimino-(C₁-C₆)alkyl, (C₁-C₆)Alkylsulfimino-(C₂-C₆)alkylcarbonyl, (C₁-C₆)Alkylsulfoximino, (C₁-C₆)Alkylsulfoximino-(C₁-C₆)alkyl, (C₁-C₆)Alkylsulfoximino-(C₂-C₆)alkylcarbonyl, (C₁-C₆)Alkoxycarbonyl, (C₁-C₆)Alkylcarbonyl, (C₃-C₆)Trialkylsilyl oder Benzyl substituiert sein können,
- R³: für Wasserstoff, Cyano, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, Cyano(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, -NHCO-(C₁-C₆)alkyl ((C₁-C₆)Alkylcarbonylamino) oder
für jeweils gegebenenfalls einfach oder mehrfach, gleich oder verschieden substituiertes Aryl oder Hetaryl steht, wobei (im Fall von Hetaryl) gegebenenfalls mindestens eine Carbonylgruppe enthalten sein kann und wobei als Substituenten jeweils in Frage kommen: Cyano, Carboxyl, Halogen, Nitro, Acetyl, Hydroxy, Amino, SCN, Tri-(C₁-C₆)alkylsilyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkylaminosulfonyl, (C₁-C₆)Alkylsulfoximino, Aminothiocarbonyl, (C₁-C₆)Alkylaminothiocarbonyl, Di-(C₁-C₆)alkylaminothiocarbonyl oder (C₃-C₈)Cycloalkylamino,
- R⁴: für Wasserstoff, Cyano, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, Cyano(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, NHCO-(C₁-C₆)alkyl ((C₁-C₆)Alkylcarbonylamino) oder
für jeweils gegebenenfalls einfach oder mehrfach, gleich oder verschieden substituiertes Aryl oder Hetaryl steht, wobei (im Fall von Hetaryl) gegebenenfalls mindestens eine Carbonylgruppe enthalten sein kann und wobei als Substituenten jeweils in Frage kommen: Cyano, Carboxyl, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkylaminosulfonyl, (C₁-C₆)Alkylsulfoximino, Aminothiocarbonyl, (C₁-C₆)Alkylaminothiocarbonyl, Di-(C₁-C₆)alkylaminothiocarbonyl, (C₃-C₈)Cycloalkylamino, (C₁-C₆)Alkylcarbonylamino,
- R⁵: für Wasserstoff, Cyano, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino,(C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio,(C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino oder-NHCO-(C₁-C₆)alkyl ((C₁-C₆)Alkylcarbonylamino) steht und
- R⁶: für (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Halogenalkoxy-(C₁-C₆)alkyl, (C₂-C₆)Alkenyl, (C₂-C₆)Alkenyloxy-(C₁-C₆)alkyl, (C₂-C₆)Halogenalkenyloxy-(C₁-C₆)alkyl, (C₂-C₆)Halogenalkenyl, (C₂-C₆)Cyanoalkenyl, (C₂-C₆)Alkinyl, (C₂-C₆)Alkinyloxy-(C₁-C₆)alkyl, (C₂-C₆)Halogenalkinyloxy-(C₁-C₆)alkyl, (C₂-C₆)Halogenalkinyl, (C₂-C₆)Cyanoalkinyl, (C₃-C₈)Cycloalkyl, (C₃-C₈)Cycloalkyl-(C₃-C₈)Cycloalkyl, (C₁-C₆)Alkyl-(C₃-C₈)Cycloalkyl, Halogen(C₃-C₈)cycloalkyl, (C₁-C₆)Alkylthio-(C₁-C₆)alkyl, (C₁-C₆)Halogenalkylthio-(C₁-C₆)alkyl, (C₁-C₆)Alkylsulfinyl-(C₁-C₆)alkyl, (C₁-C₆)Halogenalkylsulfinyl-(C₁-C₆)alkyl, (C₁-C₆)Alkylsulfonyl-(C₁-C₆)alkyl, (C₁-C₆)Halogenalkylsulfonyl-(C₁-C₆)alkyl, (C₁-C₆)Alkoxy-(C₁-C₆)alkylthio-(C₁-C₆)alkyl, (C₁-C₆)Alkoxy-(C₁-C₆)alkylsulfinyl-(C₁-C₆)alkyl, (C₁-C₆)Alkoxy-(C₁-C₆)alkylsulfonyl-(C₁-C₆)alkyl, (C₁-C₆)Alkylcarbonyl-(C₁-C₆)alkyl, (C₁-C₆)Halogenalkylcarbonyl-(C₁-C₆)alkyl, (C₁-C₆)Alkoxycarbonyl-(C₁-C₆)alkyl, (C₁-C₆)Halogenalkoxycarbonyl-(C₁-C₆)alkyl, Aminocarbonyl-(C₁-C₆)alkyl, (C₁-C₆)Alkylamino-(C₁-C₆)alkyl, Di-(C₁-C₆)alkylamino-(C₁-C₆)alkyl oder (C₃-C₈)Cycloalkylamino-(C₁-C₆)alkyl steht,
dadurch gekennzeichnet, dass in einem ersten Verfahrensschritt a) eine Verbindung der Formel (I) 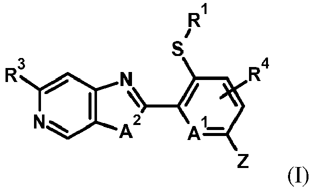
in welcher Z, R¹, R³, R⁴, A¹ und A² jeweils die oben genannten Bedeutungen haben,
mit einer Zink-metallorganischen Base der Struktur (NR^{a}R^{b})-Zn-R^{c} oder (NR^{a}R^{b})₂-Zn, in welcher
R^{c} für Halogen oder -O-Pivaloyl steht und
R^{a} und R^{b} gemeinsam eine -(CH₂)₄-, -(CH₂)₅- oder -(CH₂)₂O(CH₂)₂-Gruppe bilden, wobei jede dieser Gruppen optional durch 1, 2, 3 oder 4 R^{d}-Radikale substituiert sein kann und R^{d} ausgewählt ist aus der Gruppe bestehend aus Methyl, Ethyl, n-Propyl und i-Propyl,
zu einer Verbindung der Formel (IVa) oder der Formel (IVb) umgesetzt wird, 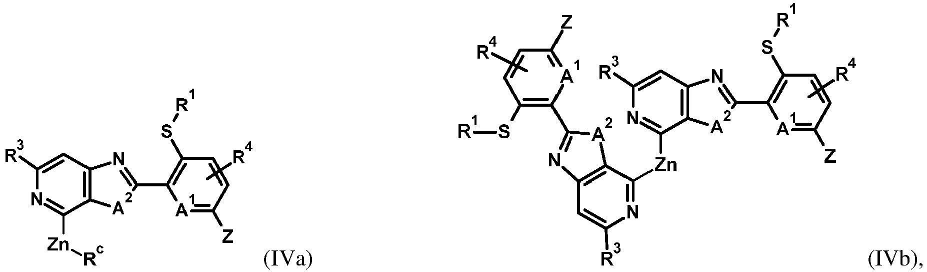 in welcher Z, R¹, R³, R⁴, R^{c}, A¹ und A² jeweils die oben genannten Bedeutungen haben,
und diese Verbindung der Formel (IVa) oder (IVb) in einem zweiten Verfahrensschritt b) mit einer Verbindung der Struktur Q-V, in welcher V für Halogen steht und Q die oben genannte Bedeutung hat, zur Verbindung der Formel (II) umgesetzt wird.

Bei der Verbindung Q-V handelt es sich, wie aus den Definitionen für Q und V hervorgeht, um eine Interhalogenverbindung, vorzugsweise um elementares Halogen. Q und V müssen nicht notwendigerweise für das gleiche Halogen stehen. Beispielsweise kann Q für Iod oder Brom stehen und V für Chlor, Brom oder Iod. Vorzugsweise handelt es sich bei der Verbindung Q-V aber um ein elementares Halogen, insbesondere F₂, Cl₂, Br₂ oder I₂. Besonders bevorzugt sind I₂ oder Br₂, ganz besonders bevorzugt ist I₂.

Bevorzugt stehen die Reste Z und Q für unterschiedliche Halogene, besonders bevorzugt steht Z für Fluor, Chlor oder Brom und für den Fall, dass
- Z: für Fluor steht, Q für Iod, Brom oder Chlor oder
- Z: für Chlor steht, Q für Iod oder Brom oder
- Z: für Brom steht, Q für Iod.

Die Verbindungen der Formel (IVa) und (IVb) können auch mit Salzen komplexiert vorliegen, wobei es sich bei den Salzen bevorzugt um Alkali- oder Erdalkalihalogenide handelt, vorzugsweise um Lithiumchlorid und/oder Magnesiumchlorid und besonders bevorzugt um Lithiumchlorid.

Bevorzugte und besonders bevorzugte Bedeutungen der in den vorstehend erwähnten Formeln (I), (II), (IVa) und (IVb) des erfindungsgemäßen Verfahrens aufgeführten Reste Q, Z, R¹, R³, R⁴, R^{c}, A¹ und A² werden im Folgenden erläutert, wobei die Zink-metallorganische Base weiter unten noch genau beschrieben wird, so dass die bevorzugten Ausgestaltungen der Base dort angegeben sind.

### (Ausgestaltung 2)

- Q: steht bevorzugt für Chlor, Iod oder Brom,
- Z: steht bevorzugt für Brom, Fluor oder Chlor,
- R^{c}: steht bevorzugt für -O-Pivaloyl, Chlor, Brom oder Iod,
- A¹: steht bevorzugt für Stickstoff, =N⁺-O⁻ oder =C-R⁵,
- A²: steht bevorzugt für ―N-R⁶ oder Sauerstoff,
- R¹: steht bevorzugt für (C₁-C₄)Alkyl, (C₁-C₄)Hydroxyalkyl, (C₁-C₄)Halogenalkyl, (C₁-C₄)Cyanoalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyloxy-(C₁-C₄)alkyl, (C₂-C₄)Halogenalkinyl, (C₂-C₄)Cyanoalkinyl, (C₃-C₆)Cycloalkyl, (C₃-C₆)Halogencycloalkyl, (C₃-C₆)Cyanocycloalkyl, (C₃-C₆)Cycloalkyl(C₃-C₆)cycloalkyl, (C₁-C₄)Alkyl-(C₃-C₆)cycloalkyl, Halogen(C₃-C₆)cycloalkyl, (C₁-C₄)Alkylamino, Di-(C₁-C₄)alkyl-amino, (C₃-C₆)Cycloalkylamino, (C₁-C₄)Alkylcarbonyl-amino, (C₁-C₄)Alkylthio-(C₁-C₄)alkyl, (C₁-C₄)Halogenalkylthio-(C₁-C₄)alkyl, (C₁-C₄)Alkylsulfinyl-(C₁-C₄)alkyl, (C₁-C₄)Halogenalkylsulfinyl-(C₁-C₄)alkyl, (C₁-C₄)Alkylsulfonyl-(C₁-C₄)alkyl, (C₁-C₄)Alkylcarbonyl-(C₁-C₄)alkyl, (C₁-C₄)Halogenalkylcarbonyl-(C₁-C₄)alkyl, (C₁-C₄)Alkylsulfonylamino,
oder für jeweils gegebenenfalls einfach oder zweifach, gleich oder verschieden durch Aryl, Hetaryl oder Heterocyclyl substituiertes (C₁-C₄)Alkyl, (C₁-C₄)Alkoxy, (C₂-C₄)Alkenyl, (C₂-C₄)Alkinyl, (C₃-C₆)Cycloalkyl, wobei Aryl, Hetaryl oder Heterocyclyl jeweils gegebenenfalls einfach oder zweifach gleich oder verschieden durch Halogen, Cyano, Carbamoyl, Aminosulfonyl, (C₁-C₄)Alkyl, (C₃-C₄)Cycloalkyl, (C₁-C₄)Alkoxy, (C₁-C₄)Halogenalkyl, (C₁-C₄)Halogenalkoxy, (C₁-C₄)Alkylthio, (C₁-C₄)Alkylsulfinyl, (C₁-C₄)Alkylsulfonyl, (C₁-C₄)Alkylsulfimino substituiert sein können,
- R³: steht bevorzugt für Wasserstoff, Cyano, Halogen, Nitro, Acetyl, Hydroxy, Amino, SCN, Tri-(C₁-C₄)alkylsilyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, Cyano(C₃-C₈)cycloalkyl, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Cyanoalkoxy, (C₁-C₄)Alkoxy-(C₁-C₄)alkoxy, (C₁-C₄)Alkylhydroxyimino, (C₁-C₄)Alkoxyimino, (C₁-C₄)Alkyl-(C₁-C₄)alkoxyimino, (C₁-C₄)Halogenalkyl-(C₁-C₄)alkoxyimino, (C₁-C₄)Alkylthio, (C₁-C₄)Halogenalkylthio, (C₁-C₄)Alkylthio-(C₁-C₄)alkyl, (C₁-C₄)Alkylsulfinyl, (C₁-C₄)Halogenalkylsulfinyl, (C₁-C₄)Alkylsulfinyl-(C₁-C₄)alkyl, (C₁-C₄)Alkylsulfonyl, (C₁-C₄)Halogenalkylsulfonyl, (C₁-C₄)Alkylsulfonyl-(C₁-C₄)alkyl, (C₁-C₄)Alkylsulfonyloxy, (C₁-C₄)Alkylcarbonyl, (C₁-C₄)Halogenalkylcarbonyl, Aminocarbonyl, Aminothiocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl, Di-(C₁-C₄)alkyl-aminosulfonyl, Aminothiocarbonyl, NHCO-(C₁-C₄)alkyl ((C₁-C₄)Alkylcarbonylamino) oder
für jeweils gegebenenfalls einfach oder zweifach, gleich oder verschieden substituiertes Phenyl oder Hetaryl, wobei (im Fall von Hetaryl) gegebenenfalls mindestens eine Carbonylgruppe enthalten sein kann und wobei als Substituenten jeweils in Frage kommen: Cyano, Halogen, Nitro, Acetyl, Amino, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Cyanoalkoxy, (C₁-C₄)Alkoxy-(C₁-C₄)alkoxy, (C₁-C₄)Alkylhydroxyimino, (C₁-C₄)Alkoxyimino, (C₁-C₄)Alkyl-(C₁-C₄)alkoxyimino, (C₁-C₄)Halogenalkyl-(C₁-C₄)alkoxyimino, (C₁-C₄)Alkylthio, (C₁-C₄)Halogenalkylthio, (C₁-C₄)Alkylthio-(C₁-C₄)alkyl, (C₁-C₄)Alkylsulfinyl, (C₁-C₄)Halogenalkylsulfinyl, (C₁-C₄)Alkylsulfinyl-(C₁-C₄)alkyl, (C₁-C₄)Alkylsulfonyl, (C₁-C₄)Halogenalkylsulfonyl, (C₁-C₄)Alkylsulfonyl-(C₁-C₄)alkyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl und Di-(C₁-C₄)alkylaminosulfonyl,

- R⁴: steht bevorzugt für Wasserstoff, Cyano, Halogen, Nitro, Acetyl, Hydroxy, Amino, SCN, Tri-(C₁-C₄)alkylsilyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, Cyano(C₃-C₈)cycloalkyl, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Cyanoalkoxy, (C₁-C₄)Alkoxy-(C₁-C₄)alkoxy, (C₁-C₄)Alkylhydroxyimino, (C₁-C₄)Alkoxyimino, (C₁-C₄)Alkyl-(C₁-C₄)alkoxyimino, (C₁-C₄)Halogenalkyl-(C₁-C₄)alkoxyimino, (C₁-C₄)Alkylthio, (C₁-C₄)Halogenalkylthio, (C₁-C₄)Alkylthio-(C₁-C₄)alkyl, (C₁-C₄)Alkylsulfinyl, (C₁-C₄)Halogenalkylsulfinyl, (C₁-C₄)Alkylsulfinyl-(C₁-C₄)alkyl, (C₁-C₄)Alkylsulfonyl, (C₁-C₄)Halogenalkylsulfonyl, (C₁-C₄)Alkylsulfonyl-(C₁-C₄)alkyl, (C₁-C₄)Alkylsulfonyloxy, (C₁-C₄)Alkylcarbonyl, (C₁-C₄)Halogenalkylcarbonyl, Aminocarbonyl, Aminothiocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl, Di-(C₁-C₄)alkyl-aminosulfonyl, Aminothiocarbonyl, NHCO-(C₁-C₄)alkyl ((C₁-C₄)Alkylcarbonylamino),
weiterhin bevorzugt für jeweils gegebenenfalls einfach oder zweifach, gleich oder verschieden substituiertes Phenyl oder Hetaryl, wobei (im Fall von Hetaryl) gegebenenfalls mindestens eine Carbonylgruppe enthalten sein kann und wobei als Substituenten jeweils in Frage kommen: Cyano, Halogen, Nitro, Acetyl, Amino, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Cyanoalkoxy, (C₁-C₄)Alkoxy-(C₁-C₄)alkoxy, (C₁-C₄)Alkylhydroxyimino, (C₁-C₄)Alkoxyimino, (C₁-C₄)Alkyl-(C₁-C₄)alkoxyimino, (C₁-C₄)Halogenalkyl-(C₁-C₄)alkoxyimino,(C₁-C₄)Alkylthio, (C₁-C₄)Halogenalkylthio, (C₁-C₄)Alkylthio-(C₁-C₄)alkyl, (C₁-C₄)Alkylsulfinyl, (C₁-C₄)Halogenalkylsulfinyl, (C₁-C₄)Alkylsulfinyl-(C₁-C₄)alkyl, (C₁-C₄)Alkylsulfonyl, (C₁-C₄)Halogenalkylsulfonyl, (C₁-C₄)Alkylsulfonyl-(C₁-C₄)alkyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino,Di-(C₁-C₄)Alkylamino, Aminosulfonyl, (C₁-C₄)Alkylaminosulfonyl, Di-(C₁-C₄)alkylaminosulfonyl, NHCO-(C₁-C₄)alkyl ((C₁-C₄)Alkylcarbonylamino),
- R⁵: steht bevorzugt für Wasserstoff, Cyano, Halogen, Nitro, Acetyl, Hydroxy, Amino, SCN, Tri-(C₁-C₄)alkylsilyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Cyanoalkoxy, (C₁-C₄)Alkoxy-(C₁-C₄)alkoxy, (C₁-C₄)Alkylhydroxyimino, (C₁-C₄)Alkoxyimino, (C₁-C₄)Alkyl-(C₁-C₄)alkoxyimino, (C₁-C₄)Halogenalkyl-(C₁-C₄)alkoxyimino, (C₁-C₄)Alkylthio, (C₁-C₄)Halogenalkylthio, (C₁-C₄)Alkylthio-(C₁-C₄)alkyl, (C₁-C₄)Alkylsulfinyl, (C₁-C₄)Halogenalkylsulfinyl, (C₁-C₄)Alkylsulfinyl-(C₁-C₄)alkyl, (C₁-C₄)Alkylsulfonyl, (C₁-C₄)Halogenalkylsulfonyl, (C₁-C₄)Alkylsulfonyl-(C₁-C₄)alkyl, (C₁-C₄)Alkylsulfonyloxy, (C₁-C₄)Alkylcarbonyl, (C₁-C₄)Halogenalkylcarbonyl, Aminocarbonyl, Aminothiocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl, Di-(C₁-C₄)alkyl-aminosulfonyl, Aminothiocarbonyl, NHCO-(C₁-C₄)alkyl ((C₁-C₄)Alkylcarbonylamino) und
- R⁶: steht bevorzugt für (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Halogenalkylthio-(C₁-C₄)alkyl, (C₁-C₄)Alkylsulfinyl-(C₁-C₄)alkyl, (C₁-C₄)Halogenalkylsulfinyl-(C₁-C₄)alkyl, (C₁-C₄)Alkylsulfonyl-(C₁-C₄)alkyl, (C₁-C₄)Halogenalkylsulfonyl-(C₁-C₄)alkyl, (C₁-C₄)Alkoxy-(C₁-C₄)alkylthio-(C₁-C₄)alkyl oder (C₁-C₄)Alkylcarbonyl-(C₁-C₄)alkyl.

### (Ausgestaltung 3)

- Q: steht besonders bevorzugt für Chlor, Iod oder Brom,
- Z: steht besonders bevorzugt für Brom. Fluor oder Chlor,
- R^{c}: steht besonders bevorzugt für -O-Pivaloyl, Chlor, Brom oder Iod,
- A¹: steht besonders bevorzugt für Stickstoff oder =C-R⁵,
- A²: steht besonders bevorzugt für ―N-R⁶,
- R¹: steht besonders bevorzugt für (C₁-C₄)Alkyl, (C₁-C₄)Hydroxyalkyl, (C₁-C₄)Halogenalkyl, (C₂-C₄)Alkenyl, (C₂-C₄)Halogenalkenyl, (C₂-C₄)Alkinyl, (C₂-C₄)Halogenalkinyl, (C₃-C₆)Cycloalkyl, (C₁-C₄)Alkylthio-(C₁-C₄)alkyl, (C₁-C₄)Alkylsulfinyl-(C₁-C₄)alkyl oder (C₁-C₄)Alkylsulfonyl-(C₁-C₄)alkyl,
- R³: steht besonders bevorzugt für Wasserstoff, Halogen, (C₁-C₄)Alkyl, (C₁-C₄)Halogenalkyl, (C₁-C₄)Halogenalkoxy, (C₁-C₄)Alkylthio, (C₁-C₄)Alkylsulfinyl, (C₁-C₄)Alkylsulfonyl, (C₁-C₄)Halogenalkylthio, (C₁-C₄)Halogenalkylsulfinyl oder (C₁-C₄)Halogenalkylsulfonyl,
- R⁴: steht besonders bevorzugt für Wasserstoff, Cyano, Halogen, Nitro, Hydroxy, Amino, SCN, Tri-(C₁-C₄)alkylsilyl, (C₃-C₆)Cycloalkyl, Cyano(C₃-C₈)cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkyl, (C₁-C₄)Halogenalkyl, (C₁-C₄)Cyanoalkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Cyanoalkoxy, (C₁-C₄)Alkylhydroxyimino, (C₁-C₄)Alkoxyimino, (C₁-C₄)Alkyl-(C₁-C₄)alkoxyimino, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl, Di-(C₁-C₄)alkyl-aminosulfonyl, NHCO-(C₁-C₄)alkyl ((C₁-C₄)Alkylcarbonylamino),
weiterhin besonders bevorzugt für jeweils gegebenenfalls einfach oder zweifach, gleich oder verschieden substituiertes Phenyl oder Hetaryl, wobei (im Fall von Hetaryl) gegebenenfalls mindestens eine Carbonylgruppe enthalten sein kann und wobei als Substituenten jeweils in Frage kommen: Cyano, Halogen, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkyl, (C₁-C₄)Halogenalkyl, (C₁-C₄)Cyanoalkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Alkylhydroxyimino, (C₁-C₄)Alkoxyimino, (C₁-C₄)Alkyl-(C₁-C₄)alkoxyimino, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl, Di-(C₁-C₄)alkylaminosulfonyl, NHCO-(C₁-C₄)alkyl ((C₁-C₄)Alkylcarbonylamino),
- R⁵: steht besonders bevorzugt für Wasserstoff, Halogen, Cyano oder (C₁-C₄)Alkyl und
- R⁶: steht besonders bevorzugt für (C₁-C₄)Alkyl oder (C₁-C₄)Alkoxy-(C₁-C₄)alkyl.

### (Ausgestaltung 4)

- Q: steht ganz besonders bevorzugt für Iod oder Brom,
- Z: steht ganz besonders bevorzugt für Brom oder Chlor,
- R^{c}: steht ganz besonders bevorzugt für Chlor, Brom oder Iod,
- A¹: steht ganz besonders bevorzugt für Stickstoff oder =C-R⁵,
- A²: steht ganz besonders bevorzugt für ―N-R⁶,
- R¹: steht ganz besonders bevorzugt für Methyl, Ethyl, n-Propyl, i-Propyl, cyclo-Propyl, n-Butyl, i-Butyl, tert.-Butyl, cyclo-Butyl, Fluormethyl, Difluormethyl, Trifluormethyl, Fluorethyl, Difluorethyl, Trifluorethyl, Tetrafluorethyl oder Pentafluorethyl,
- R³: steht ganz besonders bevorzugt für Fluor, Chlor, Fluormethyl, Difluormethyl, Trifluormethyl, Fluorethyl, Difluorethyl, Trifluorethyl, Tetrafluorethyl, Pentafluorethyl, Trifluormethoxy, Difluorchlormethoxy, Dichlorfluormethoxy, Trifluormethylthio, Trifluormethylsulfonyl oder Trifluormethylsulfinyl,
- R⁴: steht ganz besonders bevorzugt für Wasserstoff, Cyano, Halogen, (C₁-C₄)Alkyl, (C₁-C₄)Halogenalkyl, (C₁-C₄)Halogenalkoxy, (C₁-C₄)Alkylthio, (C₁-C₄)Alkylsulfinyl, (C₁-C₄)Alkylsulfonyl, (C₁-C₄)Halogenalkylthio, (C₁-C₄)Halogenalkylsulfinyl, (C₁-C₄)Halogenalkylsulfonyl oder NHCO-(C₁-C₄)alkyl ((C₁-C₄)Alkylcarbonylamino),
- R⁵: steht ganz besonders bevorzugt für Wasserstoff, Fluor, Chlor, Brom oder Cyano und
- R⁶: steht ganz besonders bevorzugt für Methyl, Ethyl, i-Propyl, Methoxymethyl oder Methoxyethyl.

### (Ausgestaltung 5)

- Q: steht hervorgehoben für Iod oder Brom,
- Z: steht hervorgehoben für Brom oder Chlor,
- R^{c}: steht hervorgehoben für Chlor oder Brom,
- A¹: steht hervorgehoben für Stickstoff,
- A²: steht hervorgehoben für ―N-R⁶,
- R¹: steht hervorgehoben für Methyl, Ethyl, n-Propyl, i-Propyl oder cyclo-Propyl,
- R³: steht hervorgehoben für Trifluormethyl oder Pentafluorethyl,
- R⁴: steht hervorgehoben für Wasserstoff und
- R⁶: steht hervorgehoben für Methyl.

### (Ausgestaltung 6)

- Q: steht insbesondere für Iod oder Brom,
- Z: steht insbesondere für Brom oder Chlor,
- R^{c}: steht insbesondere für Chlor,
- A¹: steht insbesondere für Stickstoff,
- A²: steht insbesondere für ―N-R⁶,
- R¹: steht insbesondere für Ethyl,
- R³: steht insbesondere für Trifluormethyl,
- R⁴: steht insbesondere für Wasserstoff und
- R⁶: steht insbesondere für Methyl.

Die vorstehend aufgeführten Restedefinitionen bzw. Erläuterungen gelten sowohl für die Endprodukte und Zwischenprodukte als auch für die Ausgangsprodukte entsprechend. Diese Restedefinitionen können untereinander, also auch zwischen den jeweiligen Vorzugsbereichen, beliebig kombiniert werden.

Definitionsgemäß ist, sofern nichts anderes angegeben ist, Halogen ausgewählt aus der Reihe Fluor, Chlor, Brom und Iod, bevorzugt wiederum aus der Reihe Fluor, Chlor und Brom.

Sofern nicht an anderer Stelle anders definiert, wird unter dem Begriff "Alkyl", entweder in Alleinstellung oder aber in Kombination mit weiteren Begriffen, wie beispielsweise Halogenalkyl, im Rahmen der vorliegenden Erfindung ein Rest einer gesättigten, aliphatischen Kohlenwasserstoffgruppe mit 1 bis 12 Kohlenstoffatomen verstanden, die verzweigt oder unverzweigt sein kann. Beispiele für C₁-C₁₂-Alkylreste sind Methyl, Ethyl, n-Propyl, iso-Propyl, n-Butyl, iso-Butyl, sek.-Butyl, tert.-Butyl, n-Pentyl, iso-Pentyl, Neopentyl, tert.-Pentyl, 1-Methylbutyl, 2-Methylbutyl, 1-Ethylpropyl, 1,2-Dimethylpropyl, Hexyl, n-Heptyl, n-Octyl, n-Nonyl, n-Decyl, n-Undecyl und n-Dodecyl.

Sofern nicht an anderer Stelle anders definiert, wird unter dem Begriff "Alkenyl", entweder in Alleinstellung oder aber in Kombination mit weiteren Begriffen, erfindungsgemäß ein linearer oder verzweigter C₂-C₁₂-Alkenylrest, welcher mindestens eine Doppelbindung aufweist, beispielsweise Vinyl, Allyl, 1-Propenyl, Isopropenyl, 1-Butenyl, 2-Butenyl, 3-Butenyl, 1,3-Butadienyl, 1-Pentenyl, 2-Pentenyl, 3-Pentenyl, 4-Pentenyl, 1,3-Pentadienyl, 1-Hexenyl, 2-Hexenyl, 3-Hexenyl, 4-Hexenyl, 5-Hexenyl und 1,4-Hexadienyl, verstanden.

Sofern nicht an anderer Stelle anders definiert, wird unter dem Begriff "Alkinyl", entweder in Alleinstellung oder aber in Kombination mit weiteren Begriffen, erfindungsgemäß ein linearer oder verzweigter C₂-C₁₂-Alkinylrest, welcher mindestens eine Dreifachbindung aufweist, beispielsweise Ethinyl, 1-Propinyl und Propargyl, verstanden. Der Alkinylrest kann dabei auch mindestens eine Doppelbindung aufweisen.

Sofern nicht an anderer Stelle anders definiert, wird unter dem Begriff "Cycloalkyl", entweder in Alleinstellung oder aber in Kombination mit weiteren Begriffen, erfindungsgemäß ein C₃-C₈-Cycloalkylrest verstanden, beispielsweise Cyclopropyl, Cyclobutyl, Cyclopentyl, Cyclohexyl, Cycloheptyl und Cyclooctyl, verstanden.

Unter dem Begriff "Alkoxy", entweder in Alleinstellung oder aber in Kombination mit weiteren Begriffen, wie beispielsweise Halogenalkoxy, wird vorliegend ein Rest O-Alkyl verstanden, wobei der Begriff "Alkyl" die oben stehende Bedeutung aufweist.

Sofern nicht an anderer Stelle anders definiert, wird unter dem Begriff "Aryl" erfindungsgemäß ein aromatischer Rest mit 6 bis 14 Kohlenstoffatomen, vorzugsweise Phenyl, Naphthyl, Anthryl oder Phenanthrenyl, besonders bevorzugt Phenyl, verstanden.

Sofern nicht an anderer Stelle anders definiert, wird unter dem Begriff "Arylalkyl" eine Kombination von erfindungsgemäß definierten Resten "Aryl" und "Alkyl" verstanden, wobei der Rest im Allgemeinen über die Alkylgrupe gebunden wird, Beispiele hierfür sind Benzyl, Phenylethyl oder α-Methylbenzyl, wobei Benzyl besonders bevorzugt ist.

Sofern nicht an anderer Stelle anders definiert, bedeutet "Hetaryl" bzw "heteroaromatischer Ring" eine mono-, bi- oder tricyclische heterocyclische Gruppe aus C-Atomen und mindestens einem Heteroatom, wobei mindestens ein Zyklus aromatisch ist. Bevorzugt enthält die Hetaryl-Gruppe 3, 4, 5, 6, 7 oder 8 C-Atome. Besonders bevorzugt sind dabei monocyclische Gruppen aus 3, 4, 5, 6, 7 oder 8 C-Atomen und mindestens einem Heteroatom.Insbesondere bevorzugt ist die Hetaryl-Gruppe ausgewählt aus der Reihe Furyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl, Imidazopyridinyl und Indolizinyl.

Durch Halogen substituierte Reste, z.B. Halogenalkyl (=Haloalkyl), sind einfach oder mehrfach bis zur maximal möglichen Substituentenzahl halogeniert. Bei mehrfacher Halogenierung können die Halogenatome gleich oder verschieden sein. Gegebenenfalls substituierte Reste können, wenn nichts anderes erwähnt ist, einfach oder mehrfach substituiert sein, wobei bei Mehrfachsubstitutionen die Substituenten gleich oder verschieden sein können.

Vorteilhafterweise lassen sich die halogenierten Imidazopyridinderivate der Formel (II) mit dem erfindungsgemäßen Verfahren mit guten Ausbeuten und in hoher Reinheit herstellen. Aufgrund der sehr guten funktionellen Gruppen-Toleranz von Zink-Reagenzien sind Zinkbasen sehr attraktiv. Regio- und chemoselektive Metallierungen von Imidazopyridinen in Gegenwart von stöchiometrischen Mengen von selektiven Basen werden möglich, auch bei erhöhten Temperaturen, ohne dass eine Arin-Eliminierung stattfindet oder empfindliche funktionelle Gruppen angegriffen werden. Die als Zwischenstufe entstandene Zinkverbindung kann anschließend mit verschiedenen Elektrophilen abgefangen werden wie beispielsweise in Organic Letters 2009 (11), S. 1837ff beschrieben. Diese neu substituierten Imidazopyridinderivate können dann als wertvolle Synthone weiter umgesetzt werden.

Das erfindungsgemäße Verfahren kann anhand des folgenden Schemas (I) erläutert werden:

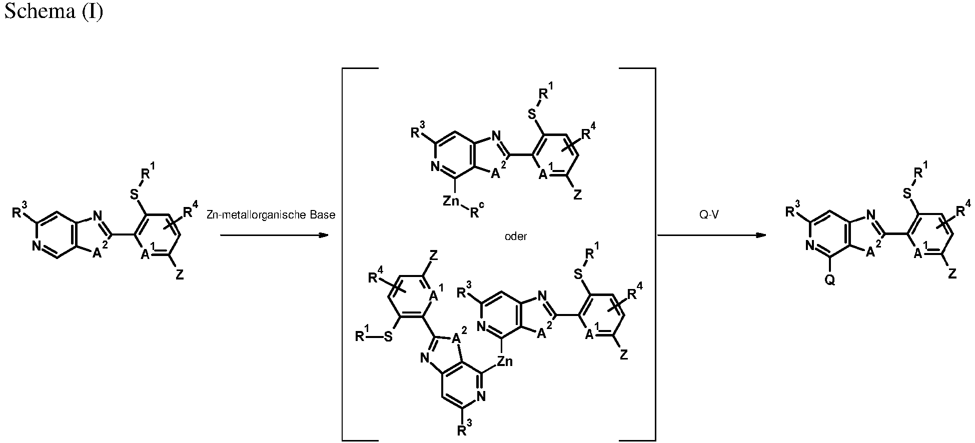

Hierin haben Q, V, Z, R¹, R³, R⁴, R^{c}, A¹ und A² die vorstehend angegebenen Bedeutungen. Die in Klammern angegebenen Verbindungen stellen die Zwischenstufe (Formel (IVa) beziehungsweise (IVb)) dar, welche weiter zur Verbindung der Formel (II) umgesetzt wird. Demnach lässt sich das erfindungsgemäße Verfahren in die beiden Verfahrensschritte a) und b) unterteilen, wobei Schritt a) die Umsetzung der Verbindung der Formel (I) zur jeweiligen Zwischenstufe und Schritt b) die weitere Umsetzung der Zwischenstufe zur Verbindung der Formel (II) ist.

### Allgemeine Definitionen

Im Zusammenhang mit der vorliegenden Erfindung umfasst der Begriff Halogen, soweit nicht anders definiert, solche Elemente, die ausgewählt sind aus der Gruppe bestehend aus Fluor, Chlor, Brom und Iod.

Der Begriff "Halogenide" beschreibt im Zusammenhang mit der vorliegenden Erfindung Verbindungen zwischen Halogenen und Elementen anderer Gruppen des Periodensystems, wobei salzartige Halogenide (ionische Verbindungen (Salze)), die aufgrund der großen Elektronegativitätsdifferenz zwischen den beteiligten Elementen aus Anionen und Kationen bestehen und durch elektrostatische Wechselwirkungen zusammengehalten werden) oder kovalente Halogenide (kovalente Verbindungen, bei denen der Elektronegativitätsunterschied nicht so groß ist wie bei den vorstehend genannten ionischen Verbindungen, die Bindungen jedoch eine Ladungspolarität aufweisen) vorliegen können, abhängig von der Art der chemischen Bindung. Erfindungsgemäß besonders bevorzugt sind salzartige Halogenide.

Der Begriff "Pivaloyl" beschreibt im Zusammenhang mit der vorliegenden Erfindung den deprotonierten Rest der Pivalinsäure (IX) mit der Summenformel (CH₃)₃CCO₂H. 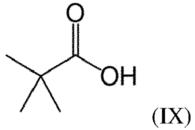 "O-Pivaloyl" bedeutet entsprechend, dass die Bindung des Pivaloylrestes über das deprotonierte Sauerstoffatom der Säuregruppe erfolgt.

Gegebenenfalls substituierte Gruppen können einfach oder mehrfach substituiert sein, wobei bei Mehrfachsubstitutionen die Substituenten gleich oder verschieden sein können.

Mit einem oder mehreren Halogenatomen (-Hal) substituierte Alkyl-Gruppen sind beispielsweise ausgewählt aus Trifluormethyl (CF₃), Difluormethyl (CHF₂), CF₃CH₂, ClCH₂ oder CF₃CCl₂.

Alkyl-Gruppen sind im Zusammenhang mit der vorliegenden Erfindung, soweit nicht abweichend definiert, lineare, verzweigte oder ringförmige gesättigte Kohlenwasserstoff-Gruppen.

Die Definition C₁-C₁₂-Alkyl umfasst den größten hierin definierten Bereich für einen Alkyl-Gruppe. Im Einzelnen umfasst diese Definition beispielsweise die Bedeutungen Methyl, Ethyl, n-, iso-Propyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl.

Die Umsetzung der Verbindungen der Formel (I) zu Verbindungen der Formel (IVa) oder Formel (IVb) im ersten Verfahrensschritt (Schritt a)) erfolgt in Gegenwart einer Zink-metallorganischen Base der Struktur (NR^{a}R^{b})-Zn-R^{c} oder (NR^{a}R^{b})₂-Zn, in welcher (Ausgestaltung B-1)
R^{c} wie vorstehend (Ausgestaltung 1) definiert ist (daher für Halogen oder -O-Pivaloyl steht),
R^{a} und R^{b} gemeinsam eine -(CH₂)₄-, -(CH₂)₅- oder -(CH₂)₂O(CH₂)₂-Gruppe bilden, wobei jede dieser Gruppen optional durch 1, 2, 3 oder 4 R^{d}-Radikale substituiert sein kann und
R^{d} ausgewählt ist aus der Gruppe bestehend aus Methyl, Ethyl, n-Propyl und i-Propyl.

Bevorzugt ist, dass (Ausgestaltung B-2)
R^{c} wie vorstehend als bevorzugt (Ausgestaltung 2) definiert ist (daher für -O-Pivaloyl, Chlor, Brom oder Iod steht),
R^{a} und R^{b} gemeinsam eine -(CH₂)₅-Gruppe bilden, wobei jede dieser Gruppen optional durch 1, 2, 3 oder 4 R^{d}-Radikale substituiert sein kann und
R^{d} ausgewählt ist aus der Gruppe bestehend aus Methyl und Ethyl.

Besonders bevorzugt ist, dass (Ausgestaltung B-3)
R^{c} wie vorstehend als besonders bevorzugt (Ausgestaltung 6) definiert ist (daher für Chlor steht) und
R^{a} und R^{b} gemeinsam eine -(CH₂)₅-Gruppe bilden, die durch 4 Methylgruppen substituiert ist.

Die vorstehend aufgeführten Restedefinitionen können untereinander, also auch zwischen den jeweiligen Vorzugsbereichen, beliebig kombiniert werden.

In einer ganz besonders bevorzugten Ausgestaltung der erfindungsgemäßen Base ist das Strukturelement (NR^{a}R^{b}) Tetramethylpiperidin (TMP) gemäß Formel (V).

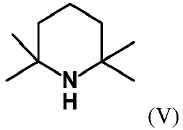

Erfindungsgemäß ganz besonders bevorzugte Zink-metallorganische Basen sind demnach dadurch gekennzeichnet, dass Zink gebunden an TMP vorliegt, insbesondere als Zinkhalogenid und ganz besonders bevorzugt als Zinkchlorid. Derartige Basen weisen folgende Struktur der Formel (VI) auf (Ausgestaltung B-4)

(VI) (TMP)ₓZnCl₂₋ₓ ,

worin x für die Zahl 1 oder 2 steht. Hierunter wiederum bevorzugt sind Basen mit x=l (Ausgestaltung B-5) gemäß Formel (VII):

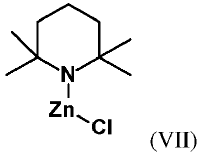

In einer weiteren bevorzugten Ausführungsform des erfindungsgemäßen Verfahrens liegt die metallorganische Base in Verbindung mit Alkali- oder Erdalkalihalogeniden vor. Dies gilt insbesondere für Basen der Formeln (VI) und (VII). Besonders bevorzugte derartige Alkali- oder Erdalkalihalogenide sind Lithiumchlorid und Magnesiumchlorid, ganz besonders bevorzugt ist Lithiumchlorid. Erfindungsgemäß ganz besonders bevorzugte metallorganische Basen sind demnach TMP ZnCl. LiCl oder (TMP)₂ Zn·2LiCl oder (TMP)₂Zn·2LiCl 2 MgCl₂ (Ausgestaltung B-6). Am meisten bevorzugt ist TMP ZnCl·LiCl (VIII; Ausgestaltung B-7).

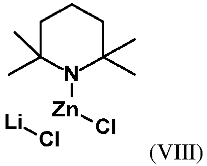

Beispielhaft werden in nachfolgender Tabelle 1 konkrete Kombinationen von Verbindungen der Formeln (I), (II) und (IVa) bzw. (IVb) mit erfindungsgemäßen Basen genannt, die in einem erfindungsgemäßen Verfahren zur Anwendung kommen können. Da in einigen Ausgestaltungen das Strukturelement R^{c} sowohl in der erfindungsgemäßen Base als auch in der Verbindung der Formel (IVa) vorliegt, gilt für R^{c} jeweils die engste Definition.

**Tabelle 1:**

| Nummer | Verbindungen der Formeln (I), (II) und (IVa) bzw. (IVb) | Base gemäß |
|---|---|---|
| 1 | Ausgestaltung 1 | Ausgestaltung B-1 |
| 2 | Ausgestaltung 1 | Ausgestaltung B-2 |
| 3 | Ausgestaltung 1 | Ausgestaltung B-3 |
| 4 | Ausgestaltung 1 | Ausgestaltung B-4 |
| 5 | Ausgestaltung 1 | Ausgestaltung B-5 |
| 6 | Ausgestaltung 1 | Ausgestaltung B-6 |
| 7 | Ausgestaltung 1 | Ausgestaltung B-7 |
| 8 | Ausgestaltung 2 | Ausgestaltung B-1 |
| 9 | Ausgestaltung 2 | Ausgestaltung B-2 |
| 10 | Ausgestaltung 2 | Ausgestaltung B-3 |
| 11 | Ausgestaltung 2 | Ausgestaltung B-4 |
| 12 | Ausgestaltung 2 | Ausgestaltung B-5 |
| 13 | Ausgestaltung 2 | Ausgestaltung B-6 |
| 14 | Ausgestaltung 2 | Ausgestaltung B-7 |
| 15 | Ausgestaltung 3 | Ausgestaltung B-1 |
| 16 | Ausgestaltung 3 | Ausgestaltung B-2 |
| 17 | Ausgestaltung 3 | Ausgestaltung B-3 |
| 18 | Ausgestaltung 3 | Ausgestaltung B-4 |
| 19 | Ausgestaltung 3 | Ausgestaltung B-5 |
| 20 | Ausgestaltung 3 | Ausgestaltung B-6 |
| 21 | Ausgestaltung 3 | Ausgestaltung B-7 |
| 22 | Ausgestaltung 4 | Ausgestaltung B-1 |
| 23 | Ausgestaltung 4 | Ausgestaltung B-2 |
| 24 | Ausgestaltung 4 | Ausgestaltung B-3 |
| 25 | Ausgestaltung 4 | Ausgestaltung B-4 |
| 26 | Ausgestaltung 4 | Ausgestaltung B-5 |
| 27 | Ausgestaltung 4 | Ausgestaltung B-6 |
| 28 | Ausgestaltung 4 | Ausgestaltung B-7 |
| 29 | Ausgestaltung 5 | Ausgestaltung B-1 |
| 30 | Ausgestaltung 5 | Ausgestaltung B-2 |
| 31 | Ausgestaltung 5 | Ausgestaltung B-3 |
| 32 | Ausgestaltung 5 | Ausgestaltung B-4 |
| 33 | Ausgestaltung 5 | Ausgestaltung B-5 |
| 34 | Ausgestaltung 5 | Ausgestaltung B-6 |
| 35 | Ausgestaltung 5 | Ausgestaltung B-7 |
| 36 | Ausgestaltung 6 | Ausgestaltung B-1 |
| 37 | Ausgestaltung 6 | Ausgestaltung B-2 |
| 38 | Ausgestaltung 6 | Ausgestaltung B-3 |
| 39 | Ausgestaltung 6 | Ausgestaltung B-4 |
| 40 | Ausgestaltung 6 | Ausgestaltung B-5 |
| 41 | Ausgestaltung 6 | Ausgestaltung B-6 |
| 42 | Ausgestaltung 6 | Ausgestaltung B-7 |

Vorzugsweise wird die metallorganische Base in dem erfindungsgemäßen Verfahren in einer Gesamtmenge von 0,5 bis 5 Äquivalenten, vorzugsweise von 0,8 bis 2 Äquivalenten, weiter bevorzugt von 1 bis 1,5 Äquivalenten und besonders bevorzugt von 1,0 bis 1,2 Äquivalenten, bezogen auf die Verbindung der Formel (I), eingesetzt. Ein Vorteil des erfindungsgemäßen Verfahrens ist es diesbezüglich, dass die metallorganische Base in nahezu stöchiometrischen Mengen eingesetzt werden kann.

In Abhängigkeit davon, ob in der eingesetzten Zink-metallorganischen Base das Strukturelement (NR^{a}R^{b}) einfach oder zweifach vorhanden ist, entstehen Zwischenverbindungen der Formel (IVa) beziehungsweise der Formel (IVb) in Verfahrensschritt a).

Die Umsetzung der Verbindungen der Formel (IVa) beziehungsweise (IVb) zu Verbindungen der Formel (II) im zweiten Verfahrensschritt (Schritt b)) erfolgt in Gegenwart einer Verbindung Q-V, in welcher Q und V jeweils die vorstehend genannten Bedeutungen haben. Da sowohl Q als auch V für Halogen stehen, handelt es sich um eine Interhalogenverbindung. Q und V müssen nicht notwendigerweise für das gleiche Halogen stehen. Beispielsweise kann Q für Iod oder Brom stehen und V für Chlor, Brom oder Iod. Vorzugsweise handelt es sich bei der Verbindung Q-V aber um ein elementares Halogen, insbesondere F₂, Cl₂, Br₂ oder I₂. Besonders bevorzugt sind I₂ oder Br₂, ganz besonders bevorzugt ist I₂.

Bevorzugt ist Q-V so gewählt, dass die Reste Z und Q für unterschiedliche Halogene stehen, besonders bevorzugt steht für den Fall, dass
Z für Fluor steht, Q für Iod, Brom oder Chlor oder
Z für Chlor steht, Q für Iod oder Brom oder
Z für Brom steht, Q für Iod.

Vorzugsweise wird die Verbindung Q-V in dem erfindungsgemäßen Verfahren in einer Gesamtmenge von 0,5 bis 10,0 Äquivalenten, vorzugsweise von 0,8 bis 5 Äquivalenten, weiter bevorzugt von 1 bis 2,5 Äquivalenten und besonders bevorzugt von 1,0 bis 1,5 Äquivalenten, bezogen auf die Verbindung der Formel (I), eingesetzt.

Die erfindungsgemäße Umsetzung der Verbindungen der Formel (I) zu Verbindungen der Formel (IVa) beziehungsweise (IVb) und weiter zu Verbindungen der Formel (II) erfolgt vorzugsweise jeweils in Gegenwart eines organischen Lösungsmittels. Als Lösungsmittel kommen prinzipiell alle organischen Lösungsmittel in Frage, die unter den angewendeten Reaktionsbedingungen inert sind und in denen die umzusetzenden Verbindungen eine ausreichende Löslichkeit aufweisen. Als geeignete Lösungsmittel sind insbesondere zu nennen: Tetrahydrofuran (THF), 1,4-Dioxan, Diethylether, Diglyme, Methyltertbutylether (MTBE), tert-Amyl-methylether (TAME), 2-Methyl-THF, Toluol, Xylole, Mesitylen, Ethylencarbonat, Propylencarbonat, N,N-Dimethylacetamid, N,N-Dimethylformamid (DMF), N-Methylpyrrolidon (NMP), N-Ethyl-2-pyrrolidon (NEP), N-Butyl-2-pyrrolidon (NBP); N,N'-Dimethylpropylenharnstoff (DMPU), Halogenkohlenwasserstoffe und aromatische Kohlenwasserstoffe, insbesondere Chlorkohlenwasserstoffe wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, insbesondere 1,2-Dichlorbenzol, Chlortoluol, Trichlorbenzol; 4-Methoxybenzol, fluorierte Aliphate und Aromaten wie Trichlortrifluorethan, Benzotrifluorid und 4-Chlorbenzotrifluorid.Es können auch Lösungsmittelgemische, vorzugsweise Gemische aus den vorstehend genannten Lösungsmitteln wie Tetrahydrofuran (THF), 1,4-Dioxan, Diethylether, Diglyme, Methyltertbutylether (MTBE), tert-Amyl-methylether (TAME), 2-Methyl-THF, Toluol, Xylole, Mesitylen, Dimethylformamid (DMF), eingesetzt werden.

Bevorzugte Lösungsmittel sind THF, N,N-Dimethylformamid (DMF), 1,4-Dioxan, Diglyme, Methyltertbutylether (MTBE), tert-Amyl-methylether (TAME), 2-Methyl-THF, Toluol und 4-Methoxybenzol.

Besonders bevorzugte Lösungsmittel sind THF und N,N-Dimethylformamid (DMF), ganz besonders bevorzugt ist THF.

Das Lösungsmittel kann ferner entgast (sauerstoff-frei) sein.

Vorzugsweise wird für beide Verfahrensschritte a) und b) das gleiche Lösungsmittel verwendet. Alternative erfindungsgemäße Ausgestaltungen, in denen für die Verfahrensschritte a) und b) unterschiedliche Lösungsmittel verwendet werden, sind allerdings ebenfalls möglich, wobei die Lösungsmittel dann ebenfalls vorzugsweise aus den vorstehend genannten Lösungsmitteln ausgewählt sind und die jeweiligen als bevorzugt, besonders bevorzugt und ganz besonders bevorzugt angegebenen Lösungsmittel auf den jeweiligen Verfahrensschritt a) oder b) zu beziehen sind.

Bei erhöhten Temperaturen ist der Umsatz des Verfahrensschrittes a) besonders gut. Die Umsetzung in Verfahrensschritt a) wird daher im Allgemeinen bei einer Temperatur zwischen 0 °C und 110 °C und zunehmend bevorzugt zwischen 20 °C und 100 °C, zwischen 30 °C und 95 °C, zwischen 40 °C und 90 °C, zwischen 60 °C und 85 °C und ganz besonders bevorzugt zwischen 70 °C und 85 °C, beispielsweise bei 80 °C, durchgeführt.

Die Umsetzung in Verfahrensschritt b) wird im Allgemeinen bei einer Temperatur zwischen 0 °C und 80 °C und zunehmend bevorzugt zwischen 10 °C und 70 °C, zwischen 15 °C und 60 °C, zwischen 20 °C und 50 °C, zwischen 20 °C und 40 °C und ganz besonders bevorzugt zwischen 20 °C und 35 °C, beispielsweise bei Raumtemperatur bzw. 25 °C, durchgeführt.

Die Reaktion wird üblicherweise bei Normaldruck durchgeführt, kann aber auch bei erhöhtem bzw. vermindertem Druck durchgeführt werden.

Die Isolierung der gewünschten Verbindungen der Formel (II) kann beispielsweise durch wässrige Aufarbeitung in Gegenwart von gesättigten Ammoniumchlorid- oder Natriumthiosulfat-Lösungen und/oder anschließende Chromatographie erfolgen. Solche Verfahren sind dem Fachmann bekannt und schließen auch eine Kristallisation aus einem organischen Lösungsmittel oder Lösungsmittelgemisch ein.

Eine besonders bevorzugte Ausführungsform des erfindungsgemäßen Verfahrens kann anhand des folgenden Schemas (II) erläutert werden:

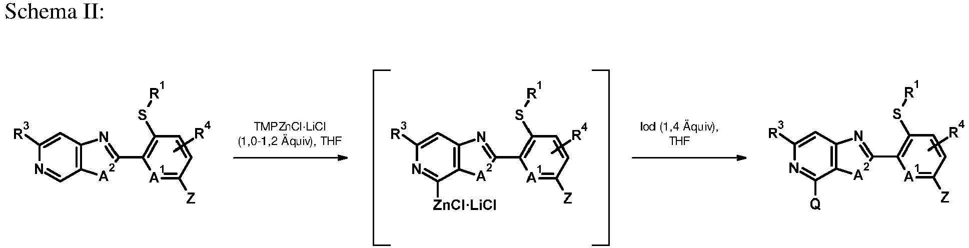

Hierin haben Q, V, Z, R¹, R³, R⁴, A¹ und A² die vorstehend angegebenen Bedeutungen. Die in Klammern angegebene Verbindung stellt die entsprechende Zwischenstufe der Formel (IVa) dar, welche weiter zum Produkt, einer Verbindung der Formel (II), umgesetzt wird. Beide Umsetzungen finden in THF als Lösungsmittel statt. "Äquiv" bezeichnet die eingesetzte Menge an Äquivalenten TMPZnCl·LiCl bzw. Iod (I₂).

In einer speziellen Ausführungsform der Erfindung werden die Verbindungen der Formel (II) in einem dritten Schritt c) weiter umgesetzt zu Verbindungen der Formel (III) 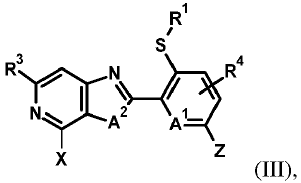 in welcher Z, R¹, R³, R⁴, A¹ und A² die oben genannten Bedeutungen und Vorzugsbereiche gemäß einer der Ausgestaltungen 1 bis 6 haben und
(Ausgestaltung (X-1))
- X: für Cyano, Carboxyl, Halogen, Nitro, Acetyl, Hydroxy, Amino, SCN, Tri-(C₁-C₆)alkylsilyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₃-C₆)Cycloalkyl, (C₃-C₆)Halogencycloalkyl, (C₃-C₆)Cyanocycloalkyl, (C₃-C₆)Cycloalkyl-(C₃-C₆)cycloalkyl, (C₁-C₄)Alkyl-(C₃-C₆)cycloalkyl (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkoxyimino, -N=C(H)-O(C₁-C₆)Alkyl, -C(H)=N-O(C₁-C₆)Alkyl, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkylaminosulfonyl, (C₁-C₆)Alkylsulfoximino, Aminothiocarbonyl, (C₁-C₆)Alkylaminothiocarbonyl, Di-(C₁-C₆)alkylaminothiocarbonyl, (C₃-C₈)Cycloalkylamino, (C₁-C₆)Alkylcarbamoyl (umfasst -NHCOO(C₁-C₆)alkyl, -N(C₁-C₆)alkylCOO(C₁-C₆)alkyl, -OCONH(C₁-C₆)alkyl oder -OCON(C₁-C₆)dialkyl), (C₁-C₆)Alkylcarbonylamino ((C₁-C₆)alkylCONH), (C₁-C₆)Alkylharnstoff (umfasst -NHCONH(C₁-C₆)alkyl, und -NHCON(C₁-C₆)dialkyl) oder einen gegebenenfalls einfach oder mehrfach, gleich oder verschieden substituierten gesättigten, teilgesättigten oder heteroaromatischen Ring, in dem mindestens ein C-Atom durch ein Heteroatom ersetzt ist oder einen einfach oder mehrfach, gleich oder verschieden substituierten aromatischen Ring steht, wobei jeweils gegebenenfalls mindestens eine Carbonylgruppe enthalten sein kann und wobei als Substituenten jeweils in Frage kommen: Cyano, Carboxyl, Halogen, Nitro, Acetyl, Hydroxy, Amino, SCN, Tri-(C₁-C₆)alkylsilyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₃-C₆)Cycloalkyl, (C₃-C₆)Halogencycloalkyl, (C₃-C₆)Cyanocycloalkyl, (C₃-C₆)Cycloalkyl-(C₃-C₆)cycloalkyl, (C₁-C₄)Alkyl-(C₃-C₆)cycloalkyl (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkoxyimino, -N=C(H)-O(C₁-C₆)Alkyl, -C(H)=N-O(C₁-C₆)Alkyl, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkylaminosulfonyl, (C₁-C₆)Alkylsulfoximino, Aminothiocarbonyl, (C₁-C₆)Alkylaminothiocarbonyl, Di-(C₁-C₆)alkylaminothiocarbonyl, (C₃-C₈)Cycloalkylamino oder (C₁-C₆)Alkylcarbonylamino.

Bevorzugt sind Verbindungen der Formel (III) in welchen Z, R¹, R³, R⁴, A¹ und A² die oben genannten Bedeutungen und Vorzugsbereiche gemäß einer der Ausgestaltungen 1 bis 6 haben und
(Ausgestaltung (X-2))
- X: bevorzugt für Cyano, Halogen, Nitro, Acetyl, Amino, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₃-C₆)Cycloalkyl, (C₃-C₆)Halogencycloalkyl, (C₃-C₆)Cyanocycloalkyl, (C₃-C₆)Cycloalkyl-(C₃-C₆)cycloalkyl, (C₁-C₄)Alkyl-(C₃-C₆)cycloalkyl, (C₁-C₄)Alkoxy, (C₁-C₄)Halogenalkoxy, (C₁-C₄)Cyanoalkoxy, (C₁-C₄)Alkoxy-(C₁-C₄)alkoxy, (C₁-C₄)Alkoxyimino,-N=C(H)-O(C₁-C₄)Alkyl, -C(H)=N-O(C₁-C₄)Alkyl, (C₁-C₄)Halogenalkyl-(C₁-C₄)alkoxyimino, (C₁-C₄)Alkylthio, (C₁-C₄)Halogenalkylthio, (C₁-C₄)Alkylthio-(C₁-C₄)alkyl, (C₁-C₄)Alkylsulfinyl, (C₁-C₄)Halogenalkylsulfinyl, (C₁-C₄)Alkylsulfinyl-(C₁-C₄)alkyl, (C₁-C₄)Alkylsulfonyl, (C₁-C₄)Halogenalkylsulfonyl, (C₁-C₄)Alkylsulfonyl-(C₁-C₄)alkyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl, Di-(C₁-C₄)alkylaminosulfonyl, (C₁-C₄)Alkylcarbamoyl (umfasst -NHCOO(C₁-C₄)alkyl, -N(C₁-C₄)alkylCOO(C₁-C₄)alkyl, -OCONH(C₁-C₄)alkyl oder -OCON(C₁-C₄)dialkyl), (C₁-C₄)Alkylcarbonylamino ((C₁-C₄)alkylCONH) oder (C₁-C₄)Alkylharnstoff (umfasst -NHCONH(C₁-C₆)alkyl und -NHCON(C₁-C₆)dialkyl) steht.

Besonders bevorzugt sind Verbindungen der Formel (III) in welchen Z, R¹, R³, R⁴, A¹ und A² die oben genannten Bedeutungen und Vorzugsbereiche gemäß einer der Ausgestaltungen 1 bis 6 haben und
(Ausgestaltung (X-3))
- X: für Cyano, Halogen, Nitro, Acetyl, Amino, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₃-C₆)Cycloalkyl, (C₃-C₆)Halogencycloalkyl, (C₃-C₆)Cyanocycloalkyl, (C₃-C₆)Cycloalkyl-(C₃-C₆)cycloalkyl, (C₁-C₄)Alkyl-(C₃-C₆)cycloalkyl, (C₁-C₄)Alkoxy, (C₁-C₄)Halogenalkoxy, (C₁-C₄)Cyanoalkoxy oder (C₁-C₄)Alkoxy-(C₁-C₄)alkoxy steht.

Ganz besonders bevorzugt sind Verbindungen der Formel (III) in welchen Z, R¹, R³, R⁴, A¹ und A² die oben genannten Bedeutungen und Vorzugsbereiche gemäß einer der Ausgestaltungen 1 bis 6 haben und
(Ausgestaltung (X-4))
- X: für Cyano, Halogen, Amino, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₃-C₆)Cycloalkyl, (C₃-C₆)Halogencycloalkyl, (C₃-C₆)Cyanocycloalkyl, (C₃-C₆)Cycloalkyl-(C₃-C₆)cycloalkyl, ((C₁-C₄)Alkoxy oder (C₁-C₄)Cyanoalkoxy steht.

Hervorgehoben sind Verbindungen der Formel (III) in welchen Z, R¹, R³, R⁴, A¹ und A² die oben genannten Bedeutungen und Vorzugsbereiche gemäß einer der Ausgestaltungen 1 bis 6 haben und
(Ausgestaltung (X-5-1))
- X: für Cyano, Halogen, (C₁-C₄)Alkyl, (C₃-C₆)Cycloalkyl, (C₂-C₄)Alkinyl, (C2-C4)Alkenyl, (C₃-C₆)Cyanocycloalkyl oder (C₃-C₆)Halogencycloalkyl steht.

Hervorgehoben sind weiterhin Verbindungen der Formel (III) in welchen Z, R¹, R³, R⁴, A¹ und A² die oben genannten Bedeutungen und Vorzugsbereiche gemäß einer der Ausgestaltungen 1 bis 6 haben und
(Ausgestaltung (X-5-2))
- X: für Cyano, Halogen, (C₁-C₄)Alkyl, (C₃-C₆)Cycloalkyl, (C₃-C₆)Cyanocycloalkyl oder (C₃-C₆)Halogencycloalkyl steht.

Insbesondere bevorzugt sind Verbindungen der Formel (III) in welchen Z, R¹, R³, R⁴, A¹ und A² die oben genannten Bedeutungen und Vorzugsbereiche gemäß einer der Ausgestaltungen 1 bis 6 haben und
(Ausgestaltung (X-6-1))
- X: für Cyano, Methyl, Ethyl, Vinyl, Ethinyl oder Chlor steht.

Insbesondere bevorzugt sind weiterhin Verbindungen der Formel (III) in welchen Z, R¹, R³, R⁴, A¹ und A² die oben genannten Bedeutungen und Vorzugsbereiche gemäß einer der Ausgestaltungen 1 bis 6 haben und
(Ausgestaltung (X-6-2))
- X: für Cyano, Methyl oder Chlor steht.

Die Umsetzung der Verbindungen der Formel (II) zu Verbindungen der Formel (III) (Schritt c), also die Einführung des Restes X, erfolgt bevorzugt durch Kupplung, besonders bevorzugt durch eine Kreuzkupplung oder eine Metall katalysierte Reaktionen.

Die Verbindungen der Formel (II) werden dabei bevorzugt mit metallorganischen Zink Reagenzien in Gegenwart eines Katalysators oder mit Metallsalzen, insbesondere Kupfersalzen umgesetzt.

Beispielsweise sind derartige Umsetzungen beschrieben in Chemistry, A European Journal 15 (2009) (metallorganische Zink Reagenzien) und Organic Letters, 13 (2011), 648-651 oder Organic & Biomolecular Chemistry, 7 (2009), 1043-1052 (Metallsalze).

Die erfindungsgemäße Umsetzung der Verbindungen der Formel (II) zu Verbindungen der Formel (III) erfolgt vorzugsweise jeweils in Gegenwart eines organischen Lösungsmittels. Als Lösungsmittel kommen prinzipiell alle organischen Lösungsmittel in Frage, die unter den angewendeten Reaktionsbedingungen inert sind und in denen die umzusetzenden Verbindungen eine ausreichende Löslichkeit aufweisen. Das Lösungsmittel kann ferner entgast (sauerstoff-frei) sein.

Vorzugsweise wird die metallorganische Zink Verbindung oder das Metallsalz in dem erfindungsgemäßen Verfahren in einer Gesamtmenge von 0,5 bis 10 Äquivalenten, bevorzugt 0,8 bis 5 Äquivalenten, besonders bevorzugt von 1 bis 3,0 Äquivalenten, bezogen auf die Verbindung der Formel (II), eingesetzt.

Die Umsetzung in Verfahrensschritt c) wird im Allgemeinen bei einer Temperatur zwischen 0 °C und 110 °C und zunehmend bevorzugt zwischen 10 °C und 100 °C, zwischen 20 °C und 90 °C und ganz besonders bevorzugt zwischen 30 °C und 90 °C durchgeführt.

Die Reaktion wird üblicherweise bei Normaldruck durchgeführt, kann aber auch bei erhöhtem bzw. vermindertem Druck durchgeführt werden.

### Einführung des Restes X über metallorganische Zink Reagenzien:

Die Verbindungen der Formel (III) lassen sich herstellen durch Negishi Kreuzkupplung mit metallorganischen Zink Reagenzien in Gegenwart eines Katalysators, wie in Chemistry, A European Journal 15 (2009), 1468-1477 beschrieben.

Vorzugsweise wird die metallorganische Zink Verbindung in dem erfindungsgemäßen Verfahren in einer Gesamtmenge von 0,5 bis 10 Äquivalenten, vorzugsweise von 0,8 bis 5 Äquivalenten, weiter bevorzugt von 1,0 bis 3,5 Äquivalenten und besonders bevorzugt von 1,5 bis 3,0 Äquivalenten, bezogen auf die Verbindung der Formel (II), eingesetzt. Ein Vorteil des erfindungsgemäßen Verfahrens ist es diesbezüglich, dass das metallorganische Zink Reagenz kommerziell erhältlich ist und sehr regio- und chemoselektiv eingesetzt werden kann.

Bevorzugte Zink Reagenzien sind solche der Formel YZnCl oder YZnBr, besonders bevorzugt YZnCl, wobei
Ausgestaltung (Y-1)
- Y: für (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₃-C₆)Cycloalkyl, (C₃-C₆)Halogencycloalkyl, (C₃-C₆)Cyanocycloalkyl, (C₃-C₆)Cycloalkyl-(C₃-C₆)cycloalkyl, (C₁-C₄)Alkyl-(C₃-C₆)cycloalkyl steht
und die Reagenzien auch komplexiert mit Lithiumchlorid und/oder Magnesiumchlorid vorliegen können.

Besonders bevorzugt steht (Ausgestaltung Y-2)
- Y: für (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₂-C₄)Alkenyl, (C₂-C₄)Alkinyl, (C₂-C₄)Halogenalkenyl, (C₂-C₄)Cyanoalkenyl, (C₃-C₆)Cycloalkyl, (C₃-C₆)Halogencycloalkyl, (C₃-C₆)Cyanocycloalkyl oder (C₁-C₄)Alkyl-(C₃-C₆)cycloalkyl.

Ganz besonders bevorzugt steht (Ausgestaltung Y-3) Y für (C₁-C₄)Alkyl, (C₂-C₄)Alkenyl, (C₂-C₄)Alkinyl oder (C₃-C₆)Cycloalkyl.

Insbesondere steht (Ausgestaltung Y-4) Y für Methyl, Ethyl, Propyl, Butyl, Vinyl, Ethinyl oder Cyclopropyl.

Die Umsetzung mit metallorganischen Zink Reagenzien in Verfahrensschritt c) erfolgt ferner bevorzugt in Gegenwart eines Katalysators. Vorzugsweise ist der Katalysator eine Palladiumverbindung oder eine Nickelverbindung. Besonders bevorzugt ist der Katalysator eine Palladiumverbindung. Ganz besonders bevorzugt handelt es sich um Tetrakis(triphenylphosphine)palladium(0), abgekürzt Pd(PPh₃)₄, der Formel (XI). 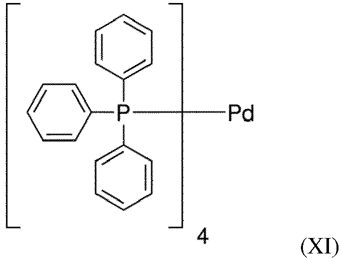 Üblicherweise wird bei der Umsetzung mit metallorganischen Zink Reagenzien in Schritt c) 2,5 bis 25 mol% und vorzugsweise 5 bis 20 mol% Katalysator eingesetzt.

Die erfindungsgemäße Umsetzung der Verbindungen der Formel (II) zu Verbindungen der Formel (III) mit metallorganischen Zink Reagenzien erfolgt vorzugsweise jeweils in Gegenwart eines organischen Lösungsmittels. Als geeignete Lösungsmittel sind insbesondere zu nennen: Tetrahydrofuran (THF), 1,4-Dioxan, Diethylether, Diglyme, Methyltertbutylether (MTBE), tert-Amyl-methylether (TAME), 2-Methyl-THF, Toluol, Xylole, Mesitylen, Ethylencarbonat, Propylencarbonat, N,N-Dimethylacetamid, N,N-Dimethylformamid (DMF), N-Methylpyrrolidon (NMP), N-Ethyl-2-pyrrolidon (NEP), N-Butyl-2-pyrrolidon (NBP); N,N'-Dimethylpropylenharnstoff (DMPU), Halogenkohlenwasserstoffe und aromatische Kohlenwasserstoffe, insbesondere Chlorkohlenwasserstoffe wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, insbesondere 1,2-Dichlorbenzol, Chlortoluol, Trichlorbenzol; 4-Methoxybenzol, fluorierte Aliphate und Aromaten wie Trichlortrifluorethan, Benzotrifluorid und 4-Chlorbenzotrifluorid. Es können auch Lösungsmittelgemische, vorzugsweise Gemische aus den vorstehend genannten Lösungsmitteln wie Tetrahydrofuran (THF), 1,4-Dioxan, Diethylether, Diglyme, Methyltertbutylether (MTBE), tert-Amyl-methylether (TAME), 2-Methyl-THF, Toluol, Xylole, Mesitylen, Dimethylformamid (DMF), eingesetzt werden.

Bevorzugte Lösungsmittel sind THF, N,N-Dimethylformamid (DMF), 1,4-Dioxan, Diglyme, Methyltertbutylether (MTBE), tert-Amyl-methylether (TAME), 2-Methyl-THF, Toluol und 4-Methoxybenzol.

Besonders bevorzugte Lösungsmittel sind THF und N,N-Dimethylformamid (DMF), ganz besonders bevorzugt ist THF.

Die Umsetzung mit metallorganischen Zink Reagenzien in Verfahrensschritt c) wird im Allgemeinen bei einer Temperatur zwischen 0 °C und 80 °C und zunehmend bevorzugt zwischen 5 °C und 75 °C, zwischen 10 °C und 70 °C, zwischen 20 °C und 60 °C, zwischen 25 °C und 65 °C und ganz besonders bevorzugt zwischen 30 °C und 50 °C, beispielsweise bei 40 °C, durchgeführt.

Die Umsetzung mit metallorganischen Zink Reagenzien in Verfahrensschritt c) wird im Allgemeinen mit einer Reaktionszeit zwischen 0,5 und 10 h und zunehmend bevorzugt zwischen 1 und 7 h, zwischen 1,5 und 6 h und ganz besonders bevorzugt zwischen 2 und 5 h, beispielsweise für 3 h, durchgeführt. Die Isolierung der gewünschten Verbindungen der Formel (III) kann beispielsweise durch wässrige Aufarbeitung in Gegenwart von gesättigten Ammoniumchlorid- oder Natriumthiosulfat-Lösungen und/oder anschließende Chromatographie erfolgen. Solche Verfahren sind dem Fachmann bekannt und schließen auch eine Kristallisation aus einem organischen Lösungsmittel oder Lösungsmittelgemisch ein.

### Einführung des Restes X über Metallsalze:

Die Verbindungen der Formel (III) lassen sich herstellen durch Negishi Kreuzkupplung mit Metallsalzen, wie in Organic Letters, 13 (2011), 648-651 oder Organic & Biomolecular Chemistry, 7 (2009), 1043-1052 beschrieben.

Vorzugsweise werden die Metallsalze in dem erfindungsgemäßen Verfahren in einer Gesamtmenge von 0,5 bis 10 Äquivalenten, vorzugsweise von 0,8 bis 5 Äquivalenten, weiter bevorzugt von 1 bis 2,5 Äquivalenten und besonders bevorzugt von 1,0 bis 1,5 Äquivalenten, bezogen auf die Verbindung der Formel (II), eingesetzt. Ein Vorteil des erfindungsgemäßen Verfahrens ist es diesbezüglich, dass die Metallsalze kommerziell erhältlich sind und sehr regio- und chemoselektiv eingesetzt werden können. Zudem kann auf die Zugabe eines Katalysators verzichtet werden.

Bevorzugte Metallsalze sind Kupfersalze und besonders bevorzugt handelt es sich um CuCN oder CuCl.

Die erfindungsgemäße Umsetzung der Verbindungen der Formel (II) zu Verbindungen der Formel (III) mit Metallsalzen erfolgt vorzugsweise jeweils in Gegenwart eines organischen Lösungsmittels. Als geeignete Lösungsmittel sind insbesondere zu nennen: Pyridin, Tetrahydrofuran (THF), 1,4-Dioxan, Diethylether, Diglyme, Methyltertbutylether (MTBE), tert-Amyl-methylether (TAME), 2-Methyl-THF, Toluol, Xylole, Mesitylen, Ethylencarbonat, Propylencarbonat, N,N-Dimethylacetamid, N,N-Dimethylformamid (DMF), N-Methylpyrrolidon (NMP), N-Ethyl-2-pyrrolidon (NEP), N-Butyl-2-pyrrolidon (NBP); N,N'-Dimethylpropylenharnstoff (DMPU), Halogenkohlenwasserstoffe und aromatische Kohlenwasserstoffe, insbesondere Chlorkohlenwasserstoffe wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, insbesondere 1,2-Dichlorbenzol, Chlortoluol, Trichlorbenzol; 4-Methoxybenzol, fluorierte Aliphate und Aromaten wie Trichlortrifluorethan, Benzotrifluorid und 4-Chlorbenzotrifluorid. Es können auch Lösungsmittelgemische, vorzugsweise Gemische aus den vorstehend genannten Lösungsmitteln wie Tetrahydrofuran (THF), 1,4-Dioxan, Diethylether, Diglyme, Methyltertbutylether (MTBE), tert-Amyl-methylether (TAME), 2-Methyl-THF, Toluol, Xylole, Mesitylen, Dimethylformamid (DMF), eingesetzt werden.

Bevorzugte Lösungsmittel sind Pyridin, THF, Dioxan und DMF.

Besonders bevorzugte Lösungsmittel sind Pyridin, THF und DMF, ganz besonders bevorzugt ist Pyridin.

Die Umsetzung mit Metallsalzen in Verfahrensschritt c) wird im Allgemeinen bei einer Temperatur zwischen 0 °C und 120 °C und zunehmend bevorzugt zwischen 30 °C und 110 °C, zwischen 40 °C und 100 °C, zwischen 50 °C und 90 °C, zwischen 60 °C und 95 °C und ganz besonders bevorzugt zwischen 70 °C und 90 °C, beispielsweise bei 80 °C, durchgeführt.

Die Umsetzung mit Metallsalzen in Verfahrensschritt c) wird im Allgemeinen mit einer Reaktionszeit zwischen 1 und 8 und zunehmend bevorzugt zwischen 2 und 7, zwischen 2,5 und 7,5 und ganz besonders bevorzugt zwischen 3 h und 6 h, beispielsweise für 5 h, durchgeführt. Die Isolierung der gewünschten Verbindungen der Formel (III) kann beispielsweise durch wässrige Aufarbeitung in Gegenwart von gesättigten Ammoniumchlorid- oder Natriumthiosulfat-Lösungen und/oder anschließende Chromatographie erfolgen. Solche Verfahren sind dem Fachmann bekannt und schließen auch eine Kristallisation aus einem organischen Lösungsmittel oder Lösungsmittelgemisch ein.

Ein weiterer Gegenstand der vorliegenden Erfindung sind Verbindungen der Formel (IVa) 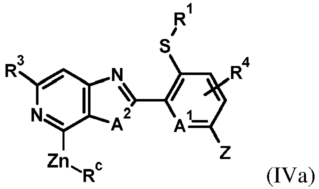 in welcher Z, R¹, R³, R⁴, R^{c}, A¹ und A² die oben genannten Bedeutungen und bevorzugten Ausgestaltungen gemäß einer der Ausgestaltungen 1 bis 6 haben.

Die Verbindungen der Formel (IVa) können auch mit Salzen komplexiert vorliegen, wobei es sich bei den Salzen bevorzugt um Alkali- oder Erdalkalihalogenide handelt, vorzugsweise um Lithiumchlorid und/oder Magnesiumchlorid und besonders bevorzugt um Lithiumchlorid.

Unter den Verbindungen der Formel (IVa) sind die folgenden Verbindungen ganz besonders bevorzugt, wobei die jeweilige Verbindung alleine oder als Lithiumchloridkomplex vorliegen kann:

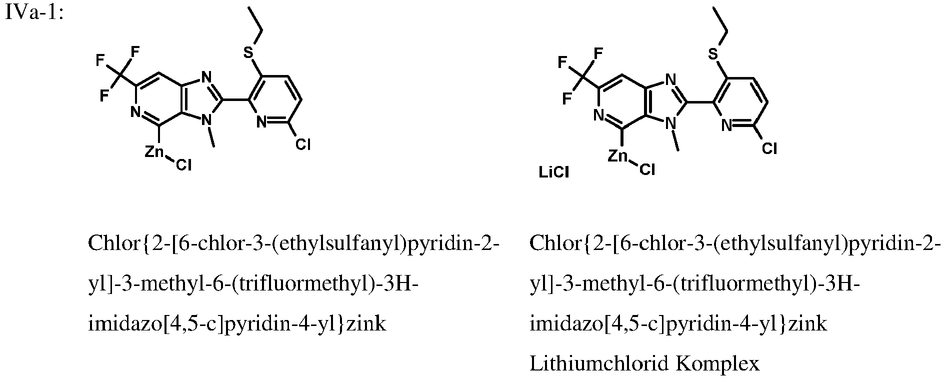

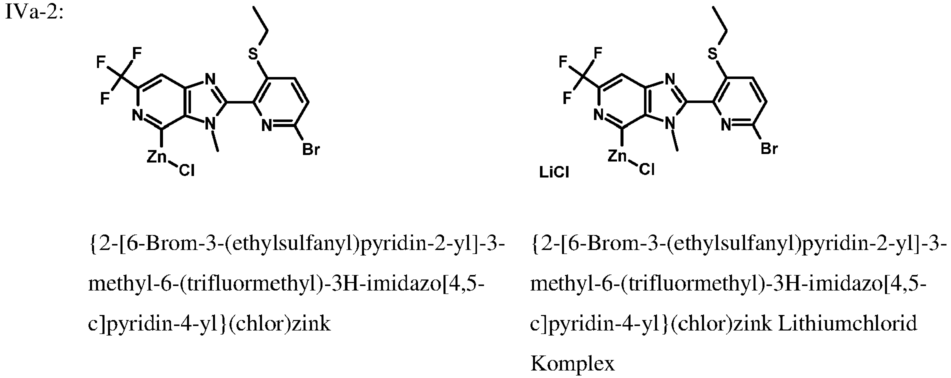

Ein weiterer Gegenstand der vorliegenden Erfindung sind Verbindungen der Formel (IVb) 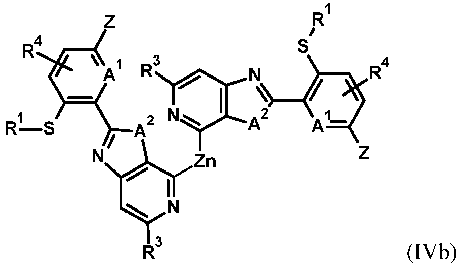 in welcher Z, R¹, R³, R⁴, A¹ und A² die oben genannten Bedeutungen und bevorzugten Ausgestaltungen haben.

Die Verbindungen der Formel (IVb) können auch mit Salzen komplexiert vorliegen, wobei es sich bei den Salzen bevorzugt um Alkali- oder Erdalkalihalogenide handelt, vorzugsweise um Lithiumchlorid und/oder Magnesiumchlorid, wie in Struktur (IVb-1) oder (IVb-2) und besonders bevorzugt um Lithiumchlorid (Struktur (IVb-1).

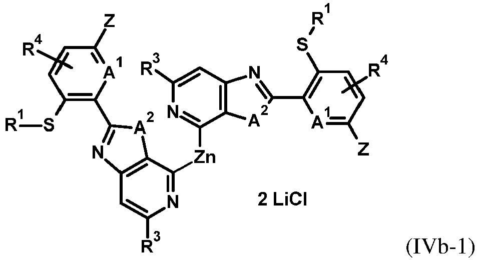

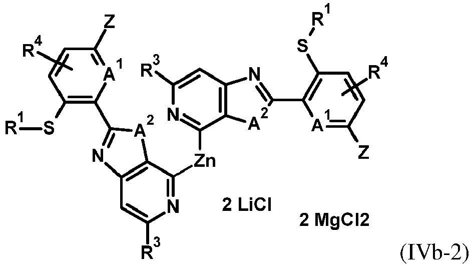

Z, R¹, R³, R⁴, A¹ und A² haben in Formel (IVb-1) und (IVb-2) die oben genannten Bedeutungen und bevorzugten Ausgestaltungen.

Ein weiterer Gegenstand der vorliegenden Erfindung sind Verbindungen der Formel (II) 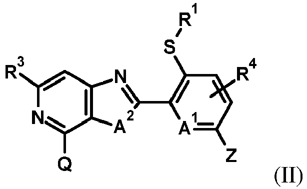 in welcher Q, Z, R¹, R³, R⁴, A¹ und A² die oben genannten Bedeutungen und bevorzugten Ausgestaltungen haben.

Unter den Verbindungen der Formel (II) sind die folgenden Verbindungen ganz besonders bevorzugt:

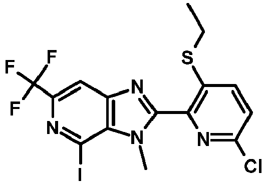

### ((II-1),2-[6-Chlor-3-(ethylsulfanyl)pyridin-2-yl]-4-iod-3-methyl-6-(trifluormethyl)-3H-imidazo[4,5-c]pyridin)

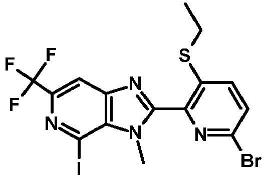

### ((II-2), 2-[6-Brom-3-(ethylsulfanyl)pyridin-2-yl]-4-iod-3-methyl-6-(trifluormethyl)-3H-imidazo[4,5-c]pyridin)

Aus diesen Verbindungen der Formeln (II-1) bis (II-2) ergeben sich die zugehörigen Edukte (1-1 bis 1-2) der Formel (I) des erfindungsgemäßen Verfahrens, welche jeweils ganz besonders bevorzugte Verbindungen der Formel (I) sind.

Ein weiterer Gegenstand der vorliegenden Offenbarung sind Verbindungen der Formel (III) 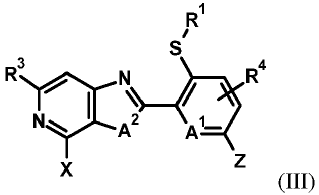 in welcher Z, R¹, R³, R⁴, A¹ und A² die oben genannten Bedeutungen und bevorzugten Ausgestaltungen gemäß einer der Ausgestaltungen 1 bis 6 haben und X die oben genannten Bedeutungen gemäß einer der Ausgestaltungen X-1 bis X-6 hat.

Unter den Verbindungen der Formel (III) sind die folgenden Verbindungen ganz besonders bevorzugt:

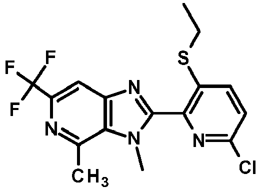

### ((III-1), 2-[6-Chlor-3-(ethylsulfanyl)pyridin-2-yl]-3,4-dimethyl-6-(trifluormethyl)-3H-imidazo[4,5-c]pyridin)

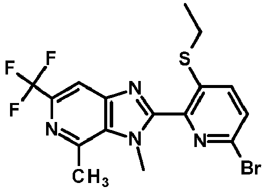

### ((III-2), 2-[6-Brom-3-(ethylsulfanyl)pyridin-2-yl]-3,4-dimethyl-6-(trifluormethyl)-3H-imidazo[4,5-c]pyridin)

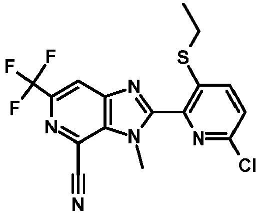

### ((III-3), 2-[6-Chlor-3-(ethylsulfanyl)pyridin-2-yl]-3-methyl-6-(trifluormethyl)-3H-imidazo[4,5-c]pyridin-4-carbonitril)

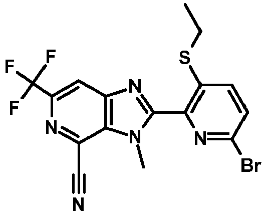

### ((III-4), 2-[6-Brom-3-(ethylsulfanyl)pyridin-2-yl]-3-methyl-6-(trifluormethyl)-3H-imidazo[4,5-c]pyridin-4-carbonitril)

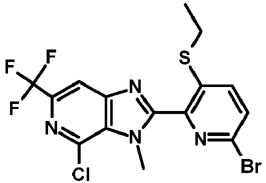

### ((III-5), 2-[6-Brom-3-(ethylsulfanyl)pyridin-2-yl]-4-chlor-3-methyl-6-(trifluormethyl)-3H-imidazo[4,5-c]pyridin)

Die vorliegende Erfindung wird anhand der nachfolgenden Beispiele näher erläutert, wobei die Beispiele nicht in einer die Erfindung einschränkenden Weise zu interpretieren sind.

### Methoden:

Die Messung der logP Werte erfolgt gemäß EEC Directive 79/831 Annex V.A8 durch HPLC (High Performance Liquid Chromatography) an einer Phasenumkehrsäule (C 18). Temperatur: 55°C.

Die Bestimmung mit der LC-MS im sauren Bereich erfolgt bei pH 2,7 mit 0,1% wässriger Ameisensäure und Acetonitril (enthält 0,1% Ameisensäure) als Eluenten; linearer Gradient von 10% Acetonitril bis 95% Acetonitril.

Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10% Acetonitril bis 95% Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die NMR-Daten ausgewählter Beispiele werden in klassischer Form (δ-Werte, Multiplettaufspaltung, Anzahl der H-Atome) aangegeben.

Das Lösungsmittel, in welchem das NMR-Spektrum aufgenommen wurde ist jeweils angegeben.

### Beispiel 1

### Synthese von 2-[6-Chlor-3-(ethylsulfanyl)pyridin-2-yl]-4-iod-3-methyl-6-(trifluormethyl)-3H-imidazo[4,5-c]pyridin:

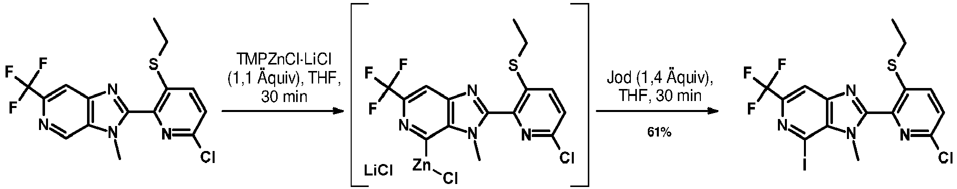

In einem trockenen, mit Argon befüllten 25-mL-Schlenk-Kolben, ausgestattet mit einem Magnetrührfisch und einem Septum, wurde 2-[6-Chlor-3-(ethylsulfanyl)pyridin-2-yl]-3-methyl-6-(trifluormethyl)-3H-imidazo[4,5-c]pyridin (628 mg, 1,42 mmol) in wasserfreiem THF (3 mL) vorgelegt. Zinkchlorid-2,2,6,6-tetramethylpiperidin-l-id Lithiumchlorid Komplex (TMPZnCl·LiCl) (1,31 M in THF, 1,2 mL, 1,56 mmol) wurde tropfenweise zugegeben, und die Mischung wurde 30 min bei 80 °C gerührt. Die Reaktionsmischung wurde auf 25 °C abgekühlt, dann wurde Iod (507 mg, 1.99 mmol) zugegeben, und anschließend wurde 30 min bei 25 °C gerührt. Die Reaktionsmischung wurde mit gesättigter wässriger Ammoniumchlorid-Lösung (25 mL) und Natriumthiosulfat-Lösung (25 mL) versetzt, mit Ethylacetat (3 ^{∗} 50 mL) extrahiert und über wasserfreiem Natriumsulfat getrocknet. Nach der Filtration wurde das Lösungsmittel im Vakuum entfernt. Das Rohprodukt wurde chromatographisch gereinigt, was 2-[6-Chlor-3-(ethylsulfanyl)pyridin-2-yl]-4-iod-3-methyl-6-(trifluormethyl)-3H-imidazo[4,5-c]pyridin (464 mg, 61%) als weißen Feststoff ergab.
HPLC-MS: logP =4,17; Masse (m/z): 499; ¹H-NMR (d₆-DMSO): δ 8,32 (s, 1H), 8,16 (d, 1H), 7,80 (d, 1H), 4,06 (s, 3H), 3,05 (q, 2H), 1,20 (t, 3H).

### Beispiel 2

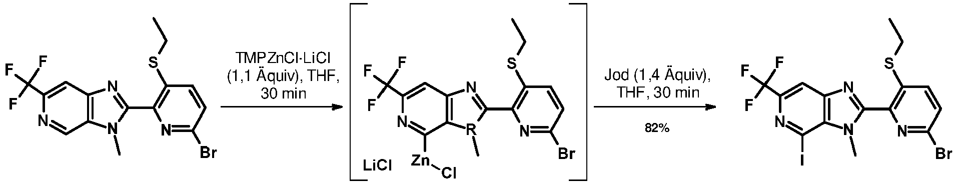

### Synthese von 2-[6-Brom-3-(ethylsulfanyl)pyridin-2-yl]-4-iod-3-methyl-6-(trifluormethyl)-3H-imidazo[4,5-c]pyridin:

In einem trockenen, mit Argon befüllten 25-mL-Schlenk-Kolben, ausgestattet mit einem Magnetrührfisch und einem Septum, wurde 2-[6-Brom-3-(ethylsulfanyl)pyridin-2-yl]-3-methyl-6-(trifluormethyl)-3H-imidazo[4,5-c]pyridin (500 mg, 1,19 mmol) in wasserfreiem THF (3 mL) vorgelegt. Zinkchlorid-2,2,6,6-tetramethylpiperidin-1-id Lithiumchlorid Komplex (TMPZnCl·LiCl) (1,31 M in THF, 1,0 mL, 1,31 mmol) wurde tropfenweise zugegeben, und die Mischung wurde 30 min bei 80 °C gerührt. Die Reaktionsmischung wurde auf 25 °C abgekühlt, dann wurde Iod (426 mg, 1.67 mmol) zugegeben, und anschließend wurde 30 min bei 25 °C gerührt. Die Reaktionsmischung wurde mit gesättigter wässriger Ammoniumchlorid-Lösung (25 mL) und Natriumthiosulfat-Lösung (25 mL) versetzt, mit Ethylacetat (3 ^{∗} 50 mL) extrahiert und über wasserfreiem Natriumsulfat getrocknet. Nach der Filtration wurde das Lösungsmittel im Vakuum entfernt. Das Rohprodukt wurde chromatographisch gereinigt, was 2-[6-Brom-3-(ethylsulfanyl)pyridin-2-yl]-4-iod-3-methyl-6-(trifluormethyl)-3H-imidazo[4,5-c]pyridin (302 mg, 82%) als weißen Feststoff ergab.
HPLC-MS: logP = 4,34; Masse (m/z): 543; ¹H-NMR (d₆-DMSO): δ 8,32 (s, 1H), 8,04 (d, 1H), 7,90 (d, 1H), 4,06 (s, 3H), 3,04 (q, 2H), 1,20 (t, 3H).

### Beispiel 3 (Schritt c)

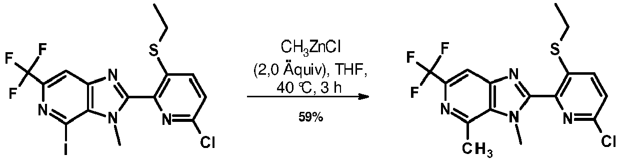

### Synthese von 2-[6-Chlor-3-(ethylsulfanyl)pyridin-2-yl]-3,4-dimethyl-6-(trifluormethyl)-3H-imidazo[4,5-c]pyridin:

2-[6-Chlor-3-(ethylsulfanyl)pyridin-2-yl]-4-iod-3-methyl-6-(trifluormethyl)-3H-imidazo[4,5-c]pyridin (700 mg, 1,4 mmol), gelöst in THF (4 ml), wurde mit MeZnCl (2,0 M in THF, 1,41 ml, 2,8 mmol) in Gegenwart von Tetrakis(triphenylphosphin)palladium(0) (163 mg, 0,14 mmol) bei 40 °C unter Argon versetzt; diese Reaktionslösung wurde 3 Stunden gerührt. Nach üblicher Aufarbeitung durch Zugabe von gesättigter Ammoniumchlorid- Lösung wurde das Reaktionsgemisch mit Ethylacetat extrahiert, die vereinigten organischen Phasen über Na₂SO₄ getrocknet und im Membranpumpenvakuum eingeengt. Nach säulenchromatographischer Reinigung (Ethylacetat/Cyclohexan) erhält man 2-[6-Chlor-3-(ethylsulfanyl)pyridin-2-yl]-3,4-dimethyl-6-(trifluormethyl)-3H-imidazo[4,5-c]pyridin (344 mg, 59%) als gelben Feststoff.
HPLC-MS: logP = 3,23; Masse (m/z): 387; ¹H-NMR (d₆-DMSO): δ 8,14 (d, 1H), 8,08 (s, 1H), 7,76 (d, 1H), 4,04 (s, 3H), 3,05 (q, 2H), 3,04 (s, 3H), 1,21 (t, 3H).

### Beispiel 4 (Schritt c)

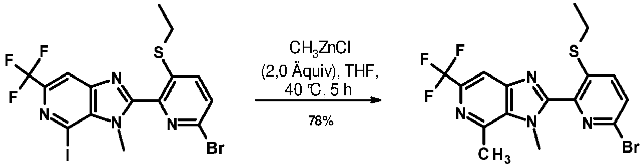

### Synthese von 2-[6-Brom-3-(ethylsulfanyl)pyridin-2-yl]-3,4-dimethyl-6-(trifluormethyl)-3H-imidazo[4,5-c]pyridin:

2-[6-Brom-3-(ethylsulfanyl)pyridin-2-yl]-4-iod-3-methyl-6-(trifluormethyl)-3H-imidazo[4,5-c]pyridin (310 mg, 0,57 mmol), gelöst in THF (3 ml), wurde mit MeZnCl (2,0 M in THF, 0,57 ml, 1,14 mmol) in Gegenwart von Tetrakis(triphenylphosphin)palladium(0) (66 mg, 0,05 mmol) bei 40 °C unter Argon versetzt; diese Reaktionslösung wurde 5 Stunden gerührt. Nach üblicher Aufarbeitung durch Zugabe von gesättigter Ammoniumchlorid- Lösung wurde das Reaktionsgemisch mit Ethylacetat extrahiert, die vereinigten organischen Phasen über Na₂SO₄ getrocknet und im Membranpumpenvakuum eingeengt. Nach säulenchromatographischer Reinigung (Ethylacetat/Cyclohexan) erhält man 2-[6-Brom-3-(ethylsulfanyl)pyridin-2-yl]-3,4-dimethyl-6-(trifluormethyl)-3H-imidazo[4,5-c]pyridin (190 mg, 78%) als gelben Feststoff.
HPLC-MS: logP = 3,36; Masse (m/z): 431; ¹H-NMR (d₆-DMSO): δ 8,08 (s, 1H), 8,02 (d, 1H), 7,87 (d, 1H), 4,04 (s, 3H), 3,03 (q, 2H), 3,02 (s, 3H), 1,19 (t, 3H).

### Beispiel 5 (Schritt c)

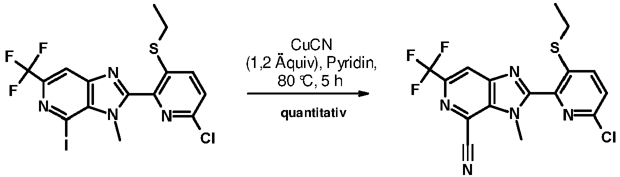

### Synthese von 2-[6-Chlor-3-(ethylsulfanyl)pyridin-2-yl]-3-methyl-6-(trifluormethyl)-3H-imidazo[4,5-c]pyridin-4-carbonitril:

2-[6-Chlor-3-(ethylsulfanyl)pyridin-2-yl]-4-iod-3-methyl-6-(trifluormethyl)-3H-imidazo[4,5-c]pyridin (100 mg, 0,20 mmol), gelöst in Pyridin (2 ml), wurde mit CuCN (22 mg, 0,24 mmol) bei 80 °C unter Argon versetzt; diese Reaktionslösung wurde 5 Stunden gerührt. Nach üblicher Aufarbeitung durch Zugabe von gesättigter Natriumchlorid- und Ammoniumchlorid- Lösungen wurde das Reaktionsgemisch mit Ethylacetat extrahiert, die vereinigten organischen Phasen über Na₂SO₄ getrocknet und im Membranpumpenvakuum eingeengt. Nach säulenchromatographischer Reinigung (Ethylacetat/Cyclohexan) erhält man 2-[6-Chlor-3-(ethylsulfanyl)pyridin-2-yl]-3-methyl-6-(trifluormethyl)-3H-imidazo[4,5-c]pyridin-4-carbonitril (99 mg, quantitativ) als gelben Feststoff.
HPLC-MS: logP = 3,84; Masse (m/z): 398; ¹H-NMR (d₆-DMSO): δ 8,73 (s, 1H), 8,18 (d, 1H), 7,79 (d, 1H), 4,14 (s, 3H), 3,05 (q, 2H), 1,21 (t, 3H).

### Beispiel 6 (Schritt c)

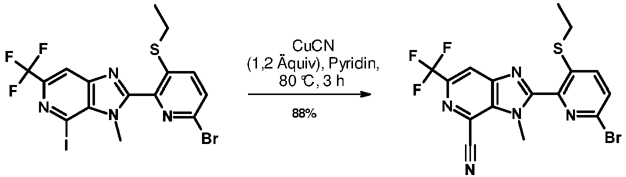

### Synthese von 2-[6-Brom-3-(ethylsulfanyl)pyridin-2-yl]-3-methyl-6-(trifluormethyl)-3H-imidazo[4,5-c]pyridin-4-carbonitril:

2-[6-Brom-3-(ethylsulfanyl)pyridin-2-yl]-3-methyl-6-(trifluormethyl)-3H-imidazo[4,5-c]pyridin (752 mg, 1,03 mmol), gelöst in Pyridin (4 ml), wurde mit CuCN (112 mg, 1,24 mmol) bei 80 °C unter Argon versetzt; diese Reaktionslösung wurde 3 Stunden gerührt. Nach üblicher Aufarbeitung durch Zugabe von gesättigter Natriumchlorid- und Ammoniumchlorid- Lösungen wurde das Reaktionsgemisch mit Ethylacetat extrahiert, die vereinigten organischen Phasen über Na₂SO₄ getrocknet und im Membranpumpenvakuum eingeengt. Nach säulenchromatographischer Reinigung (Ethylacetat/Cyclohexan) erhält man 2-[6-Brom-3-(ethylsulfanyl)pyridin-2-yl]-3-methyl-6-(trifluormethyl)-3H-imidazo[4,5-c]pyridin-4-carbonitril (473 mg, 88%) als gelben Feststoff.
HPLC-MS: logP = 3,87; Masse (m/z): 442; ¹H-NMR (d₆-DMSO): δ 8,73 (s, 1H), 8,06 (d, 1H), 7,91 (d, 1H), 4,14 (s, 3H), 3,06 (q, 2H), 1,21 (t, 3H).

### Beispiel 7 (Schritt c)

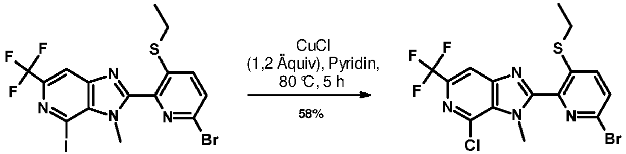

### Synthese von 2-[6-Brom-3-(ethylsulfanyl)pyridin-2-yl]-4-chlor-3-methyl-6-(trifluormethyl)-3H-imidazo[4,5-c]pyridin:

2-[6-Brom-3-(ethylsulfanyl)pyridin-2-yl]-3-methyl-6-(trifluormethyl)-3H-imidazo[4,5-c]pyridin (258 mg, 0,47 mmol), gelöst in Pyridin (2 ml), wurde mit CuCl (57 mg, 0,57 mmol) bei 80 °C unter Argon versetzt; diese Reaktionslösung wurde 5 Stunden gerührt. Nach üblicher Aufarbeitung durch Zugabe von gesättigter Natriumchlorid- und Ammoniumchlorid- Lösungen wurde das Reaktionsgemisch mit Ethylacetat extrahiert, die vereinigten organischen Phasen über Na₂SO₄ getrocknet und im Membranpumpenvakuum eingeengt. Nach säulenchromatographischer Reinigung (Ethylacetat/Cyclohexan) erhält man 2-[6-Brom-3-(ethylsulfanyl)pyridin-2-yl]-4-chlor-3-methyl-6-(trifluormethyl)-3H-imidazo[4,5-c]pyridin (209 mg, 58%) als gelben Feststoff.
HPLC-MS: logP = 4,18; Masse (m/z): 452; ¹H-NMR (d₆-DMSO): δ 8,39 (s, 1H), 8,07 (d, 1H), 7,90 (d, 1H), 4,07 (s, 3H), 3,04 (q, 2H), 1,20 (t, 3H).

### Beispiele für weitere mögliche Umsetzungen der Verbindungen der Formel (III) zu als Pestizide wirksamen Verbindungen

### Oxidation

### Synthese von 2-[6-Chlor-3-(ethylsulfonyl)pyridin-2-yl]-3-methyl-6-(trifluormethyl)-3H-imidazo[4,5-c]pyridin-4-carbonitril:

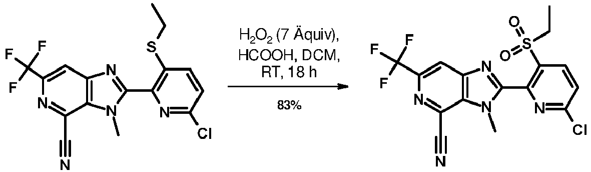

In einem Kolben, ausgestattet mit einem Magnetrührfisch, wurde 2-[6-Chlor-3-(ethylsulfanyl)pyridin-2-yl]-3-methyl-6-(trifluormethyl)-3H-imidazo[4,5-c]pyridin-4-carbonitril (99 mg, 0,24 mmol) in Dichlormethan (2 mL) vorgelegt. Ameisensäure (57 mg, 1,24 mmol) und Wasserstoffperoxid 35%ig (169 mg, 1,73 mmol) wurden anschließend zugegeben, und die Mischung wurde übernacht bei 25 °C gerührt. Die Reaktionsmischung wurde auf 25 °C abgekühlt, dann wurde eine verdünnte HCl Lösung zugegeben (bis pH 3-4). Die Reaktionsmischung wurde mit etwas Wasser und einer Natriumbisulfit Lösung versetzt, anschließend mit einer Natriumchlorid Lösung und 20%iger Natriumbicarbonat Lösung, mit Dichlormethan (3 ^{∗} 50 mL) extrahiert und über wasserfreiem Natriumsulfat getrocknet. Nach der Filtration wurde das Lösungsmittel im Vakuum entfernt. Das Rohprodukt wurde chromatographisch gereinigt, was 2-[6-Chlor-3-(ethylsulfonyl)pyridin-2-yl]-3-methyl-6-(trifluormethyl)-3H-imidazo[4,5-c]pyridin-4-carbonitril (94 mg, 83%) als weißen Feststoff ergab.
MH⁺: 430; ¹H-NMR (400 MHz, d6-DMSO) δ ppm: 1,22 (t, 3H), 3,71 (q, 2H), 4,06 (s, 3H), 8,17 (d, 1H), 8,59 (d, 1H), 8,76 (s, 1H).

### Kreuzkupplung

### Synthese von 2-[3-(Ethylsulfonyl)-6-(1H-1,2,4-triazol-1-yl)pyridin-2-yl]-3-methyl-6-(trifluormethyl)-3H-imidazo[4,5-c]pyridin-4-carbonitril (Verbindung Nr. 1):

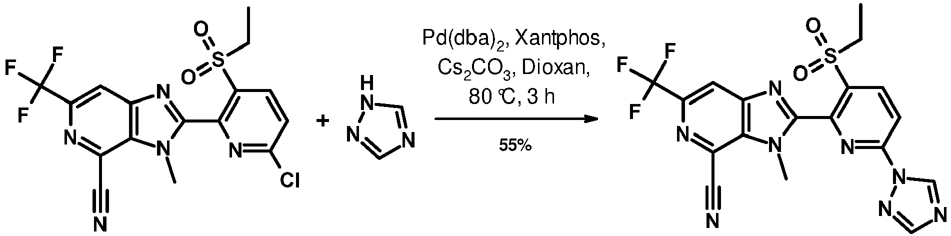

In einem trockenen, mit Argon befüllten 25-mL-Schlenk-Kolben, ausgestattet mit einem Magnetrührfisch und einem Septum, wurde 2-[6-Chlor-3-(ethylsulfonyl)pyridin-2-yl]-3-methyl-6-(trifluormethyl)-3H-imidazo[4,5-c]pyridin-4-carbonitril (83,6 mg, 0,19 mmol) in wasserfreiem Dioxan (4 mL) vorgelegt. 1H-1,2,4-Triazol (16,1 mg, 0,23 mmol), Caesiumcarbonat (95,1 mg, 0,29 mmol), 9,9-Dimethyl-4,5-bis(diphenylphosphino)xanthene (11,3 mg, 0,02 mmol) und Bis(dibenzylidenaceton)-palladium(0) (5,6 mg, 0,01 mmol) wurden anschließend zugegeben, und die Mischung wurde 3 h bei 80 °C gerührt. Die Reaktionsmischung wurde mit gesättigter wässriger Natriumchlorid-Lösung (25 mL) versetzt, mit Ethylacetat (3 ^{∗} 50 mL) extrahiert und über wasserfreiem Natriumsulfat getrocknet. Nach der Filtration wurde das Lösungsmittel im Vakuum entfernt. Das Rohprodukt wurde chromatographisch gereinigt, was 2-[3-(Ethylsulfonyl)-6-(1H-1,2,4-triazol-1-yl)pyridin-2-yl]-3-methyl-6-(trifluormethyl)-3H-imidazo[4,5-c]pyridin-4-carbonitril (52.4 mg, 55%) als weißen Feststoff ergab.
MH⁺: 463; ¹H-NMR (400 MHz, d6-DMSO) δ ppm: 1,23 (t, 3H), 3,84 (q, 2H), 4,19 (s, 3H), 8,37 (d, 1H), 8,475 (s, 1H), 8,79 (d, 1H), 8,80 (s, 1H), 9.52 (s, 1H).

Die Herstellung der Verbindungen Nr. 2 und 3 erfolgte analog der Verbindung Nr. 1.

| Verbindung Nr. | Strukur |
|---|---|
| 1 | 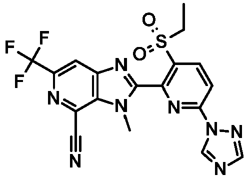 |
| 2 | 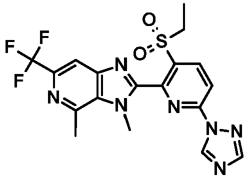 |
| 3 | 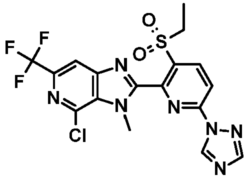 |

### Anwendungsbeispiele

### Myzus persicae - Oraltest

| | |
|---|---|
| Lösungsmittel: | 100 Gewichtsteile Aceton |

Zur Herstellung einer zweckmäßigen Wirkstoffzubereitung löst man 1 Gewichtsteil Wirkstoff mit den angegebenen Gewichtsteilen Lösungsmittel und füllt mit Wasser bis zum Erreichen der gewünschten Konzentration auf.

50 µl der Wirkstoffzubereitung werden in Mikrotiterplatten überführt und mit 150 µl IPL41Insektenmedium (33% + 15% Zucker) auf eine Endvolumen von 200 µl aufgefüllt. Anschließend werden die Platten mit Parafilm verschlossen, durch den eine gemischte Population der Grünen Pfirsichblattlaus (*Myzus persicae*)*,* die sich in einer zweiten Mikrotiterplatte befindet, hindurchstechen und die Lösung aufnehmen kann.

Nach 5 Tagen wird die Wirkung in % bestimmt. Dabei bedeutet 100%, dass alle Blattläuse abgetötet wurden; 0% bedeutet, dass keine Blattläuse abgetötet wurden.

Bei diesem Test zeigen z. B. die folgenden Verbindungen der Herstellungsbeispiele Wirkung von 100% bei einer Aufwandmenge von 4 ppm: 1, 2, 3

### Myzus persicae - Sprühtest

| | |
|---|---|
| Lösungsmittel: | 78 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (*Brassica pekinensis*)*,* die von allen Stadien der Grünen Pfirsichblattlaus (*Myzus persicae*) befallen sind, werden mit einer Wirkstoffzubereitung der gewünschten Konzentration gespritzt.

Nach 5 Tagen wird die Wirkung in % bestimmt. Dabei bedeutet 100%, dass alle Blattläuse abgetötet wurden; 0% bedeutet, dass keine Blattläuse abgetötet wurden.

Bei diesem Test zeigen z. B. die folgenden Verbindungen der Herstellungsbeispiele Wirkung von 100% bei einer Aufwandmenge von 100 g/ha: 3

Bei diesem Test zeigen z. B. die folgenden Verbindungen der Herstellungsbeispiele Wirkung von 100% bei einer Aufwandmenge von 20 g/ha: 3

Bei diesem Test zeigen z. B. die folgenden Verbindungen der Herstellungsbeispiele Wirkung von 90% bei einer Aufwandmenge von 20 g/ha: 2

### Phaedon cochleariae - Sprühtest

| | |
|---|---|
| Lösungsmittel: | 78,0 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (*Brassica pekinensis*) werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100%, dass alle Käferlarven abgetötet wurden; 0% bedeutet, dass keine Käferlarven abgetötet wurden.

Bei diesem Test zeigen z. B. die folgenden Verbindungen der Herstellungsbeispiele Wirkung von 83% bei einer Aufwandmenge von 100 g/ha: 1, 2

### Spodoptera frugiperda - Sprühtest

| | |
|---|---|
| Lösungsmittel: | 78,0 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach 7 Tagen wird die Wirkung in % bestimmt. Dabei bedeutet 100%, dass alle Raupen abgetötet wurden; 0% bedeutet, dass keine Raupe abgetötet wurde.

Bei diesem Test zeigen z. B. die folgenden Verbindungen der Herstellungsbeispiele Wirkung von 100% bei einer Aufwandmenge von 100 g/ha: 3

## Patentansprüche

1. Verfahren zur Herstellung von Verbindungen der Formel (II) 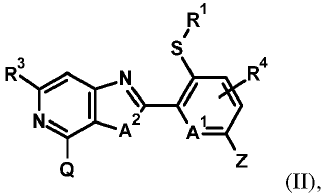 in welcher
Q und Z unabhängig voneinander für Halogen stehen,
A¹ für Stickstoff, =N⁺-O⁻ oder =C-R' steht,
A² für ―N-R⁶, Sauerstoff oder Schwefel steht,
R¹ für (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Halogenalkoxy-(C₁-C₆)alkyl, (C₂-C₆)Alkenyl, (C₂-C₆)Alkenyloxy-(C₁-C₆)alkyl, (C₂-C₆)Halogenalkenyloxy-(C₁-C₆)alkyl, (C₂-C₆)Halogenalkenyl, (C₂-C₆)Cyanoalkenyl, (C₂-C₆)Alkinyl, (C₂-C₆)Alkinyloxy-(C₁-C₆)alkyl, (C₂-C₆)Halogenalkinyloxy-(C₁-C₆)alkyl, (C₂-C₆)Halogenalkinyl, (C₂-C₆)Cyanoalkinyl, (C₃-C₈)Cycloalkyl, (C₃-C₆)Halogencycloalkyl, (C₃-C₆)Cyanocycloalkyl, (C₃-C₈)Cycloalkyl-(C₃-C₈)Cycloalkyl, (C₁-C₆)Alkyl-(C₃-C₈)Cycloalkyl, Halogen(C₃-C₈)cycloalkyl, Amino, (C₁-C₆)Alkylamino, Di-(C₁-C₆)alkyl-amino, (C₃-C₈)Cycloalkylamino, (C₁-C₆)Alkylcarbonyl-amino, (C₁-C₆)Alkylthio-(C₁-C₆)alkyl, (C₁-C₆)Halogenalkylthio-(C₁-C₆)alkyl, (C₁-C₆)Alkylsulfinyl-(C₁-C₆)alkyl, (C₁-C₆)Halogenalkylsulfinyl-(C₁-C₆)alkyl, (C₁-C₆)Alkylsulfonyl-(C₁-C₆)alkyl, (C₁-C₆)Halogenalkylsulfonyl-(C₁-C₆)alkyl, (C₁-C₆)Alkoxy-(C₁-C₆)alkylthio-(C₁-C₆)alkyl, (C₁-C₆)Alkoxy-(C₁-C₆)alkylsulfinyl-(C₁-C₆)alkyl, (C₁-C₆)Alkoxy-(C₁-C₆)alkylsulfonyl-(C₁-C₆)alkyl, (C₁-C₆)Alkylcarbonyl-(C₁-C₆)alkyl, (C₁-C₆)Halogenalkylcarbonyl-(C₁-C₆)alkyl, (C₁-C₆)Alkoxycarbonyl-(C₁-C₆)alkyl, (C₁-C₆)Halogenalkoxycarbonyl-(C₁-C₆)alkyl, (C₁-C₆)Alkylsulfonylamino, Aminosulfonyl-(C₁-C₆)alkyl, (C₁-C₆)Alkylaminosulfonyl-(C₁-C₆)alkyl, Di-(C₁-C₆)alkyl-aminosulfonyl-(C₁-C₆)alkyl,
oder für jeweils gegebenenfalls einfach oder mehrfach, gleich oder verschieden durch Aryl, Hetaryl oder Heterocyclyl substituiertes (C₁-C₆)Alkyl, (C₁-C₆)Alkoxy, (C₂-C₆)Alkenyl, (C₂-C₆)Alkinyl, (C₃-C₈)Cycloalkyl steht, wobei Aryl, Hetaryl oder Heterocyclyl jeweils gegebenenfalls einfach oder mehrfach, gleich oder verschieden durch Halogen, Cyano, Nitro, Hydroxy, Amino, Carboxy, Carbamoyl, Aminosulfonyl, (C₁-C₆)Alkyl, (C₃-C₆)Cycloalkyl, (C₁-C₆)Alkoxy, (C₁-C₆)Halogenalkyl, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfimino, (C₁-C₆)Alkylsulfimino-(C₁-C₆)alkyl, (C₁-C₆)Alkylsulfimino-(C₂-C₆)alkylcarbonyl, (C₁-C₆)Alkylsulfoximino, (C₁-C₆)Alkylsulfoximino-(C₁-C₆)alkyl, (C₁-C₆)Alkylsulfoximino-(C₂-C₆)alkylcarbonyl, (C₁-C₆)Alkoxycarbonyl, (C₁-C₆)Alkylcarbonyl, (C₃-C₆)Trialkylsilyl oder Benzyl substituiert sein können, R³ für Wasserstoff, Cyano, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, Cyano(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, -NHCO-(C₁-C₆)alkyl ((C₁-C₆)Alkylcarbonylamino) oder
für jeweils gegebenenfalls einfach oder mehrfach, gleich oder verschieden substituiertes Aryl oder Hetaryl steht, wobei (im Fall von Hetaryl) gegebenenfalls mindestens eine Carbonylgruppe enthalten sein kann und wobei als Substituenten jeweils in Frage kommen: Cyano, Carboxyl, Halogen, Nitro, Acetyl, Hydroxy, Amino, SCN, Tri-(C₁-C₆)alkylsilyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkylaminosulfonyl, (C₁-C₆)Alkylsulfoximino, Aminothiocarbonyl, (C₁-C₆)Alkylaminothiocarbonyl, Di-(C₁-C₆)alkylaminothiocarbonyl oder (C₃-C₈)Cycloalkylamino,
R⁴ für Wasserstoff, Cyano, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, Cyano(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino, NHCO-(C₁-C₆)alkyl ((C₁-C₆)Alkylcarbonylamino), oder
für jeweils gegebenenfalls einfach oder mehrfach, gleich oder verschieden substituiertes Aryl oder Hetaryl steht, wobei (im Fall von Hetaryl) gegebenenfalls mindestens eine Carbonylgruppe enthalten sein kann und wobei als Substituenten jeweils in Frage kommen: Cyano, Carboxyl, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkylaminosulfonyl, (C₁-C₆)Alkylsulfoximino, Aminothiocarbonyl, (C₁-C₆)Alkylaminothiocarbonyl, Di-(C₁-C₆)alkylaminothiocarbonyl, (C₃-C₈)Cycloalkylamino, (C₁-C₆)Alkylcarbonylamino,
R⁵ für Wasserstoff, Cyano, Halogen, Nitro, Acetyl, Hydroxy, Amino, SCN, Tri-(C₁-C₆)alkylsilyl, (C₃-C₈)Cycloalkyl, (C₃-C₈)Cycloalkyl-(C₃-C₈)Cycloalkyl, (C₁-C₆)Alkyl-(C₃-C₈)cycloalkyl, Halogen(C₃-C₈)cycloalkyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(Ci-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Alkylthiocarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, (C₁-C₆)Alkylaminothiocarbonyl, Di-(C₁-C₆)alkyl-aminocarbonyl, Di-(C₁-C₆)alkyl-aminothiocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl, (C₃-C₈)Cycloalkylamino oder -NHCO-(C₁-C₆)alkyl ((C₁-C₆)Alkylcarbonylamino) steht und
R⁶ für (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Halogenalkoxy-(C₁-C₆)alkyl, (C₂-C₆)Alkenyl, (C₂-C₆)Alkenyloxy-(C₁-C₆)alkyl, (C₂-C₆)Halogenalkenyloxy-(C₁-C₆)alkyl, (C₂-C₆)Halogenalkenyl, (C₂-C₆)Cyanoalkenyl, (C₂-C₆)Alkinyl, (C₂-C₆)Alkinyloxy-(C₁-C₆)alkyl, (C₂-C₆)Halogenalkinyloxy-(C₁-C₆)alkyl, (C₂-C₆)Halogenalkinyl, (C₂-C₆)Cyanoalkinyl, (C₃-C₈)Cycloalkyl, (C₃-C₈)Cycloalkyl-(C₃-C₈)Cycloalkyl, (C₁-C₆)Alkyl-(C₃-C₈)Cycloalkyl, Halogen(C₃-C₈)cycloalkyl, (C₁-C₆)Alkylthio-(C₁-C₆)alkyl, (C₁-C₆)Halogenalkylthio-(C₁-C₆)alkyl, (C₁-C₆)Alkylsulfinyl-(C₁-C₆)alkyl, (C₁-C₆)Halogenalkylsulfinyl-(C₁-C₆)alkyl, (C₁-C₆)Alkylsulfonyl-(C₁-C₆)alkyl, (C₁-C₆)Halogenalkylsulfonyl-(C₁-C₆)alkyl, (C₁-C₆)Alkoxy-(C₁-C₆)alkylthio-(C₁-C₆)alkyl, (C₁-C₆)Alkoxy-(C₁-C₆)alkylsulfinyl-(C₁-C₆)alkyl, (C₁-C₆)Alkoxy-(C₁-C₆)alkylsulfonyl-(C₁-C₆)alkyl, (C₁-C₆)Alkylcarbonyl-(C₁-C₆)alkyl, (C₁-C₆)Halogenalkylcarbonyl-(C₁-C₆)alkyl, (C₁-C₆)Alkoxycarbonyl-(C₁-C₆)alkyl, (C₁-C₆)Halogenalkoxycarbonyl-(C₁-C₆)alkyl, Aminocarbonyl-(C₁-C₆)alkyl, (C₁-C₆)Alkylamino-(C₁-C₆)alkyl, Di-(C₁-C₆)alkylamino-(C₁-C₆)alkyl oder (C₃-C₈)Cycloalkylamino-(C₁-C₆)alkyl steht,
**dadurch gekennzeichnet, dass** in einem ersten Verfahrensschritt a) eine Verbindung der Formel (I) 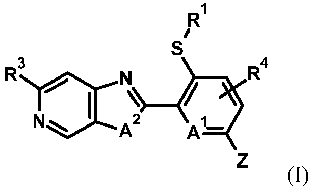
in welcher Z, R¹, R³, R⁴, A¹ und A² jeweils die oben genannten Bedeutungen haben,
mit einer Zink-metallorganischen Base der Struktur (NR^{a}R^{b})-Zn-R^{c} oder (NR^{a}R^{b})₂-Zn, in welcher R^{e} für Halogen oder -O-Pivaloyl steht und
R^{a} und R^{b} gemeinsam eine -(CH₂)₄-, -(CH₂)₅- oder -(CH₂)₂O(CH₂)₂-Gruppe bilden, wobei jede dieser Gruppen optional durch 1, 2, 3 oder 4 R^{d}-Radikale substituiert sein kann und R^{d} ausgewählt ist aus der Gruppe bestehend aus Methyl, Ethyl, n-Propyl und i-Propyl,
zu einer Verbindung der Formel (IVa) oder der Formel (IVb) umgesetzt wird, 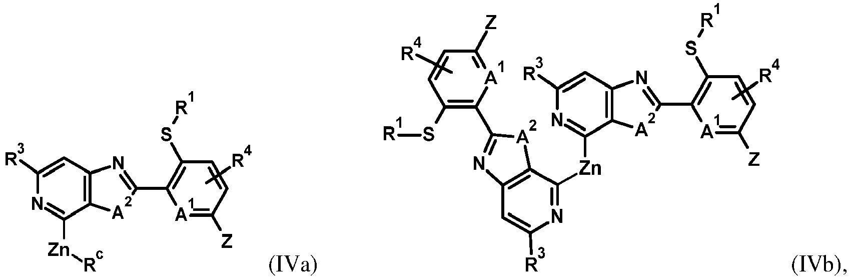 in welcher Z, R¹, R³, R⁴, R^{c}, A¹ und A² jeweils die oben genannten Bedeutungen haben,
und diese Verbindung der Formel (IVa) oder (IVb) in einem zweiten Verfahrensschritt b) mit einer Verbindung der Struktur Q-V, in welcher V für Halogen steht und Q die oben genannte Bedeutung hat, zur Verbindung der Formel (II) umgesetzt wird.

2. Verfahren gemäß Anspruch 1, **dadurch gekennzeichnet, dass**
Q für Chlor, Iod oder Brom steht,
Z für Brom, Fluor oder Chlor steht,
R^{c} für -O-Pivaloyl, Chlor, Brom oder Iod steht,
A¹ für Stickstoff, =N⁺-O⁻ oder =C-R⁵ steht,
A² für -N-R⁶ oder Sauerstoff steht,
R¹ für (C₁-C₄)Alkyl, (C₁-C₄)Hydroxyalkyl, (C₁-C₄)Halogenalkyl, (C₁-C₄)Cyanoalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyloxy-(C₁-C₄)alkyl, (C₂-C₄)Halogenalkinyl, (C₂-C₄)Cyanoalkinyl, (C₃-C₆)Cycloalkyl, (C₃-C₆)Halogencycloalkyl, (C₃-C₆)Cyanocycloalkyl, (C₃-C₆)Cycloalkyl(C₃-C₆)cycloalkyl, (C₁-C₄)Alkyl-(C₃-C₆)cycloalkyl, Halogen(C₃-C₆)cycloalkyl, (C₁-C₄)Alkylamino, Di-(C₁-C₄)alkyl-amino, (C₃-C₆)Cycloalkylamino, (C₁-C₄)Alkylcarbonyl-amino, (C₁-C₄)Alkylthio-(C₁-C₄)alkyl, (C₁-C₄)Halogenalkylthio-(C₁-C₄)alkyl, (C₁-C₄)Alkylsulfinyl-(C₁-C₄)alkyl, (C₁-C₄)Halogenalkylsulfinyl-(C₁-C₄)alkyl, (C₁-C₄)Alkylsulfonyl-(C₁-C₄)alkyl, (C₁-C₄)Alkylcarbonyl-(C₁-C₄)alkyl, (C₁-C₄)Halogenalkylcarbonyl-(C₁-C₄)alkyl, (C₁-C₄)Alkylsulfonylamino,
oder für jeweils gegebenenfalls einfach oder zweifach, gleich oder verschieden durch Aryl, Hetaryl oder Heterocyclyl substituiertes (C₁-C₄)Alkyl, (C₁-C₄)Alkoxy, (C₂-C₄)Alkenyl, (C₂-C₄)Alkinyl oder (C₃-C₆)Cycloalkyl steht, wobei Aryl, Hetaryl oder Heterocyclyl jeweils gegebenenfalls einfach oder zweifach gleich oder verschieden durch Halogen, Cyano, Carbamoyl, Aminosulfonyl, (C₁-C₄)Alkyl, (C₃-C₄)Cycloalkyl, (C₁-C₄)Alkoxy, (C₁-C₄)Halogenalkyl, (C₁-C₄)Halogenalkoxy, (C₁-C₄)Alkylthio, (C₁-C₄)Alkylsulfinyl, (C₁-C₄)Alkylsulfonyl oder (C₁-C₄)Alkylsulfimino substituiert sein können,
R³ für Wasserstoff, Cyano, Halogen, Nitro, Acetyl, Hydroxy, Amino, SCN, Tri-(C₁-C₄)alkylsilyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, Cyano(C₃-C₈)cycloalkyl, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Cyanoalkoxy, (C₁-C₄)Alkoxy-(C₁-C₄)alkoxy, (C₁-C₄)Alkylhydroxyimino, (C₁-C₄)Alkoxyimino, (C₁-C₄)Alkyl-(C₁-C₄)alkoxyiniino, (C₁-C₄)Halogenalkyl-(C₁-C₄)alkoxyimino, (C₁-C₄)Alkylthio, (C₁-C₄)Halogenalkylthio, (C₁-C₄)Alkylthio-(C₁-C₄)alkyl, (C₁-C₄)Alkylsuₗfinyl, (C₁-C₄)Halogenalkylsulfinyl, (C₁-C₄)Alkylsulfinyl-(C₁-C₄)alkyl, (C₁-C₄)Alkylsulfonyl, (C₁-C₄)Halogenalkylsulfonyl, (C₁-C₄)Alkylsulfonyl-(C₁-C₄)alkyl, (C₁-C₄)Alkylsulfonyloxy, (C₁-C₄)Alkylcarbonyl, (C₁-C₄)Halogenalkylcarbonyl, Aminocarbonyl, Aminothiocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl, Di-(C₁-C₄)alkyl-aminosulfonyl, Aminothiocarbonyl, NHCO-(C₁-C₄)alkyl ((C₁-C₄)Alkylcarbonylanüno) oder
für jeweils gegebenenfalls einfach oder zweifach, gleich oder verschieden substituiertes Phenyl oder Hetaryl steht, wobei (im Fall von Hetaryl) gegebenenfalls mindestens eine Carbonylgruppe enthalten sein kann und wobei als Substituenten jeweils in Frage kommen: Cyano, Halogen, Nitro, Acetyl, Amino, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Cyanoalkoxy, (C₁-C₄)Alkoxy-(C₁-C₄)alkoxy, (C₁-C₄)Alkylhydroxyimino, (C₁-C₄)Alkoxyimino, (C₁-C₄)Alkyl-(C₁-C₄)alkoxyimino, (C₁-C₄)Halogenalkyl-(C₁-C₄)alkoxyimino, (C₁-C₄)Alkylthio, (C₁-C₄)Halogenalkylthio, (C₁-C₄)Alkylthio-(C₁-C₄)alkyl, (C₁-C₄)Alkylsulfinyl, (C₁-C₄)Halogenalkylsulfinyl, (C₁-C₄)Alkylsulfinyl-(C₁-C₄)alkyl, (C₁-C₄)Alkylsulfonyl, (C₁-C₄)Halogenalkylsulfonyl, (C₁-C₄)Alkylsulfonyl-(C₁-C₄)alkyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl und Di-(C₁-C₄)alkylaminosulfonyl,
R⁴ für Wasserstoff, Cyano, Halogen, Nitro, Acetyl, Hydroxy, Amino, SCN, Tri-(C₁-C₄)alkylsilyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, Cyano(C₃-C₈)cycloalkyl, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Cyanoalkoxy, (C₁-C₄)Alkoxy-(C₁-C₄)alkoxy, (C₁-C₄)Alkylhydroxyimino, (C₁-C₄)Alkoxyimino, (C₁-C₄)Alkyl-(C₁-C₄)alkoxyimino, (C₁-C₄)Halogenalkyl-(C₁-C₄)alkoxyimino, (C₁-C₄)Alkylthio, (C₁-C₄)Halogenalkylthio, (C₁-C₄)Alkylthio-(C₁-C₄)alkyl, (C₁-C₄)Alkylsulfinyl, (C₁-C₄)Halogenalkylsulfinyl, (C₁-C₄)Alkylsulfinyl-(C₁-C₄)alkyl, (C₁-C₄)Alkylsulfonyl, (C₁-C₄)Halogenalkylsulfonyl, (C₁-C₄)Alkylsulfonyl-(C₁-C₄)alkyl, (C₁-C₄)Alkylsulfonyloxy, (C₁-C₄)Alkylcarbonyl, (C₁-C₄)Halogenalkylcarbonyl, Aminocarbonyl, Aminothiocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl, Di-(C₁-C₄)alkyl-aminosulfonyl, Aminothiocarbonyl, NHCO-(C₁-C₄)alkyl ((C₁-C₄)Alkylcarbonylanüno) steht,
weiterhin für jeweils gegebenenfalls einfach oder zweifach, gleich oder verschieden substituiertes Phenyl oder Hetaryl steht, wobei (im Fall von Hetaryl) gegebenenfalls mindestens eine Carbonylgruppe enthalten sein kann und wobei als Substituenten jeweils in Frage kommen: Cyano, Halogen, Nitro, Acetyl, Amino, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Cyanoalkoxy, (C₁-C₄)Alkoxy-(C₁-C₄)alkoxy, (C₁-C₄)Alkylhydroxyimino, (C₁-C₄)Alkoxyimino, (C₁-C₄)Alkyl-(C₁-C₄)alkoxyimino, (C₁-C₄)Halogenalkyl-(C₁-C₄)alkoxyimino, (C₁-C₄)Alkylthio, (C₁-C₄)Halogenalkylthio, (C₁-C₄)Alkylthio-(C₁-C₄)alkyl, (C₁-C₄)Alkylsulfinyl, (C₁-C₄)Halogenalkylsulfinyl, (C₁-C₄)Alkylsulfinyl-(C₁-C₄)alkyl, (C₁-C₄)Alkylsulfonyl, (C₁-C₄)Halogenalkylsulfonyl, (C₁-C₄)Alkylsulfonyl-(C₁-C₄)alkyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl, Di-(C₁-C₄)alkylaminosulfonyl, NHCO-(C₁-C₄)alkyl ((C₁-C₄)Alkylcarbonylamino),
R⁵ für Wasserstoff, Cyano, Halogen, Nitro, Acetyl, Hydroxy, Amino, SCN, Tri-(C₁-C₄)alkylsilyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Cyanoalkoxy, (C₁-C₄)Alkoxy-(C₁-C₄)alkoxy, (C₁-C₄)Alkylhydroxyimino, (C₁-C₄)Alkoxyimino, (C₁-C₄)Alkyl-(C₁-C₄)alkoxyimino, (C₁-C₄)Halogenalkyl-(C₁-C₄)alkoxyimino, (C₁-C₄)Alkylthio, (C₁-C₄)Halogenalkylthio, (C₁-C₄)Alkylthio-(C₁-C₄)alkyl, (C₁-C₄)Alkylsulfinyl, (C₁-C₄)Halogenalkylsulfinyl, (C₁-C₄)Alkylsulfinyl-(C₁-C₄)alkyl, (C₁-C₄)Alkylsulfonyl, (C₁-C₄)Halogenalkylsulfonyl, (C₁-C₄)Alkylsulfonyl-(C₁-C₄)alkyl, (C₁-C₄)Alkylsulfonyloxy, (C₁-C₄)Alkylcarbonyl, (C₁-C₄)Halogenalkylcarbonyl, Aminocarbonyl, Aminothiocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl, Di-(C₁-C₄)alkyl-aminosulfonyl, Aminothiocarbonyl oder NHCO-(C₁-C₄)alkyl ((C₁-C₄)Alkylcarbonylanüno) steht und
R⁶ für (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Halogenalkylthio-(C₁-C₄)alkyl, (C₁-C₄)Alkylsulfinyl-(C₁-C₄)alkyl, (C₁-C₄)Halogenalkylsulfinyl-(C₁-C₄)alkyl, (C₁-C₄)Alkylsulfonyl-(C₁-C₄)alkyl, (C₁-C₄)Halogenalkylsulfonyl-(C₁-C₄)alkyl, (C₁-C₄)Alkoxy-(C₁-C₄)alkylthio-(C₁-C₄)alkyl oder (C₁-C₄)Alkylcarbonyl-(C₁-C₄)alkyl steht.

3. Verfahren gemäß Anspruch 1, **dadurch gekennzeichnet, dass**
Q für Chlor, Iod oder Brom steht,
Z für Brom. Fluor oder Chlor steht,
R^{c} für -O-Pivaloyl, Chlor, Brom oder Iod steht,
A¹ für Stickstoff oder =C-R⁵ steht,
A² für -N-R⁶ steht,
R¹ für (C₁-C₄)Alkyl, (C₁-C₄)Hydroxyalkyl, (C₁-C₄)Halogenalkyl, (C₂-C₄)Alkenyl, (C₂-C₄)Halogenalkenyl, (C₂-C₄)Alkinyl, (C₂-C₄)Halogenalkinyl, (C₃-C₆)Cycloalkyl, (C₁-C₄)Alkylthio-(C₁-C₄)alkyl, (C₁-C₄)Alkylsulfinyl-(C₁-C₄)alkyl oder (C₁-C₄)Alkylsulfonyl-(C₁-C₄)alkyl steht,
R³ für Wasserstoff, Halogen, (C₁-C₄)Alkyl, (C₁-C₄)Halogenalkyl, (C₁-C₄)Halogenalkoxy, (C₁-C₄)Alkylthio, (C₁-C₄)Alkylsulfinyl, (C₁-C₄)Alkylsulfonyl, (C₁-C₄)Halogenalkylthio, (C₁-C₄)Halogenalkylsulfinyl oder (C₁-C₄)Halogenalkylsulfonyl steht,
R⁴ für Wasserstoff, Cyano, Halogen, Nitro, Hydroxy, Amino, SCN, Tri-(C₁-C₄)alkylsilyl, (C₃-C₆)Cycloalkyl, Cyano(C₃-C₈)cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkyl, (C₁-C₄)Halogenalkyl, (C₁-C₄)Cyanoalkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Cyanoalkoxy, (C₁-C₄)Alkylhydroxyimino, (C₁-C₄)Alkoxyimino, (C₁-C₄)Alkyl-(C₁-C₄)alkoxyimino, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl, Di-(C₁-C₄)alkyl-aminosulfonyl oder NHCO-(C₁-C₄)alkyl ((C₁-C₄)Alkylcarbonylamino) steht,
weiterhin für jeweils gegebenenfalls einfach oder zweifach, gleich oder verschieden substituiertes Phenyl oder Hetaryl steht, wobei (im Fall von Hetaryl) gegebenenfalls mindestens eine Carbonylgruppe enthalten sein kann und wobei als Substituenten jeweils in Frage kommen: Cyano, Halogen, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkyl, (C₁-C₄)Halogenalkyl, (C₁-C₄)Cyanoalkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Alkylhydroxyimino, (C₁-C₄)Alkoxyimino, (C₁-C₄)Alkyl-(C₁-C₄)alkoxyimino, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl, Di-(C₁-C₄)alkylaminosulfonyl, NHCO-(C₁-C₄)alkyl ((C₁-C₄)Alkylcarbonylamino),
R⁵ für Wasserstoff, Halogen, Cyano oder (C₁-C₄)Alkyl steht und
R⁶ für (C₁-C₄)Alkyl oder (C₁-C₄)Alkoxy-(C₁-C₄)alkyl steht.

4. Verfahren gemäß Anspruch 1, **dadurch gekennzeichnet, dass**
Q für Iod oder Brom steht,
Z für Brom oder Chlor steht,
R^{c} für Chlor, Brom oder Iod steht,
A¹ für Stickstoff oder =C-R⁵ steht,
A² für -N-R⁶ steht,
R¹ für Methyl, Ethyl, n-Propyl, i-Propyl, cyclo-Propyl, n-Butyl, i-Butyl, tert.-Butyl, cyclo-Butyl, Fluormethyl, Difluormethyl, Trifluormethyl, Fluorethyl, Difluorethyl, Trifluorethyl, Tetrafluorethyl oder Pentafluorethyl steht,
R³ für Fluor, Chlor, Fluormethyl, Difluormethyl, Trifluormethyl, Fluorethyl, Difluorethyl, Trifluorethyl, Tetrafluorethyl, Pentafluorethyl, Trifluormethoxy, Difluorchlormethoxy, Dichlorfluormethoxy, Trifluormethylthio, Trifluormethylsulfonyl oder Trifluormethylsulfinyl steht,
R⁴ für Wasserstoff, Cyano, Halogen, (C₁-C₄)Alkyl, (C₁-C₄)Halogenalkyl, (C₁-C₄)Halogenalkoxy, (C₁-C₄)Alkylthio, (C₁-C₄)Alkylsulfinyl, (C₁-C₄)Alkylsulfonyl, (C₁-C₄)Halogenalkylthio, (C₁-C₄)Halogenalkylsulfinyl, (C₁-C₄)Halogenalkylsulfonyl oder NHCO-(C₁-C₄)alkyl ((C₁-C₄)Alkylcarbonylamino) steht,
R⁵ für Wasserstoff, Fluor, Chlor, Brom oder Cyano steht und
R⁶ für Methyl, Ethyl, i-Propyl, Methoxymethyl oder Methoxyethyl steht.

5. Verfahren gemäß Anspruch 1, **dadurch gekennzeichnet, dass**
Q für Iod oder Brom steht,
Z für Brom oder Chlor steht,
R^{c} für Chlor oder Brom steht,
A¹ für Stickstoff steht,
A² für -N-R⁶ steht,
R¹ für Methyl, Ethyl, n-Propyl, i-Propyl oder cyclo-Propyl steht,
R³ für Trifluormethyl oder Pentafluorethyl steht,
R⁴ für Wasserstoff steht und
R⁶ für Methyl steht.

6. Verfahren gemäß Anspruch 1, **dadurch gekennzeichnet, dass**
Q für Iod oder Brom steht,
Z für Brom oder Chlor steht,
R^{c} für Chlor steht,
A¹ für Stickstoff steht,
A² für -N-R⁶ steht,
R¹ für Ethyl steht,
R³ für Trifluormethyl steht,
R⁴ für Wasserstoff steht und
R⁶ für Methyl steht.

7. Verfahren gemäß einem der Ansprüche 1 bis 6, **dadurch gekennzeichnet, dass** es sich bei der Zink-metallorganischen Base um eine Verbindung der Formel (VI) handelt
(VI) (TMP)ₓ ZnCL₂₋ₓ ,
worin x für die Zahl 1 oder 2 steht.

8. Verfahren gemäß einem der Ansprüche 1 bis 7, **dadurch gekennzeichnet, dass** die Zinkmetallorganische Base in Verbindung mit einem Alkali- oder Erdalkalihalogenid, vorzugsweise Lithiumchlorid und/oder Magnesiumchlorid, vorliegt.

9. Verfahren gemäß einem der Ansprüche 1 bis 8, **dadurch gekennzeichnet, dass** es sich bei der Verbindung Q-V um ein elementares Halogen, insbesondere um F₂, Cl₂, Br₂ oder I₂, handelt.

10. Verfahren gemäß einem der Ansprüche 1 bis 9, **dadurch gekennzeichnet, dass** das Lösungsmittel THF oder N,N-Dimethylformamid (DMF) ist.

11. Verfahren gemäß einem der Ansprüche 1 bis 10, **dadurch gekennzeichnet, dass** Verfahrensschritt
a) bei einer Temperatur zwischen 0 °C und 80 °C durchgeführt wird.

12. Verfahren gemäß einem der Ansprüche 1 bis 11, **dadurch gekennzeichnet, dass** Verfahrensschritt
b) bei einer Temperatur zwischen 0 °C und 80 °C durchgeführt wird.

13. Verfahren gemäß einem der Ansprüche 1 bis 12, **dadurch gekennzeichnet, dass** die Verbindungen der Formel (II) in einem dritten Schritt c) weiter umgesetzt zu Verbindungen der Formel (III) 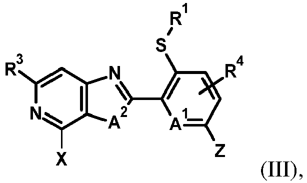 in welcher Z, R¹, R³, R⁴, A¹ und A² Bedeutungen gemäß einer der Ansprüche 1 bis 6 haben und
X für Cyano, Carboxyl, Halogen, Nitro, Acetyl, Hydroxy, Amino, SCN, Tri-(C₁-C₆)alkylsilyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₃-C₆)Cycloalkyl, (C₃-C₆)Halogencycloalkyl, (C₃-C₆)Cyanocycloalkyl, (C₃-C₆)Cycloalkyl-(C₃-C₆)cycloalkyl, (C₁-C₄)Alkyl-(C₃-C₆)cycloalkyl (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkoxyimino, -N=C(H)-O(C₁-C₆)Alkyl, -C(H)=N-O(C₁-C₆)Alkyl, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkylaminosulfonyl, (C₁-C₆)Alkylsulfoximino, Aminothiocarbonyl, (C₁-C₆)Alkylaminothiocarbonyl, Di-(C₁-C₆)alkylaminothiocarbonyl, (C₃-C₈)Cycloalkylamino, (C₁-C₆)Alkylcarbamoyl (umfasst -NHCOO(C₁-C₆)alkyl, -N(C₁-C₆)alkylCOO(C₁-C₆)alkyl, -OCONH(C₁-C₆)alkyl oder -OCON(C₁-C₆)dialkyl), (C₁-C₆)Alkylcarbonylamino ((C₁-C₆)alkylCONH), (C₁-C₆)Alkylharnstoff (umfasst -NHCONH(C₁-C₆)alkyl, und -NHCON(C₁-C₆)dialkyl) oder einen gegebenenfalls einfach oder mehrfach, gleich oder verschieden substituierten gesättigten, teilgesättigten oder heteroaromatischen Ring, in dem mindestens ein C-Atom durch ein Heteroatom ersetzt ist oder einen einfach oder mehrfach, gleich oder verschieden substituierten aromatischen Ring steht, wobei jeweils gegebenenfalls mindestens eine Carbonylgruppe enthalten sein kann und wobei als Substituenten jeweils in Frage kommen: Cyano, Carboxyl, Halogen, Nitro, Acetyl, Hydroxy, Amino, SCN, Tri-(C₁-C₆)alkylsilyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₃-C₆)Cycloalkyl, (C₃-C₆)Halogencycloalkyl, (C₃-C₆)Cyanocycloalkyl, (C₃-C₆)Cycloalkyl-(C₃-C₆)cycloalkyl, (C₁-C₄)Alkyl-(C₃-C₆)cycloalkyl (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₁-C₆)Alkoxyimino, -N=C(H)-O(C₁-C₆)Alkyl, -C(H)=N-O(C₁-C₆)Alkyl, (C₁-C₆)Halogenalkyl-(C₁-C₆)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(Ci-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Di-(C₁-C₆)Alkylamino, Aminosulfonyl, (C₁-C₆)Alkylaminosulfonyl, Di-(C₁-C₆)alkylaminosulfonyl, (C₁-C₆)Alkylsulfoximino, Aminothiocarbonyl, (C₁-C₆)Alkylaminothiocarbonyl, Di-(C₁-C₆)alkylaminothiocarbonyl, (C₃-C₈)Cycloalkylamino oder (C₁-C₆)Alkylcarbonylamino.

14. Verfahren gemäß Anspruch 13, **dadurch gekennzeichnet, dass**
X für Cyano, Halogen, Nitro, Acetyl, Amino, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₃-C₆)Cycloalkyl, (C₃-C₆)Halogencycloalkyl, (C₃-C₆)Cyanocycloalkyl, (C₃-C₆)Cycloalkyl-(C₃-C₆)cycloalkyl, (C₁-C₄)Alkyl-(C₃-C₆)cycloalkyl, (C₁-C₄)Alkoxy, (C₁-C₄)Halogenalkoxy, (C₁-C₄)Cyanoalkoxy, (C₁-C₄)Alkoxy-(C₁-C₄)alkoxy, (C₁-C₄)Alkoxyimino, -N=C(H)-O(C₁-C₄)Alkyl, -C(H)=N-O(C₁-C₄)Alkyl, (C₁-C₄)Halogenalkyl-(C₁-C₄)alkoxyimino, (C₁-C₄)Alkylthio, (C₁-C₄)Halogenalkylthio, (C₁-C₄)Alkylthio-(C₁-C₄)alkyl, (C₁-C₄)Alkylsulfinyl, (C₁-C₄)Halogenalkylsulfinyl, (C₁-C₄)Alkylsulfinyl-(C₁-C₄)alkyl, (C₁-C₄)Alkylsulfonyl, (C₁-C₄)Halogenalkylsulfonyl, (C₁-C₄)Alkylsulfonyl-(C₁-C₄)alkyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl, Di-(C₁-C₄)alkylaminosulfonyl, (C₁-C₄)Alkylcarbamoyl (umfasst -NHCOO(C₁-C₄)alkyl, -N(C₁-C₄)alkylCOO(C₁-C₄)alkyl, -OCONH(C₁-C₄)alkyl oder -OCON(C₁-C₄)dialkyl), (C₁-C₄)Alkylcarbonylamino ((C₁-C₄)alkylCONH) oder (C₁-C₄)Alkylharnstoff (umfasst -NHCONH(C₁-C₆)alkyl und -NHCON(C₁-C₆)dialkyl) steht.

15. Verfahren gemäß Anspruch 13, **dadurch gekennzeichnet, dass**
X für Cyano, Halogen, Nitro, Acetyl, Amino, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₃-C₆)Cycloalkyl, (C₃-C₆)Halogencycloalkyl, (C₃-C₆)Cyanocycloalkyl, (C₃-C₆)Cycloalkyl-(C₃-C₆)cycloalkyl, (C₁-C₄)Alkyl-(C₃-C₆)cycloalkyl, (C₁-C₄)Alkoxy, (C₁-C₄)Halogenalkoxy, (C₁-C₄)Cyanoalkoxy oder (C₁-C₄)Alkoxy-(C₁-C₄)alkoxy steht.

16. Verfahren gemäß Anspruch 13, **dadurch gekennzeichnet, dass**
X für Cyano, Halogen, Amino, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₃-C₆)Cycloalkyl, (C₃-C₆)Halogencycloalkyl, (C₃-C₆)Cyanocycloalkyl, (C₃-C₆)Cycloalkyl-(C₃-C₆)cycloalkyl, ((C₁-C₄)Alkoxy oder (C₁-C₄)Cyanoalkoxy steht.

17. Verfahren gemäß einem der Ansprüche 13 bis 16, **dadurch gekennzeichnet, dass** die Umsetzung in Verfahrensschritt c) bei einer Temperatur zwischen 0 °C und 110 °C durchgeführt wird.

18. Verfahren gemäß einem der Ansprüche 13 bis 17, **dadurch gekennzeichnet, dass** die Verbindungen der Formel (II) mit metallorganischen Zink Reagenzien in Gegenwart eines Katalysators oder mit Metallsalzen umgesetzt werden.

19. Verbindungen der Formel (IVa) 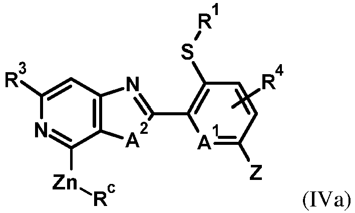 in welcher Z, R¹, R³, R⁴, R^{c}, A¹ und A² die Bedeutungen gemäß einem der Ansprüche 1 bis 6 haben.

20. Verbindungen der Formel (IVb) 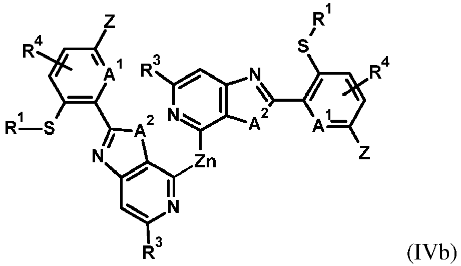 in welcher Z, R¹, R³, R⁴, A¹ und A² die Bedeutungen gemäß einem der Ansprüche 1 bis 6 haben.

21. Verbindungen der Formel (II) 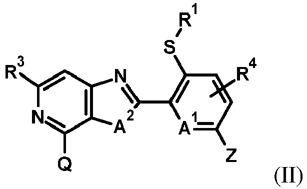 in welcher Q, Z, R¹, R³, R⁴, A¹ und A² die Bedeutungen gemäß einem der Ansprüche 1 bis 6 haben.

## Claims

1. Method for preparing compounds of the formula (II) 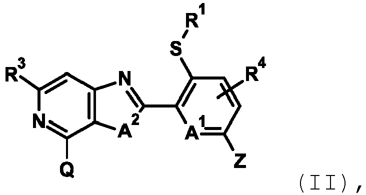 in which
Q and Z are each independently halogen,
A¹ is nitrogen, =N⁺O⁻ or =C-R⁵,
A² is -N-R⁶, oxygen or sulfur,
R¹ is (C₁-C₆)alkyl, (C₁-C₆)haloalkyl, (C₁-C₆)cyanoalkyl, (C₁-C₆)hydroxyalkyl, (C₁-C₆)alkoxy-(C₁-C₆)alkyl, (C₁-C₆)haloalkoxy-(C₁-C₆)alkyl, (C₂-C₆)alkenyl, (C₂-C₆)alkenyloxy-(C₁-C₆)alkyl, (C₂-C₆)haloalkenyloxy-(C₁-C₆)alkyl, (C₂-C₆)haloalkenyl, (C₂-C₆)cyanoalkenyl, (C₂-C₆)alkynyl, (C₂-C₆)alkynyloxy-(C₁-C₆)alkyl, (C₂-C₆)haloalkynyloxy-(C₁-C₆)alkyl, (C₂-C₆)haloalkynyl, (C₂-C₆)cyanoalkynyl, (C₃-C₈)cycloalkyl, (C₃-C₆)halocycloalkyl, (C₃-C₆)cyanocycloalkyl, (C₃-C₈)cycloalkyl-(C₃-C₈)cycloalkyl, (C₁-C₆)alkyl-(C₃-C₈)cycloalkyl, halo (C₃-C₈)cycloalkyl, amino, (C₁-C₆)alkylamino, di(C₁-C₆)alkylamino, (C₃-C₈)cycloalkylamino, (C₁-C₆)alkylcarbonylamino, (C₁-C₆)alkylthio-(C₁-C₆)alkyl, (C₁-C₆)haloalkylthio-(C₁-C₆)alkyl, (C₁-C₆)alkylsulfinyl-(C₁-C₆)alkyl, (C₁-C₆)haloalkylsulfinyl-(C₁-C₆)alkyl, (C₁-C₆)alkylsulfonyl-(C₁-C₆)alkyl, (C₁-C₆)haloalkylsulfonyl- (C₁-C₆)alkyl, (C₁-C₆)alkoxy-(C₁-C₆)alkylthio-(C₁-C₆)alkyl, (C₁-C₆)alkoxy-(C₁-C₆)alkylsulfinyl- (C₁-C₆)alkyl, (C₁-C₆)alkoxy- (C₁-C₆)alkylsulfonyl- (C₁-C₆)alkyl, (C₁-C₆)alkylcarbonyl- (C₁-C₆)alkyl, (C₁-C₆)haloalkylcarbonyl-(C₁-C₆)alkyl, (C₁-C₆)alkoxycarbonyl- (C₁-C₆)alkyl, (C₁-C₆)haloalkoxycarbonyl-(C₁-C₆)alkyl, (C₁-C₆)alkylsulfonylamino, aminosulfonyl- (C₁-C₆)alkyl, (C₁-C₆)alkylaminosulfonyl- (C₁-C₆)alkyl, di(C₁-C₆)alkylaminosulfonyl-(C₁-C₆)alkyl,
or is (C₁-C₆)alkyl, (C₁-C₆)alkoxy, (C₂-C₆)alkenyl, (C₂-C₆)alkynyl, (C₃-C₈)cycloalkyl, each of which is optionally mono- or polysubstituted by identical or different substituents from the group consisting of aryl, hetaryl and heterocyclyl, where aryl, hetaryl or heterocyclyl may each optionally be mono- or polysubstituted by identical or different substituents from the group consisting of halogen, cyano, nitro, hydroxyl, amino, carboxy, carbamoyl, aminosulfonyl, (C₁-C₆)alkyl, (C₃-C₆)cycloalkyl, (C₁-C₆)alkoxy, (C₁-C₆)haloalkyl, (C₁-C₆)haloalkoxy, (C₁-C₆)alkylthio, (C₁-C₆)alkylsulfinyl, (C₁-C₆)alkylsulfonyl, (C₁-C₆)alkylsulfimino, (C₁-C₆)alkylsulfimino-(C₁-C₆)alkyl, (C₁-C₆)alkylsulfimino-(C₂-C₆)alkylcarbonyl, (C₁-C₆)alkylsulfoximino, (C₁-C₆)alkylsulfoximino-(C₁-C₆)alkyl, (C₁-C₆)alkylsulfoximino-(C₂-C₆)alkylcarbonyl, (C₁-C₆)alkoxycarbonyl, (C₁-C₆)alkylcarbonyl, (C₃-C₆)trialkylsilyl and benzyl, R³ is hydrogen, cyano, halogen, nitro, acetyl, hydroxyl, amino, SCN, tri(C₁-C₆)alkylsilyl, (C₃-C₈)cycloalkyl, (C₃-C₈)cycloalkyl- (C₃-C₈)cycloalkyl, (C₁-C₆)alkyl- (C₃-C₈)cycloalkyl, halo (C₃-C₈)cycloalkyl, cyano (C₃-C₈)cycloalkyl, (C₁-C₆)alkyl, (C₁-C₆)haloalkyl, (C₁-C₆)cyanoalkyl, (C₁-C₆)hydroxyalkyl, hydroxycarbonyl- (C₁-C₆)-alkoxy, (C₁-C₆)alkoxycarbonyl-(C₁-C₆)alkyl, (C₁-C₆)alkoxy- (C₁-C₆)alkyl, (C₂-C₆)alkenyl, (C₂-C₆)haloalkenyl, (C₂-C₆)cyanoalkenyl, (C₂-C₆)alkynyl, (C₂-C₆)haloalkynyl, (C₂-C₆)cyanoalkynyl, (C₁-C₆)alkoxy, (C₁-C₆)haloalkoxy, (C₁-C₆)cyanoalkoxy, (C₁-C₆)alkoxycarbonyl- (C₁-C₆)alkoxy, (C₁-C₆)alkoxy- (C₁-C₆)alkoxy, (C₁-C₆)alkylhydroxyimino, (C₁-C₆)alkoxyimino, (C₁-C₆)alkyl-(C₁-C₆)alkoxyimino, (C₁-C₆)haloalkyl-(C₁-C₆)alkoxyimino, (C₁-C₆)alkylthio, (C₁-C₆)haloalkylthio, (C₁-C₆)alkoxy-(C₁-C₆)alkylthio, (C₁-C₆)alkylthio-(C₁-C₆)alkyl, (C₁-C₆)alkylsulfinyl, (C₁-C₆)haloalkylsulfinyl, (C₁-C₆)alkoxy-(C₁-C₆)alkylsulfinyl, (C₁-C₆)alkylsulfinyl-(C₁-C₆)alkyl, (C₁-C₆)alkylsulfonyl, (C₁-C₆)haloalkylsulfonyl, (C₁-C₆)alkoxy-(C₁-C₆)alkylsulfonyl, (C₁-C₆)alkylsulfonyl-(C₁-C₆)alkyl, (C₁-C₆)alkylsulfonyloxy, (C₁-C₆)alkylcarbonyl, (C₁-C₆)alkylthiocarbonyl, (C₁-C₆)haloalkylcarbonyl, (C₁-C₆)alkylcarbonyloxy, (C₁-C₆)alkoxycarbonyl, (C₁-C₆)haloalkoxycarbonyl, aminocarbonyl, (C₁-C₆)alkylaminocarbonyl, (C₁-C₆)alkylaminothiocarbonyl, di (C₁-C₆)alkylaminocarbonyl, di(C₁-C₆)alkylaminothiocarbonyl, (C₂-C₆)alkenylaminocarbonyl, di(C₂-C₆)-alkenylaminocarbonyl, (C₃-C₈)cycloalkylaminocarbonyl, (C₁-C₆)alkylsulfonylamino, (C₁-C₆)alkylamino, di(C₁-C₆)alkylamino, aminosulfonyl, (C₁-C₆)alkylaminosulfonyl, di(C₁-C₆)alkylaminosulfonyl, (C₁-C₆)alkylsulfoximino, aminothiocarbonyl, (C₁-C₆)alkylaminothiocarbonyl, di(C₁-C₆)alkylaminothiocarbonyl, (C₃-C₈)cycloalkylamino, -NHCO-(C₁-C₆)alkyl ((C₁-C₆)alkylcarbonylamino) or
is aryl or hetaryl, each of which is optionally mono- or polysubstituted by identical or different substituents, where (in the case of hetaryl) at least one carbonyl group may optionally be present and where possible substituents in each case are as follows: cyano, carboxyl, halogen, nitro, acetyl, hydroxyl, amino, SCN, tri(C₁-C₆)alkylsilyl, (C₁-C₆)alkyl, (C₁-C₆)haloalkyl, (C₁-C₆)cyanoalkyl, (C₁-C₆)hydroxyalkyl, hydroxycarbonyl-(C₁-C₆)-alkoxy, (C₁-C₆)alkoxycarbonyl-(C₁-C₆)alkyl, (C₁-C₆)alkoxy-(C₁-C₆)alkyl, (C₂-C₆)alkenyl, (C₂-C₆)haloalkenyl, (C₂-C₆)cyanoalkenyl, (C₂-C₆)alkynyl, (C₂-C₆)haloalkynyl, (C₂-C₆)cyanoalkynyl, (C₁-C₆)alkoxy, (C₁-C₆)haloalkoxy, (C₁-C₆)cyanoalkoxy, (C₁-C₆)alkoxycarbonyl-(C₁-C₆)alkoxy, (C₁-C₆)alkoxy-(C₁-C₆)alkoxy, (C₁-C₆)alkylhydroxyimino, (C₁-C₆)alkoxyimino, (C₁-C₆)alkyl-(C₁-C₆)alkoxyimino, (C₁-C₆)haloalkyl-(C₁-C₆)alkoxyimino, (C₁-C₆)alkylthio, (C₁-C₆)haloalkylthio, (C₁-C₆)alkoxy-(C₁-C₆)alkylthio, (C₁-C₆)alkylthio-(C₁-C₆)alkyl, (C₁-C₆)alkylsulfinyl, (C₁-C₆)haloalkylsulfinyl, (C₁-C₆)alkoxy-(C₁-C₆)alkylsulfinyl, (C₁-C₆)alkylsulfinyl-(C₁-C₆)-alkyl, (C₁-C₆)alkylsulfonyl, (C₁-C₆)haloalkylsulfonyl, (C₁-C₆)alkoxy-(C₁-C₆)alkylsulfonyl, (C₁-C₆)alkylsulfonyl-(C₁-C₆)alkyl, (C₁-C₆)alkylsulfonyloxy, (C₁-C₆)alkylcarbonyl, (C₁-C₆)haloalkylcarbonyl, (C₁-C₆)alkylcarbonyloxy, (C₁-C₆)alkoxycarbonyl, (C₁-C₆)haloalkoxycarbonyl, aminocarbonyl, (C₁-C₆)alkylaminocarbonyl, di(C₁-C₆)alkylaminocarbonyl, (C₂-C₆)alkenylaminocarbonyl, di(C₂-C₆)alkenylaminocarbonyl, (C₃-C₈)cycloalkylaminocarbonyl, (C₁-C₆)alkylsulfonylamino, (C₁-C₆)alkylamino, di(C₁-C₆)alkylamino, aminosulfonyl, (C₁-C₆)alkylaminosulfonyl, di(C₁-C₆)alkylaminosulfonyl, (C₁-C₆)alkylsulfoximino, aminothiocarbonyl, (C₁-C₆)alkylaminothiocarbonyl, di(C₁-C₆)alkylaminothiocarbonyl or (C₃-C₈)cycloalkylamino, R⁴ is hydrogen, cyano, halogen, nitro, acetyl, hydroxyl, amino, SCN, tri(C₁-C₆)alkylsilyl, (C₃-C₈)cycloalkyl, (C₃-C₈)cycloalkyl-(C₃-C₈)cycloalkyl, (C₁-C₆)alkyl-(C₃-C₈)cycloalkyl, halo(C₃-C₈)cycloalkyl, cyano (C₃-C₈)cycloalkyl, (C₁-C₆)alkyl, (C₁-C₆)haloalkyl, (C₁-C₆)cyanoalkyl, (C₁-C₆)hydroxyalkyl, hydroxycarbonyl-(C₁-C₆)-alkoxy, (C₁-C₆)alkoxycarbonyl-(C₁-C₆)alkyl, (C₁-C₆)alkoxy-(C₁-C₆)alkyl, (C₂-C₆)alkenyl, (C₂-C₆)haloalkenyl, (C₂-C₆)cyanoalkenyl, (C₂-C₆)alkynyl, (C₂-C₆)haloalkynyl, (C₂-C₆)cyanoalkynyl, (C₁-C₆)alkoxy, (C₁-C₆)haloalkoxy, (C₁-C₆)cyanoalkoxy, (C₁-C₆)alkoxycarbonyl-(C₁-C₆)alkoxy, (C₁-C₆)alkoxy-(C₁-C₆)alkoxy, (C₁-C₆)alkylhydroxyimino, (C₁-C₆)alkoxyimino, (C₁-C₆)alkyl-(C₁-C₆)alkoxyimino, (C₁-C₆)haloalkyl-(C₁-C₆)alkoxyimino, (C₁-C₆)alkylthio, (C₁-C₆)haloalkylthio, (C₁-C₆)alkoxy- (C₁-C₆)alkylthio, (C₁-C₆)alkylthio-(C₁-C₆)alkyl, (C₁-C₆)alkylsulfinyl, (C₁-C₆)haloalkylsulfinyl, (C₁-C₆)alkoxy-(C₁-C₆)alkylsulfinyl, (C₁-C₆)alkylsulfinyl-(C₁-C₆)alkyl, (C₁-C₆)alkylsulfonyl, (C₁-C₆)haloalkylsulfonyl, (C₁-C₆)alkoxy-(C₁-C₆)alkylsulfonyl, (C₁-C₆)alkylsulfonyl-(C₁-C₆)alkyl, (C₁-C₆)alkylsulfonyloxy, (C₁-C₆)alkylcarbonyl, (C₁-C₆)alkylthiocarbonyl, (C₁-C₆)haloalkylcarbonyl, (C₁-C₆)alkylcarbonyloxy, (C₁-C₆)alkoxycarbonyl, (C₁-C₆)haloalkoxycarbonyl, aminocarbonyl, (C₁-C₆)alkylaminocarbonyl, (C₁-C₆)alkylaminothiocarbonyl, di(C₁-C₆)alkylaminocarbonyl, di(C₁-C₆)alkylaminothiocarbonyl, (C₂-C₆)alkenylaminocarbonyl, di(C₂-C₆)alkenylaminocarbonyl, (C₃-C₈)cycloalkylaminocarbonyl, (C₁-C₆)alkylsulfonylamino, (C₁-C₆)alkylamino, di(C₁-C₆)alkylamino, aminosulfonyl, (C₁-C₆)alkylaminosulfonyl, di(C₁-C₆)alkylaminosulfonyl, (C₁-C₆)alkylsulfoximino, aminothiocarbonyl, (C₁-C₆)alkylaminothiocarbonyl, di(C₁-C₆)alkylaminothiocarbonyl, (C₃-C₈)cycloalkylamino, NHCO-(C₁-C₆)alkyl ((C₁-C₆)alkylcarbonylamino), or
is aryl or hetaryl, each of which is optionally mono- or polysubstituted by identical or different substituents, where (in the case of hetaryl) at least one carbonyl group may optionally be present and where possible substituents in each case are as follows: cyano, carboxyl, halogen, nitro, acetyl, hydroxyl, amino, SCN, tri(C₁-C₆)alkylsilyl, (C₃-C₈)cycloalkyl, (C₃-C₈)cycloalkyl-(C₃-C₈)cycloalkyl, (C₁-C₆)alkyl-(C₃-C₈)cycloalkyl, halo(C₃-C₈)cycloalkyl, (C₁-C₆)alkyl, (C₁-C₆)haloalkyl, (C₁-C₆)cyanoalkyl, (C₁-C₆)hydroxyalkyl, hydroxycarbonyl-(C₁-C₆)-alkoxy, (C₁-C₆)alkoxycarbonyl-(C₁-C₆)alkyl, (C₁-C₆)alkoxy-(C₁-C₆)alkyl, (C₂-C₆)alkenyl, (C₂-C₆)haloalkenyl, (C₂-C₆)cyanoalkenyl, (C₂-C₆)alkynyl, (C₂-C₆)haloalkynyl, (C₂-C₆)cyanoalkynyl, (C₁-C₆)alkoxy, (C₁-C₆)haloalkoxy, (C₁-C₆)cyanoalkoxy, (C₁-C₆)alkoxycarbonyl-(C₁-C₆)alkoxy, (C₁-C₆)alkoxy-(C₁-C₆)alkoxy, (C₁-C₆)alkylhydroxyimino, (C₁-C₆)alkoxyimino, (C₁-C₆)alkyl-(C₁-C₆)alkoxyimino, (C₁-C₆)haloalkyl-(C₁-C₆)alkoxyimino, (C₁-C₆)alkylthio, (C₁-C₆)haloalkylthio, (C₁-C₆)alkoxy-(C₁-C₆)alkylthio, (C₁-C₆)alkylthio-(C₁-C₆)alkyl, (C₁-C₆)alkylsulfinyl, (C₁-C₆)haloalkylsulfinyl, (C₁-C₆)alkoxy-(C₁-C₆)alkylsulfinyl, (C₁-C₆)alkylsulfinyl-(C₁-C₆)alkyl, (C₁-C₆)alkylsulfonyl, (C₁-C₆)haloalkylsulfonyl, (C₁-C₆)alkoxy-(C₁-C₆)alkylsulfonyl, (C₁-C₆)alkylsulfonyl-(C₁-C₆)alkyl, (C₁-C₆)alkylsulfonyloxy, (C₁-C₆)alkylcarbonyl, (C₁-C₆)haloalkylcarbonyl, (C₁-C₆)alkylcarbonyloxy, (C₁-C₆)alkoxycarbonyl, (C₁-C₆)haloalkoxycarbonyl, aminocarbonyl, (C₁-C₆)alkylaminocarbonyl, di(C₁-C₆)alkylaminocarbonyl, (C₂-C₆)alkenylaminocarbonyl, di(C₂-C₆)alkenylaminocarbonyl, (C₃-Cs)cycloalkylaminocarbonyl, (C₁-C₆)alkylsulfonylamino, (C₁-C₆)alkylamino, di(C₁-C₆)alkylamino, aminosulfonyl, (C₁-C₆)alkylaminosulfonyl, di(C₁-C₆)alkylaminosulfonyl, (C₁-C₆)alkylsulfoximino, aminothiocarbonyl, (C₁-C₆)alkylaminothiocarbonyl, di(C₁-C₆)alkylaminothiocarbonyl, (C₃-C₈)cycloalkylamino, (C₁-C₆)alkylcarbonylamino,
R⁵ is hydrogen, cyano, halogen, nitro, acetyl, hydroxyl, amino, SCN, tri (C₁-C₆)alkylsilyl, (C₃-C₈)cycloalkyl, (C₃-C₈)cycloalkyl-(C₃-C₈)cycloalkyl, (C₁-C₆)alkyl-(C₃-C₈)cycloalkyl, halo(C₃-C₈)cycloalkyl, (C₁-C₆)alkyl, (C₁-C₆)haloalkyl, (C₁-C₆)cyanoalkyl, (C₁-C₆)hydroxyalkyl, hydroxycarbonyl-(C₁-C₆)alkoxy, (C₁-C₆)alkoxycarbonyl-(C₁-C₆)alkyl, (C₁-C₆)alkoxy-(C₁-C₆)alkyl, (C₂-C₆)alkenyl, (C₂-C₆)haloalkenyl, (C₂-C₆)cyanoalkenyl, (C₂-C₆)alkynyl, (C₂-C₆)haloalkynyl, (C₂-C₆)cyanoalkynyl, (C₁-C₆)alkoxy, (C₁-C₆)haloalkoxy, (C₁-C₆)cyanoalkoxy, (C₁-C₆)alkoxycarbonyl-(C₁-C₆)alkoxy, (C₁-C₆)alkoxy-(C₁-C₆)alkoxy, (C₁-C₆)alkylhydroxyimino, (C₁-C₆)alkoxyimino, (C₁-C₆)alkyl-(C₁-C₆)alkoxyimino, (C₁-C₆)haloalkyl-(C₁-C₆)alkoxyimino, (C₁-C₆)alkylthio, (C₁-C₆)haloalkylthio, (C₁-C₆)alkoxy-(C₁-C₆)alkylthio, (C₁-C₆)alkylthio-(C₁-C₆)alkyl, (C₁-C₆)alkylsulfinyl, (C₁-C₆)haloalkylsulfinyl, (C₁-C₆)alkoxy-(C₁-C₆)alkylsulfinyl, (C₁-C₆)alkylsulfinyl-(C₁-C₆)alkyl, (C₁-C₆)alkylsulfonyl, (C₁-C₆)haloalkylsulfonyl, (C₁-C₆)alkoxy-(C₁-C₆)alkylsulfonyl, (C₁-C₆)alkylsulfonyl-(C₁-C₆)alkyl, (C₁-C₆)alkylsulfonyloxy, (C₁-C₆)alkylcarbonyl, (C₁-C₆)alkylthiocarbonyl, (C₁-C₆)haloalkylcarbonyl, (C₁-C₆)alkylcarbonyloxy, (C₁-C₆)alkoxycarbonyl, (C₁-C₆)haloalkoxycarbonyl, aminocarbonyl, (C₁-C₆)alkylaminocarbonyl, (C₁-C₆)alkylaminothiocarbonyl, di(C₁-C₆)alkylaminocarbonyl, di(C₁-C₆)alkylaminothiocarbonyl, (C₂-C₆)alkenylaminocarbonyl, di(C₂-C₆)alkenylaminocarbonyl, (C₃-Cs)cycloalkylaminocarbonyl, (C₁-C₆)alkylsulfonylamino, (C₁-C₆)alkylamino, di(C₁-C₆)alkylamino, aminosulfonyl, (C₁-C₆)alkylaminosulfonyl, di(C₁-C₆)alkylaminosulfonyl, (C₁-C₆)alkylsulfoximino, aminothiocarbonyl, (C₁-C₆)alkylaminothiocarbonyl, di(C₁-C₆)alkylaminothiocarbonyl, (C₃-C₈)cycloalkylamino or - NHCO- (C₁-C₆)alkyl ((C₁-C₆)alkylcarbonylamino) and
R⁶ is (C₁-C₆)alkyl, (C₁-C₆)haloalkyl, (C₁-C₆)cyanoalkyl, (C₁-C₆)hydroxyalkyl, (C₁-C₆)alkoxy-(C₁-C₆)alkyl, (C₁-C₆)haloalkoxy-(C₁-C₆)alkyl, (C₂-C₆)alkenyl, (C₂-C₆)alkenyloxy-(C₁-C₆)alkyl, (C₂-C₆)haloalkenyloxy-(C₁-C₆)alkyl, (C₂-C₆)haloalkenyl, (C₂-C₆)cyanoalkenyl, (C₂-C₆)alkynyl, (C₂-C₆)alkynyloxy-(C₁-C₆)alkyl, (C₂-C₆)haloalkynyloxy-(C₁-C₆)alkyl, (C₂-C₆)haloalkynyl, (C₂-C₆)cyanoalkynyl, (C₃-C₈)cycloalkyl, (C₃-C₈)cycloalkyl-(C₃-C₈)cycloalkyl, (C₁-C₆)alkyl- (C₃-C₈)cycloalkyl, halo (C₃-C₈)cycloalkyl, (C₁-C₆)alkylthio- (C₁-C₆)alkyl, (C₁-C₆)haloalkylthio-(C₁-C₆)alkyl, (C₁-C₆)alkylsulfinyl-(C₁-C₆)alkyl, (C₁-C₆)haloalkylsulfinyl-(C₁-C₆)alkyl, (C₁-C₆)alkylsulfonyl-(C₁-C₆)alkyl, (C₁-C₆)haloalkylsulfonyl-(C₁-C₆)alkyl, (C₁-C₆)alkoxy- (C₁-C₆)alkylthio- (C₁-C₆)alkyl, (C₁-C₆)alkoxy-(C₁-C₆)alkylsulfinyl-(C₁-C₆)alkyl, (C₁-C₆)alkoxy-(C₁-C₆)alkylsulfonyl- (C₁-C₆)alkyl, (C₁-C₆)alkylcarbonyl-(C₁-C₆)alkyl, (C₁-C₆)haloalkylcarbonyl-(C₁-C₆)alkyl, (C₁-C₆)alkoxycarbonyl-(C₁-C₆)alkyl, (C₁-C₆)haloalkoxycarbonyl-(C₁-C₆)alkyl, aminocarbonyl-(C₁-C₆)alkyl, (C₁-C₆)alkylamino- (C₁-C₆)alkyl, di(C₁-C₆)alkylamino-(C₁-C₆)alkyl or (C₃-C₈)cycloalkylamino-(C₁-C₆)alkyl,
**characterized in that**, in a first method step a), a compound of the formula (I) 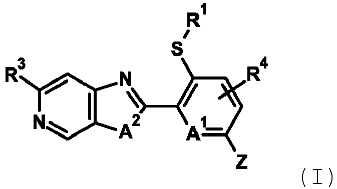
in which Z, R¹, R³, R⁴, A¹ and A² are each as defined above,
is reacted with an organozinc base of the structure (NR^{a}R^{b})-Zn-R^{c} or (NR^{a}R^{b})₂-Zn, in which
R^{c} is halogen or -O-pivaloyl and
R^{a} and R^{b} together form a -(CH₂)₄-, -(CH₂)₅- or - (CH₂)₂O(CH₂)₂- group, where each of these groups may optionally be substituted by 1, 2, 3 or 4 R^{d} radicals and R^{d} is selected from the group consisting of methyl, ethyl, n-propyl and isopropyl,
to give a compound of the formula (IVa) or the formula (IVb), 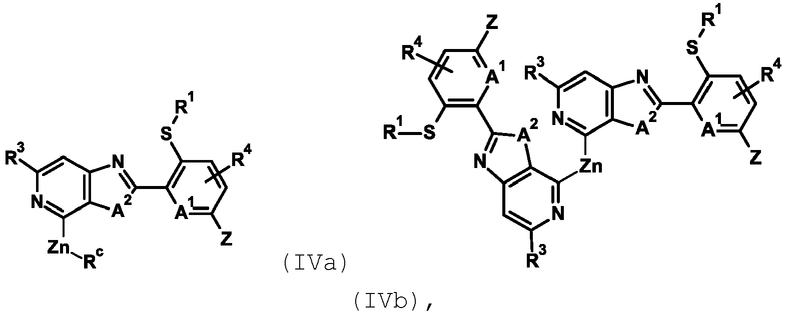 in which Z, R¹, R³, R⁴, R^{c}, A¹ and A² are each as defined above,
and this compound of the formula (IVa) or (IVb) is reacted in a second method step b) with a compound of the structure Q-V, in which V is halogen and Q has the abovementioned definition, to give the compound of the formula (II).

2. Method according to Claim 1, **characterized in that**
Q is chlorine, iodine or bromine,
Z is bromine, fluorine or chlorine,
R^{c} is -O-pivaloyl, chlorine, bromine or iodine,
A¹ is nitrogen, =N⁺-O⁻ or =C-R⁵,
A² is -N-R⁶ or oxygen,
R¹ is (C₁-C₄)alkyl, (C₁-C₄)hydroxyalkyl, (C₁-C₄)haloalkyl, (C₁-C₄)cyanoalkyl, (C₁-C₄)alkoxy-(C₁-C₄)alkyl, (C₁-C₄)haloalkoxy-(C₁-C₄)alkyl, (C₂-C₄)alkenyl, (C₂-C₄)alkenyloxy-(C₁-C₄)alkyl, (C₂-C₄)haloalkenyloxy-(C₁-C₄)alkyl, (C₂-C₄)haloalkenyl, (C₂-C₄)cyanoalkenyl, (C₂-C₄)alkynyl, (C₂-C₄)alkynyloxy-(C₁-C₄)alkyl, (C₂-C₄)haloalkynyloxy-(C₁-C₄)alkyl, (C₂-C₄)haloalkynyl, (C₂-C₄)cyanoalkynyl, (C₃-C₆)cycloalkyl, (C₃-C₆)halocycloalkyl, (C₃-C₆)cyanocycloalkyl, (C₃-C₆)cycloalkyl-(C₃-C₆)cycloalkyl, (C₁-C₄)alkyl-(C₃-C₆)cycloalkyl, halo(C₃-C₆)cycloalkyl, (C₁-C₄)alkylamino, di(C₁-C₄)alkylamino, (C₃-C₆)cycloalkylamino, (C₁-C₄)alkylcarbonylamino, (C₁-C₄)alkylthio-(C₁-C₄)alkyl, (C₁-C₄)haloalkylthio-(C₁-C₄)alkyl, (C₁-C₄)alkylsulfinyl-(C₁-C₄)alkyl, (C₁-C₄)haloalkylsulfinyl-(C₁-C₄)alkyl, (C₁-C₄)alkylsulfonyl-(C₁-C₄)alkyl, (C₁-C₄)alkylcarbonyl-(C₁-C₄)alkyl, (C₁-C₄)haloalkylcarbonyl-(C₁-C₄)alkyl, (C₁-C₄)alkylsulfonylamino,
or is (C₁-C₄)alkyl, (C₁-C₄)alkoxy, (C₂-C₄)alkenyl, (C₂-C₄)alkynyl or (C₃-C₆)cycloalkyl, each of which is optionally mono- or disubstituted by identical or different substituents from the group consisting of aryl, hetaryl and heterocyclyl, where aryl, hetaryl or heterocyclyl may each optionally be mono- or disubstituted by identical or different substituents from the group consisting of halogen, cyano, carbamoyl, aminosulfonyl, (C₁-C₄)alkyl, (C₃-C₄) cycloalkyl, (C₁-C₄)alkoxy, (C₁-C₄)haloalkyl, (C₁-C₄)haloalkoxy, (C₁-C₄)alkylthio, (C₁-C₄)alkylsulfinyl, (C₁-C₄)alkylsulfonyl and (C₁-C₄)alkylsulfimino,
R³ is hydrogen, cyano, halogen, nitro, acetyl, hydroxyl, amino, SCN, tri (C₁-C₄)alkylsilyl, (C₃-C₆)cycloalkyl, (C₃-C₆)cycloalkyl-(C₃-C₆)cycloalkyl, (C₁-C₄)alkyl-(C₃-C₆)cycloalkyl, halo(C₃-C₆)cycloalkyl, cyano (C₃-C₈)cycloalkyl, (C₁-C₄)alkyl, (C₁-C₄)haloalkyl, (C₁-C₄)cyanoalkyl, (C₁-C₄)hydroxyalkyl, (C₁-C₄)alkoxy-(C₁-C₄)alkyl, (C₂-C₄)alkenyl, (C₂-C₄)haloalkenyl, (C₂-C₄)cyanoalkenyl, (C₂-C₄)alkynyl, (C₂-C₄)haloalkynyl, (C₂-C₄)cyanoalkynyl, (C₁-C₄)alkoxy, (C₁-C₄)haloalkoxy, (C₁-C₄)cyanoalkoxy, (C₁-C₄)alkoxy-(C₁-C₄)alkoxy, (C₁-C₄)alkylhydroxyimino, (C₁-C₄)alkoxyimino, (C₁-C₄)alkyl-(C₁-C₄)alkoxyimino, (C₁-C₄)haloalkyl-(C₁-C₄)alkoxyimino, (C₁-C₄)alkylthio, (C₁-C₄)haloalkylthio, (C₁-C₄)alkylthio-(C₁-C₄)alkyl, (C₁-C₄)alkylsulfinyl, (C₁-C₄)haloalkylsulfinyl, (C₁-C₄)alkylsulfinyl-(C₁-C₄)alkyl, (C₁-C₄)alkylsulfonyl, (C₁-C₄)haloalkylsulfonyl, (C₁-C₄)alkylsulfonyl-(C₁-C₄)alkyl, (C₁-C₄)alkylsulfonyloxy, (C₁-C₄)alkylcarbonyl, (C₁-C₄)haloalkylcarbonyl, aminocarbonyl, aminothiocarbonyl, (C₁-C₄)alkylaminocarbonyl, di(C₁-C₄)alkylaminocarbonyl, (C₁-C₄)alkylsulfonylamino, (C₁-C₄)alkylamino, di(C₁-C₄)alkylamino, aminosulfonyl, (C₁-C₄)alkylaminosulfonyl, di(C₁-C₄)alkylaminosulfonyl, aminothiocarbonyl, NHCO-(C₁-C₄)alkyl ((C₁-C₄)alkylcarbonylamino) or
is phenyl or hetaryl, each of which is optionally mono- or disubstituted by identical or different substituents, where (in the case of hetaryl) at least one carbonyl group may optionally be present and where possible substituents in each case are as follows: cyano, halogen, nitro, acetyl, amino, (C₁-C₄)alkyl, (C₁-C₄)haloalkyl, (C₁-C₄)cyanoalkyl, (C₁-C₄)hydroxyalkyl, (C₁-C₄)alkoxy-(C₁-C₄)alkyl, (C₂-C₄)alkenyl, (C₂-C₄)haloalkenyl, (C₂-C₄)cyanoalkenyl, (C₂-C₄)alkynyl, (C₂-C₄)haloalkynyl, (C₂-C₄)cyanoalkynyl, (C₁-C₄)alkoxy, (C₁-C₄)haloalkoxy, (C₁-C₄)cyanoalkoxy, (C₁-C₄)alkoxy-(C₁-C₄)alkoxy, (C₁-C₄)alkylhydroxyimino, (C₁-C₄)alkoxyimino, (C₁-C₄)alkyl-(C₁-C₄)alkoxyimino, (C₁-C₄)haloalkyl-(C₁-C₄)alkoxyimino, (C₁-C₄)alkylthio, (C₁-C₄)haloalkylthio, (C₁-C₄)alkylthio-(C₁-C₄)alkyl, (C₁-C₄)alkylsulfinyl, (C₁-C₄)haloalkylsulfinyl, (C₁-C₄)alkylsulfinyl-(C₁-C₄)alkyl, (C₁-C₄)alkylsulfonyl, (C₁-C₄)haloalkylsulfonyl, (C₁-C₄)alkylsulfonyl-(C₁-C₄)alkyl, (C₁-C₄)alkylsulfonyloxy, (C₁-C₄)alkylcarbonyl, (C₁-C₄)haloalkylcarbonyl, aminocarbonyl, (C₁-C₄)alkylaminocarbonyl, di(C₁-C₄)alkylaminocarbonyl, (C₁-C₄)alkylsulfonylamino, (C₁-C₄)alkylamino, di(C₁-C₄) alkylamino, aminosulfonyl, (C₁-C₄)alkylaminosulfonyl and di(C₁-C₄)alkylaminosulfonyl, R⁴ is hydrogen, cyano, halogen, nitro, acetyl, hydroxyl, amino, SCN, tri(C₁-C₄)alkylsilyl, (C₃-C₆)cycloalkyl, (C₃-C₆)cycloalkyl- (C₃-C₆)cycloalkyl, (C₁-C₄)alkyl- (C₃-C₆)cycloalkyl, halo (C₃-C₆)cycloalkyl, cyano (C₃-C₈)cycloalkyl, (C₁-C₄)alkyl, (C₁-C₄)haloalkyl, (C₁-C₄)cyanoalkyl, (C₁-C₄)hydroxyalkyl, (C₁-C₄)alkoxy-(C₁-C₄)alkyl, (C₂-C₄)alkenyl, (C₂-C₄)haloalkenyl, (C₂-C₄)cyanoalkenyl, (C₂-C₄)alkynyl, (C₂-C₄)haloalkynyl, (C₂-C₄)cyanoalkynyl, (C₁-C₄)alkoxy, (C₁-C₄)haloalkoxy, (C₁-C₄)cyanoalkoxy, (C₁-C₄)alkoxy- (C₁-C₄)alkoxy, (C₁-C₄)alkylhydroxyimino, (C₁-C₄)alkoxyimino, (C₁-C₄)alkyl-(C₁-C₄)alkoxyimino, (C₁-C₄)haloalkyl- (C₁-C₄)alkoxyimino, (C₁-C₄)alkylthio, (C₁-C₄)haloalkylthio, (C₁-C₄)alkylthio-(C₁-C₄)alkyl, (C₁-C₄)alkylsulfinyl, (C₁-C₄)haloalkylsulfinyl, (C₁-C₄)alkylsulfinyl-(C₁-C₄)alkyl, (C₁-C₄)alkylsulfonyl, (C₁-C₄)haloalkylsulfonyl, (C₁-C₄)alkylsulfonyl-(C₁-C₄)alkyl, (C₁-C₄)alkylsulfonyloxy, (C₁-C₄)alkylcarbonyl, (C₁-C₄)haloalkylcarbonyl, aminocarbonyl, aminothiocarbonyl, (C₁-C₄)alkylaminocarbonyl, di(C₁-C₄)alkylaminocarbonyl, (C₁-C₄)alkylsulfonylamino, (C₁-C₄)alkylamino, di(C₁-C₄)alkylamino, aminosulfonyl, (C₁-C₄)alkylaminosulfonyl, di(C₁-C₄)alkylaminosulfonyl, aminothiocarbonyl, NHCO-(C₁-C₄)alkyl ((C₁-C₄)alkylcarbonylamino),
is furthermore phenyl or hetaryl, each of which is optionally mono- or disubstituted by identical or different substituents, where (in the case of hetaryl) at least one carbonyl group may optionally be present and where possible substituents in each case are as follows: cyano, halogen, nitro, acetyl, amino, (C₃-C₆)cycloalkyl, (C₃-C₆)cycloalkyl-(C₃-C₆)cycloalkyl, (C₁-C₄)alkyl-(C3-C₆)cycloalkyl, halo(C₃-C₆)cycloalkyl, (C₁-C₄)alkyl, (C₁-C₄)haloalkyl, (C₁-C₄)cyanoalkyl, (C₁-C₄)hydroxyalkyl, (C₁-C₄)alkoxy-(C₁-C₄)alkyl, (C₂-C₄)alkenyl, (C₂-C₄)haloalkenyl, (C₂-C₄)cyanoalkenyl, (C₂-C₄)alkynyl, (C₂-C₄)haloalkynyl, (C₂-C₄)cyanoalkynyl, (C₁-C₄)alkoxy, (C₁-C₄)haloalkoxy, (C₁-C₄)cyanoalkoxy, (C₁-C₄)alkoxy- (C₁-C₄)alkoxy, (C₁-C₄)alkylhydroxyimino, (C₁-C₄)alkoxyimino, (C₁-C₄)alkyl-(C₁-C₄)alkoxyimino, (C₁-C₄)haloalkyl- (C₁-C₄)alkoxyimino, (C₁-C₄)alkylthio, (C₁-C₄)haloalkylthio, (C₁-C₄)alkylthio-(C₁-C₄)alkyl, (C₁-C₄)alkylsulfinyl, (C₁-C₄)haloalkylsulfinyl, (C₁-C₄)alkylsulfinyl-(C₁-C₄)alkyl, (C₁-C₄)alkylsulfonyl, (C₁-C₄)haloalkylsulfonyl, (C₁-C₄)alkylsulfonyl-(C₁-C₄)alkyl, (C₁-C₄)alkylsulfonyloxy, (C₁-C₄)alkylcarbonyl, (C₁-C₄)haloalkylcarbonyl, aminocarbonyl, (C₁-C₄)alkylaminocarbonyl, di(C₁-C₄)alkylaminocarbonyl, (C₁-C₄)alkylsulfonylamino, (C₁-C₄)alkylamino, di(C₁-C₄)alkylamino, aminosulfonyl, (C₁-C₄)alkylaminosulfonyl, di(C₁-C₄)alkylaminosulfonyl, NHCO-(C₁-C₄)alkyl ((C₁-C₄)alkylcarbonylamino),
R⁵ is hydrogen, cyano, halogen, nitro, acetyl, hydroxyl, amino, SCN, tri(C₁-C₄)alkylsilyl, (C₃-C₆)cycloalkyl, (C₃-C₆)cycloalkyl-(C₃-C₆)cycloalkyl, (C₁-C₄)alkyl-(C₃-C₆)cycloalkyl, halo(C₃-C₆)cycloalkyl, (C₁-C₄)alkyl, (C₁-C₄)haloalkyl, (C₁-C₄)cyanoalkyl, (C₁-C₄)hydroxyalkyl, (C₁-C₄)alkoxy-(C₁-C₄)alkyl, (C₂-C₄)alkenyl, (C₂-C₄)haloalkenyl, (C₂-C₄)cyanoalkenyl, (C₂-C₄)alkynyl, (C₂-C₄)haloalkynyl, (C₂-C₄)cyanoalkynyl, (C₁-C₄)alkoxy, (C₁-C₄)haloalkoxy, (C₁-C₄)cyanoalkoxy, (C₁-C₄)alkoxy-(C₁-C₄)alkoxy, (C₁-C₄)alkylhydroxyimino, (C₁-C₄)alkoxyimino, (C₁-C₄)alkyl-(C₁-C₄)alkoxyimino, (C₁-C₄)haloalkyl-(C₁-C₄)alkoxyimino, (C₁-C₄)alkylthio, (C₁-C₄)haloalkylthio, (C₁-C₄)alkylthio-(C₁-C₄)alkyl, (C₁-C₄)alkylsulfinyl, (C₁-C₄)haloalkylsulfinyl, (C₁-C₄)alkylsulfinyl-(C₁-C₄)alkyl, (C₁-C₄)alkylsulfonyl, (C₁-C₄)haloalkylsulfonyl, (C₁-C₄)alkylsulfonyl-(C₁-C₄)alkyl, (C₁-C₄)alkylsulfonyloxy, (C₁-C₄)alkylcarbonyl, (C₁-C₄)haloalkylcarbonyl, aminocarbonyl, aminothiocarbonyl, (C₁-C₄)alkylaminocarbonyl, di(C₁-C₄)alkylaminocarbonyl, (C₁-C₄)alkylsulfonylamino, (C₁-C₄)alkylamino, di (C₁-C₄)alkylamino, aminosulfonyl, (C₁-C₄)alkylaminosulfonyl, di(C₁-C₄)alkylaminosulfonyl, aminothiocarbonyl or NHCO-(C₁-C₄)alkyl ((C₁-C₄)alkylcarbonylamino) and
R⁶ is (C₁-C₄)alkyl, (C₁-C₄)haloalkyl, (C₁-C₄)cyanoalkyl, (C₁-C₄)hydroxyalkyl, (C₁-C₄)alkoxy- (C₁-C₄)alkyl, (C₁-C₄)haloalkoxy-(C₁-C₄)alkyl, (C₂-C₄)alkenyl, (C₂-C₄)alkenyloxy-(C₁-C₄)alkyl, (C₂-C₄)haloalkenyloxy- (C₁-C₄)alkyl, (C₂-C₄)haloalkenyl, (C₂-C₄)cyanoalkenyl, (C₂-C₄)alkynyl, (C₂-C₄)alkynyloxy-(C₁-C₄)alkyl, (C₂-C₄)haloalkynyl, (C₃-C₆)cycloalkyl, (C₃-C₆)cycloalkyl-(C₃-C₆)cycloalkyl, (C₁-C₄)alkyl-(C₃-C₆)cycloalkyl, halo (C₃-C₆)cycloalkyl, (C₁-C₄)alkylthio-(C₁-C₄)alkyl, (C₁-C₄)haloalkylthio-(C₁-C₄)alkyl, (C₁-C₄)alkylsulfinyl-(C₁-C₄)alkyl, (C₁-C₄)haloalkylsulfinyl-(C₁-C₄)alkyl, (C₁-C₄)alkylsulfonyl-(C₁-C₄)alkyl, (C₁-C₄)haloalkylsulfonyl-(C₁-C₄)alkyl, (C₁-C₄)alkoxy-(C₁-C₄)alkylthio-(C₁-C₄)alkyl or (C₁-C₄)alkylcarbonyl-(C₁-C₄)-alkyl.

3. Method according to Claim 1, **characterized in that**
Q is chlorine, iodine or bromine,
Z is bromine, fluorine or chlorine,
R^{c} is -O-pivaloyl, chlorine, bromine or iodine,
A¹ is nitrogen or =C-R⁵,
A² is -N-R⁶,
R¹ is (C₁-C₄)alkyl, (C₁-C₄)hydroxyalkyl, (C₁-C₄)haloalkyl, (C₂-C₄)alkenyl, (C₂-C₄)haloalkenyl, (C₂-C₄)alkynyl, (C₂-C₄)haloalkynyl, (C₃-C₆)cycloalkyl, (C₁-C₄)alkylthio-(C₁-C₄)alkyl, (C₁-C₄)alkylsulfinyl-(C₁-C₄)alkyl or (C₁-C₄)alkylsulfonyl-(C₁-C₄)alkyl,
R³ is hydrogen, halogen, (C₁-C₄)alkyl, (C₁-C₄)haloalkyl, (C₁-C₄)haloalkoxy, (C₁-C₄)alkylthio, (C₁-C₄)alkylsulfinyl, (C₁-C₄)alkylsulfonyl, (C₁-C₄)haloalkylthio, (C₁-C₄)haloalkylsulfinyl or (C₁-C₄)haloalkylsulfonyl,
R⁴ is hydrogen, cyano, halogen, nitro, hydroxyl, amino, SCN, tri(C₁-C₄)alkylsilyl, (C₃-C₆)cycloalkyl, cyano(C₃-C₈)cycloalkyl, (C₃-C₆)cycloalkyl-(C₃-C₆)cycloalkyl, (C₁-C₄)alkyl-(C₃-C₆)cycloalkyl, halo(C₃-C₆)cycloalkyl, (C₁-C₄)alkyl, (C₁-C₄)haloalkyl, (C₁-C₄)cyanoalkyl, (C₁-C₄)alkoxy-(C₁-C₄)alkyl, (C₂-C₄)alkenyl, (C₂-C₄)haloalkenyl, (C₂-C₄)cyanoalkenyl, (C₂-C₄)alkynyl, (C₂-C₄)haloalkynyl, (C₂-C₄)cyanoalkynyl, (C₁-C₄)alkoxy, (C₁-C₄)haloalkoxy, (C₁-C₄)cyanoalkoxy, (C₁-C₄)alkylhydroxyimino, (C₁-C₄)alkoxyimino, (C₁-C₄)alkyl-(C₁-C₄) alkoxyimino, (C₁-C₄)alkylthio, (C₁-C₄)haloalkylthio, (C₁-C₄)alkylsulfinyl, (C₁-C₄)haloalkylsulfinyl, (C₁-C₄)alkylsulfonyl, (C₁-C₄)haloalkylsulfonyl, (C₁-C₄)alkylsulfonyloxy, (C₁-C₄)alkylcarbonyl, (C₁-C₄)haloalkylcarbonyl, aminocarbonyl, (C₁-C₄)alkylaminocarbonyl, di(C₁-C₄)alkylaminocarbonyl, (C₁-C₄)alkylsulfonylamino, (C₁-C₄)alkylamino, di(C₁-C₄)alkylamino, aminosulfonyl, (C₁-C₄)alkylaminosulfonyl, di(C₁-C₄)alkylaminosulfonyl or NHCO-(C₁-C₄)alkyl ((C₁-C₄)alkylcarbonylamino),
is furthermore phenyl or hetaryl, each of which is optionally mono- or disubstituted by identical or different substituents, where (in the case of hetaryl) at least one carbonyl group may optionally be present and where possible substituents in each case are as follows: cyano, halogen, (C₃-C₆)cycloalkyl, (C₃-C₆)cycloalkyl-(C₃-C₆)cycloalkyl, (C₁-C₄)alkyl-(C₃-C₆)cycloalkyl, halo(C₃-C₆)cycloalkyl, (C₁-C₄)alkyl, (C₁-C₄)haloalkyl, (C₁-C₄)cyanoalkyl, (C₂-C₄)alkenyl, (C₂-C₄)haloalkenyl, (C₂-C₄)cyanoalkenyl, (C₂-C₄)alkynyl, (C₂-C₄)haloalkynyl, (C₂-C₄)cyanoalkynyl, (C₁-C₄)alkoxy, (C₁-C₄)haloalkoxy, (C₁-C₄)alkylhydroxyimino, (C₁-C₄)alkoxyimino, (C₁-C₄)alkyl-(C₁-C₄)alkoxyimino, (C₁-C₄)alkylthio, (C₁-C₄)haloalkylthio, (C₁-C₄)alkylsulfinyl, (C₁-C₄)haloalkylsulfinyl, (C₁-C₄)alkylsulfonyl, (C₁-C₄)haloalkylsulfonyl, (C₁-C₄)alkylsulfonyloxy, (C₁-C₄)alkylcarbonyl, (C₁-C₄)haloalkylcarbonyl, aminocarbonyl, (C₁-C₄)alkylaminocarbonyl, di(C₁-C₄)alkylaminocarbonyl, (C₁-C₄)alkylsulfonylamino, (C₁-C₄)alkylamino, di(C₁-C₄)alkylamino, aminosulfonyl, (C₁-C₄)alkylaminosulfonyl, di(C₁-C₄)alkylaminosulfonyl, NHCO-(C₁-C₄)alkyl ((C₁-C₄)alkylcarbonylamino),
R⁵ is hydrogen, halogen, cyano or (C₁-C₄)alkyl and
R⁶ is (C₁-C₄)alkyl or (C₁-C₄)alkoxy- (C₁-C₄)alkyl.

4. Method according to Claim 1, **characterized in that**
Q is iodine or bromine,
Z is bromine or chlorine,
R^{c} is chlorine, bromine or iodine,
A¹ is nitrogen or =C-R⁵,
A² is -N-R⁶,
R¹ is methyl, ethyl, n-propyl, isopropyl, cyclopropyl, n-butyl, isobutyl, tert-butyl, cyclobutyl, fluoromethyl, difluoromethyl, trifluoromethyl, fluoroethyl, difluoroethyl, trifluoroethyl, tetrafluoroethyl or pentafluoroethyl,
R³ is fluorine, chlorine, fluoromethyl, difluoromethyl, trifluoromethyl, fluoroethyl, difluoroethyl, trifluoroethyl, tetrafluoroethyl, pentafluoroethyl, trifluoromethoxy, difluorochloromethoxy, dichlorofluoromethoxy, trifluoromethylthio, trifluoromethylsulfonyl or trifluoromethylsulfinyl,
R⁴ is hydrogen, cyano, halogen, (C₁-C₄)alkyl, (C₁-C₄)haloalkyl, (C₁-C₄)haloalkoxy, (C₁-C₄)alkylthio, (C₁-C₄)alkylsulfinyl, (C₁-C₄)alkylsulfonyl, (C₁-C₄)haloalkylthio, (C₁-C₄)haloalkylsulfinyl, (C₁-C₄)haloalkylsulfonyl or NHCO- (C₁-C₄)alkyl ((C₁-C₄)alkylcarbonylamino),
R⁵ is hydrogen, fluorine, chlorine, bromine or cyano and
R⁶ is methyl, ethyl, isopropyl, methoxymethyl or methoxyethyl.

5. Method according to Claim 1, **characterized in that**
Q is iodine or bromine,
Z is bromine or chlorine,
R^{c} is chlorine or bromine,
A¹ is nitrogen,
A² is -N-R⁶,
R¹ is methyl, ethyl, n-propyl, isopropyl or cyclopropyl,
R³ is trifluoromethyl or pentafluoroethyl,
R⁴ is hydrogen and
R⁶ is methyl.

6. Method according to Claim 1, **characterized in that**
Q is iodine or bromine,
Z is bromine or chlorine,
R^{c} is chlorine,
A¹ is nitrogen,
A² is -N-R⁶,
R¹ is ethyl,
R³ is trifluoromethyl,
R⁴ is hydrogen and
R⁶ is methyl.

7. Method according to any of Claims 1 to 6, **characterized in that** the organozinc base is a compound of the formula (VI)
(VI) (TMP)ₓZnCl₂₋ₓ,
in which x is the number 1 or 2.

8. Method according to any of Claims 1 to 7, **characterized in that** the organozinc base is present in conjunction with an alkali metal halide or alkaline earth metal halide, preferably lithium chloride and/or magnesium chloride.

9. Method according to any of Claims 1 to 8, **characterized in that** the compound Q-V is an elemental halogen, especially F₂, Cl₂, Br₂ or I₂.

10. Method according to any of Claims 1 to 9, **characterized in that** the solvent is THF or N,N-dimethylformamide (DMF).

11. Method according to any of Claims 1 to 10, **characterized in that** method step a) is conducted at a temperature between 0°C and 80°C.

12. Method according to any of Claims 1 to 11, **characterized in that** method step b) is conducted at a temperature between 0°C and 80°C.

13. Method according to any of Claims 1 to 12, **characterized in that** the compounds of the formula (II) are further reacted in a third step c) to give compounds of the formula (III) 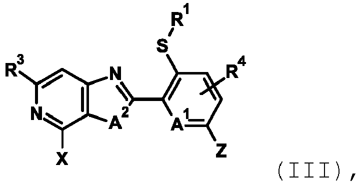
in which Z, R¹, R³, R⁴, A¹ and A² are as defined according to any of Claims 1 to 6, and
X is cyano, carboxyl, halogen, nitro, acetyl, hydroxyl, amino, SCN, tri (C₁-C₆) alkylsilyl, (C₁-C₆) alkyl, (C₁-C₆) haloalkyl, (C₁-C₆) cyanoalkyl, (C₁-C₆)hydroxyalkyl, hydroxycarbonyl- (C₁-C₆) -alkoxy, (C₁-C₆) alkoxycarbonyl-(C₁-C₆) alkyl, (C₁-C₆) alkoxy- (C₁-C₆) alkyl, (C₂-C₆)alkenyl, (C₂-C₆)haloalkenyl, (C₂-C₆)cyanoalkenyl, (C₂-C₆)alkynyl, (C₂-C₆)haloalkynyl, (C₂-C₆)cyanoalkynyl, (C₃-C₆)cycloalkyl, (C₃-C₆)halocycloalkyl, (C₃-C₆)cyanocycloalkyl, (C₃-C₆)cycloalkyl- (C₃-C₆)cycloalkyl, (C₁-C₄)alkyl-(C₃-C₆)cycloalkyl (C₁-C₆) alkoxy, (C₁-C₆)haloalkoxy, (C₁-C₆) cyanoalkoxy, (C₁-C₆) alkoxycarbonyl-(C₁-C₆) alkoxy, (C₁-C₆) alkoxy- (C₁-C₆) alkoxy, (C₁-C₆)alkoxyimino, -N=C(H)-O(C₁-C₆) alkyl, -C(H)=N-O(C₁-C₆)alkyl, (C₁-C₆) haloalkyl- (C₁-C₆) alkoxyimino, (C₁-C₆)alkylthio, (C₁-C₆)haloalkylthio, (C₁-C₆) alkoxy-(C₁-C₆)alkylthio, (C₁-C₆) alkylthio- (C₁-C₆) alkyl, (C₁-C₆)alkylsulfinyl, (C₁-C₆) haloalkylsulfinyl, (C₁-C₆)alkoxy- (C₁-C₆) alkylsulfinyl, (C₁-C₆) alkylsulfinyl-(C₁-C₆)alkyl, (C₁-C₆) alkylsulfonyl, (C₁-C₆) haloalkylsulfonyl, (C₁-C₆) alkoxy- (C₁-C₆) alkylsulfonyl, (C₁-C₆)alkylsulfonyl-(C₁-C₆) alkyl, (C₁-C₆) alkylsulfonyloxy, (C₁-C₆)alkylcarbonyl, (C₁-C₆) haloalkylcarbonyl, (C₁-C₆)alkylcarbonyloxy, (C₁-C₆) alkoxycarbonyl, (C₁-C₆)haloalkoxycarbonyl, aminocarbonyl, (C₁-C₆)alkylaminocarbonyl, di(C₁-C₆)alkylaminocarbonyl, (C₂-C₆)alkenylaminocarbonyl, di(C₂-C₆)alkenylaminocarbonyl, (C₃-C₈)cycloalkylaminocarbonyl, (C₁-C₆)alkylsulfonylamino, (C₁-C₆)alkylamino, di(C₁-C₆)alkylamino, aminosulfonyl, (C₁-C₆)alkylaminosulfonyl, di(C₁-C₆)alkylaminosulfonyl, (C₁-C₆)alkylsulfoximino, aminothiocarbonyl, (C₁-C₆)alkylaminothiocarbonyl, di(C₁-C₆)alkylaminothiocarbonyl, (C₃-C₈)cycloalkylamino, (C₁-C₆)alkylcarbamoyl (including -NHCOO(C₁-C₆) alkyl, -N(C₁-C₆)alkylCOO(C₁-C₆) alkyl, -OCONH(C₁-C₆) alkyl or -OCON(C₁-C₆)dialkyl), (C₁-C₆)alkylcarbonylamino ((C₁-C₆)alkylCONH), (C₁-C₆)alkylurea (including -NHCONH(C₁-C₆)alkyl, and -NHCON(C₁-C₆) dialkyl) or is a saturated, partially saturated or heteroaromatic ring which is optionally mono- or polysubstituted by identical or different substituents and in which at least one carbon atom is replaced by a heteroatom, or is an aromatic ring which is mono- or polysubstituted by identical or different substituents, where at least one carbonyl group may optionally be present in each case and where possible substituents in each case are as follows: cyano, carboxyl, halogen, nitro, acetyl, hydroxyl, amino, SCN, tri (C₁-C₆) alkylsilyl, (C₁-C₆) alkyl, (C₁-C₆)haloalkyl, (C₁-C₆)cyanoalkyl, (C₁-C₆)hydroxyalkyl, hydroxycarbonyl-(C₁-C₆)-alkoxy, (C₁-C₆)alkoxycarbonyl- (C₁-C₆)alkyl, (C₁-C₆)alkoxy- (C₁-C₆)alkyl, (C₂-C₆)alkenyl, (C₂-C₆)haloalkenyl, (C₂-C₆)cyanoalkenyl, (C₂-C₆)alkynyl, (C₂-C₆)haloalkynyl, (C₂-C₆)cyanoalkynyl, (C₃-C₆)cycloalkyl, (C₃-C₆)halocycloalkyl, (C₃-C₆)cyanocycloalkyl, (C₃-C₆)cycloalkyl-(C₃-C₆)cycloalkyl, (C₁-C₄)alkyl-(C₃-C₆)cycloalkyl (C₁-C₆) alkoxy, (C₁-C₆)haloalkoxy, (C₁-C₆)cyanoalkoxy, (C₁-C₆)alkoxycarbonyl- (C₁-C₆)alkoxy, (C₁-C₆)alkoxy-(C₁-C₆) alkoxy, (C₁-C₆)alkoxyimino, -N=C(H)-O(C₁-C₆)alkyl, -C(H)=N-O(C₁-C₆) alkyl, (C₁-C₆)haloalkyl- (C₁-C₆)alkoxyimino, (C₁-C₆) alkylthio, (C₁-C₆)haloalkylthio, (C₁-C₆)alkoxy- (C₁-C₆)alkylthio, (C₁-C₆)alkylthio-(C₁-C₆)alkyl, (C₁-C₆) alkylsulfinyl, (C₁-C₆)haloalkylsulfinyl, (C₁-C₆) alkoxy- (C₁-C₆) alkylsulfinyl, (C₁-C₆) alkylsulfinyl-(C₁-C₆) alkyl, (C₁-C₆) alkylsulfonyl, (C₁-C₆)haloalkylsulfonyl, (C₁-C₆) alkoxy- (C₁-C₆) alkylsulfonyl, (C₁-C₆) alkylsulfonyl- (C₁-C₆) alkyl, (C₁-C₆)alkylsulfonyloxy, (C₁-C₆) alkylcarbonyl, (C₁-C₆)haloalkylcarbonyl, (C₁-C₆)alkylcarbonyloxy, (C₁-C₆)alkoxycarbonyl, (C₁-C₆) haloalkoxycarbonyl, aminocarbonyl, (C₁-C₆)alkylaminocarbonyl, di(C₁-C₆)alkylaminocarbonyl, (C₂-C₆)alkenylaminocarbonyl, di(C₂-C₆)alkenylaminocarbonyl, (C₃-Cs)cycloalkylaminocarbonyl, (C₁-C₆)alkylsulfonylamino, (C₁-C₆) alkylamino, di (C₁-C₆) alkylamino, aminosulfonyl, (C₁-C₆) alkylaminosulfonyl, di(C₁-C₆) alkylaminosulfonyl, (C₁-C₆)alkylsulfoximino, aminothiocarbonyl, (C₁-C₆)alkylaminothiocarbonyl, di(C₁-C₆)alkylaminothiocarbonyl, (C₃-C₈)cycloalkylamino or (C₁-C₆)alkylcarbonylamino.

14. Method according to Claim 13, **characterized in that** X is cyano, halogen, nitro, acetyl, amino, (C₁-C₄)alkyl, (C₁-C₄)haloalkyl, (C₁-C₄)cyanoalkyl, (C₁-C₄)hydroxyalkyl, (C₁-C₄)alkoxy-(C₁-C₄)alkyl, (C₂-C₄)alkenyl, (C₂-C₄)haloalkenyl, (C₂-C₄)cyanoalkenyl, (C₂-C₄)alkynyl, (C₂-C₄)haloalkynyl, (C₂-C₄)cyanoalkynyl, (C₃-C₆)cycloalkyl, (C₃-C₆)halocycloalkyl, (C₃-C₆)cyanocycloalkyl, (C₃-C₆)cycloalkyl-(C₃-C₆)cycloalkyl, (C₁-C₄)alkyl-(C₃-C₆)cycloalkyl, (C₁-C₄)alkoxy, (C₁-C₄)haloalkoxy, (C₁-C₄)cyanoalkoxy, (C₁-C₄)alkoxy- (C₁-C₄)alkoxy, (C₁-C₄)alkoxyimino, -N=C(H)-O(C₁-C₄)alkyl, - C(H)=N-O(C₁-C₄)alkyl, (C₁-C₄)haloalkyl-(C₁-C₄)alkoxyimino, (C₁-C₄)alkylthio, (C₁-C₄)haloalkylthio, (C₁-C₄)alkylthio-(C₁-C₄)alkyl, (C₁-C₄)alkylsulfinyl, (C₁-C₄)haloalkylsulfinyl, (C₁-C₄)alkylsulfinyl-(C₁-C₄)alkyl, (C₁-C₄)alkylsulfonyl, (C₁-C₄)haloalkylsulfonyl, (C₁-C₄)alkylsulfonyl-(C₁-C₄)alkyl, (C₁-C₄)alkylsulfonyloxy, (C₁-C₄)alkylcarbonyl, (C₁-C₄)haloalkylcarbonyl, aminocarbonyl, (C₁-C₄)alkylaminocarbonyl, di(C₁-C₄)alkylaminocarbonyl, (C₁-C₄)alkylsulfonylamino, (C₁-C₄)alkylamino, di(C₁-C₄)alkylamino, aminosulfonyl, (C₁-C₄)alkylaminosulfonyl, di(C₁-C₄)alkylaminosulfonyl, (C₁-C₄)alkylcarbamoyl (including -NHCOO(C₁-C₄)alkyl, -N(C₁-C₄)alkylCOO(C₁-C₄)alkyl, -OCONH(C₁-C₄)alkyl or -OCON(C₁-C₄)dialkyl), (C₁-C₄)alkylcarbonylamino ((C₁-C₄)alkylCONH) or (C₁-C₄)alkylurea (including -NHCONH(C₁-C₆) alkyl and -NHCON(C₁-C₆) dialkyl) .

15. Method according to Claim 13, **characterized in that** X is cyano, halogen, nitro, acetyl, amino, (C₁-C₄)alkyl, (C₁-C₄)haloalkyl, (C₁-C₄)cyanoalkyl, (C₁-C₄)hydroxyalkyl, (C₁-C₄)alkoxy-(C₁-C₄)alkyl, (C₂-C₄)alkenyl, (C₂-C₄)haloalkenyl, (C₂-C₄)cyanoalkenyl, (C₂-C₄)alkynyl, (C₂-C₄)haloalkynyl, (C₂-C₄)cyanoalkynyl, (C₃-C₆)cycloalkyl, (C₃-C₆)halocycloalkyl, (C₃-C₆)cyanocycloalkyl, (C₃-C₆)cycloalkyl-(C₃-C₆)cycloalkyl, (C₁-C₄)alkyl-(C₃-C₆)cycloalkyl, (C₁-C₄) alkoxy, (C₁-C₄)haloalkoxy, (C₁-C₄) cyanoalkoxy or (C₁-C₄) alkoxy-(C₁-C₄)alkoxy.

16. Method according to Claim 13, **characterized in that** X is cyano, halogen, amino, (C₁-C₄) alkyl, (C₁-C₄)haloalkyl, (C₁-C₄) cyanoalkyl, (C₁-C₄)hydroxyalkyl, (C₁-C₄)alkoxy- (C₁-C₄) alkyl, (C₂-C₄)alkenyl, (C₂-C₄)haloalkenyl, (C₂-C₄)cyanoalkenyl, (C₂-C₄)alkynyl, (C₂-C₄)haloalkynyl, (C₂-C₄)cyanoalkynyl, (C₃-C₆)cycloalkyl, (C₃-C₆)halocycloalkyl, (C₃-C₆)cyanocycloalkyl, (C₃-C₆)cycloalkyl-(C₃-C₆)cycloalkyl, ((C₁-C₄) alkoxy or (C₁-C₄)cyanoalkoxy.

17. Method according to any of Claims 13 to 16, **characterized in that** the reaction in method step c) is conducted at a temperature between 0°C and 110°C.

18. Method according to any of Claims 13 to 17, **characterized in that** the compounds of the formula (II) are reacted with organozinc reagents in the presence of a catalyst or with metal salts.

19. Compounds of the formula (IVa) 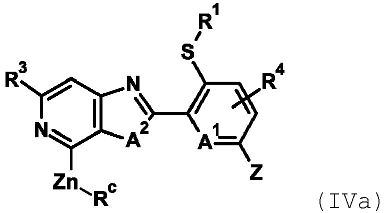 in which Z, R¹, R³, R⁴, R^{c}, A¹ and A² are as defined according to any of Claims 1 to 6.

20. Compounds of the formula (IVb) 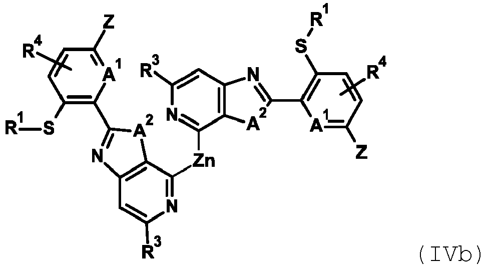 in which Z, R¹, R³, R⁴, A¹ and A² are as defined according to any of Claims 1 to 6.

21. Compounds of the formula (II) 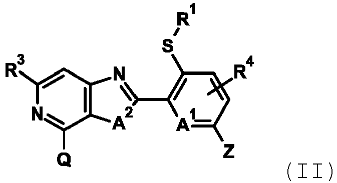 in which Q, Z, R¹, R³, R⁴, A¹ and A² are as defined according to any of Claims 1 to 6.

## Revendications

1. Procédé pour la préparation de composés de formule (II) 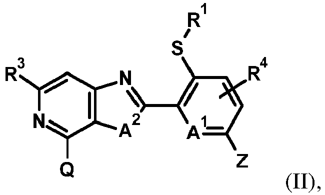 dans laquelle
Q et Z représentent, indépendamment l'un de l'autre, halogéno,
A¹ représente azote, =N⁺-O⁻ ou =C-R⁵,
A² représente -N-R⁶, oxygène ou soufre,
R¹ représente (C₁-C₆)alkyle, (C₁-C₆)halogénoalkyle, (C₁-C₆)cyanoalkyle, (C₁-C₆)hydroxyalkyle, (C₁-C₆)alcoxy-(C₁-C₆)alkyle, (C₁-C₆)halogénoalcoxy-(C₁-C₆)alkyle, (C₂-C₆)alcényle, (C₂-C₆)alcényloxy-(C₁-C₆)alkyle, (C₂-C₆)halogénoalcényloxy-(C₁-C₆)alkyle, (C₂-C₆)halogénoalcényle, (C₂-C₆)cyanoalcényle, (C₂-C₆)alcynyle, (C₂-C₆)alcynyloxy-(C₁-C₆)alkyle, (C₂-C₆)halogénoalcynyloxy-(C₁-C₆)alkyle, (C₂-C₆)halogénoalcynyle, (C₂-C₆)cyanoalcynyle, (C₃-C₈)cycloalkyle, (C₃-C₆)halogénocycloalkyle, (C₃-C₆)cyanocycloalkyle, (C₃-C₈)cycloalkyl-(C₃-C₈)cycloalkyle, (C₁-C₆)alkyl-(C₃-C₈)cycloalkyle, halogéno (C₃-C₈)cycloalkyle, amino, (C₁-C₆)alkylamino, di-(C₁-C₆)alkylamino, (C₃-C₈)cycloalkylamino, (C₁-C₆)alkylcarbonylamino, (C₁-C₆)alkylthio-(C₁-C₆)alkyle, (C₁-C₆)halogénoalkylthio-(C₁-C₆)alkyle, (C₁-C₆)alkylsulfinyl-(C₁-C₆)alkyle, (C₁-C₆)halogénoalkylsulfinyl-(C₁-C₆)alkyle, (C₁-C₆)alkylsulfonyl-(C₁-C₆)alkyle, (C₁-C₆)halogénoalkylsulfonyl-(C₁-C₆)alkyle, (C₁-C₆)alcoxy-(C₁-C₆)alkylthio-(C₁-C₆)alkyle, (C₁-C₆)alcoxy-(C₁-C₆)alkylsulfinyl-(C₁-C₆)alkyle, (C₁-C₆)alcoxy-(C₁-C₆)alkylsulfonyl-(C₁-C₆)alkyle, (C₁-C₆)alkylcarbonyl-(C₁-C₆)alkyle, (C₁-C₆)halogénoalkylcarbonyl-(C₁-C₆)alkyle, (C₁-C₆)alcoxycarbonyl-(C₁-C₆)alkyle, (C₁-C₆)halogénoalcoxycarbonyl-(C₁-C₆) alkyle, (C₁-C₆)alkylsulfonylamino, aminosulfonyl-(C₁-C₆)alkyle, (C₁-C₆)alkylaminosulfonyl-(C₁-C₆)alkyle, di-(C₁-C₆)alkylaminosulfonyl-(C₁-C₆)alkyle, ou représente à chaque fois (C₁-C₆)alkyle, (C₁-C₆)alcoxy, (C₂-C₆)alcényle, (C₂-C₆)alcynyle, (C₃-Cs)cycloalkyle le cas échéant monosubstitué ou polysubstitué, de manière identique ou différente, par aryle, hétéroaryle ou hétérocyclyle, où aryle, hétéroaryle ou hétérocyclyle peuvent à chaque fois le cas échéant être monosubstitués ou polysubstitués, de manière identique ou différente, par halogéno, cyano, nitro, hydroxy, amino, carboxy, carbamoyle, aminosulfonyle, (C₁-C₆)alkyle, (C₃-C₆)cycloalkyle, (C₁-C₆)alcoxy, (C₁-C₆)halogénoalkyle, (C₁-C₆)halogénoalcoxy, (C₁-C₆)alkylthio, (C₁-C₆)alkylsulfinyle, (C₁-C₆)alkylsulfonyle, (C₁-C₆)alkylsulfimino, (C₁-C₆)alkylsulfimino-(C₁-C₆)alkyle, (C₁-C₆)alkylsulfimino-(C₂-C₆)alkylcarbonyle, (C₁-C₆)alkylsulfoximino, (C₁-C₆)alkylsulfoximino-(C₁-C₆)alkyle, (C₁-C₆)alkylsulfoximino-(C₂-C₆)alkylcarbonyle, (C₁-C₆)alcoxycarbonyle, (C₁-C₆)alkylcarbonyle, (C₃-C₆)trialkylsilyle ou benzyle,
R³ représente hydrogène, cyano, halogéno, nitro, acétyle, hydroxy, amino, SCN, tri-(C₁-C₆)alkylsilyle, (C₃-C₈)cycloalkyle, (C₃-C₈)cycloalkyl-(C₃-C₈)cycloalkyle, (C₁-C₆)alkyl-(C₃-C₈)cycloalkyle, halogéno(C₃-C₈)cycloalkyle, cyano(C₃-C₈)cycloalkyle, (C₁-C₆)alkyle, (C₁-C₆)halogénoalkyle, (C₁-C₆)cyanoalkyle, (C₁-C₆)hydroxyalkyle, hydroxycarbonyl-(C₁-C₆)-alcoxy, (C₁-C₆)alcoxycarbonyl-(C₁-C₆)alkyle, (C₁-C₆)alcoxy-(C₁-C₆)alkyle, (C₂-C₆)alcényle, (C₂-C₆)halogénoalcényle, (C₂-C₆)cyanoalcényle, (C₂-C₆)alcynyle, (C₂-C₆)halogénoalcynyle, (C₂-C₆)cyanoalcynyle, (C₁-C₆)alcoxy, (C₁-C₆)halogénoalcoxy, (C₁-C₆)cyanoalcoxy, (C₁-C₆)alcoxycarbonyl-(C₁-C₆)alcoxy, (C₁-C₆)alcoxy-(C₁-C₆)alcoxy, (C₁-C₆)alkylhydroxyimino, (C₁-C₆)alcoxyimino, (C₁-C₆)alkyl-(C₁-C₆)alcoxyimino, (C₁-C₆)halogénoalkyl-(C₁-C₆)alcoxyimino, (C₁-C₆)alkylthio, (C₁-C₆)halogénoalkylthio, (C₁-C₆)alcoxy-(C₁-C₆)alkylthio, (C₁-C₆)alkylthio-(C₁-C₆)alkyle, (C₁-C₆)alkylsulfinyle, (C₁-C₆)halogénoalkylsulfinyle, (C₁-C₆)alcoxy-(C₁-C₆)alkylsulfinyle, (C₁-C₆)alkylsulfinyl-(C₁-C₆)alkyle, (C₁-C₆)alkylsulfonyle, (C₁-C₆)halogénoalkylsulfonyle, (C₁-C₆)alcoxy-(C₁-C₆)alkylsulfonyle, (C₁-C₆)alkylsulfonyl-(C₁-C₆)alkyle, (C₁-C₆)alkylsulfonyloxy, (C₁-C₆)alkylcarbonyle, (C₁-C₆)alkylthiocarbonyle, (C₁-C₆)halogénoalkylcarbonyle, (C₁-C₆)alkylcarbonyloxy, (C₁-C₆)alcoxycarbonyle, (C₁-C₆)halogénoalcoxycarbonyle, aminocarbonyle, (C₁-C₆)alkylaminocarbonyle, (C₁-C₆)alkylaminothiocarbonyle, di-(C₁-C₆)alkylaminocarbonyle, di-(C₁-C₆)alkylaminothiocarbonyle, (C₂-C₆)alcénylaminocarbonyle, di-(C₂-C₆)-alcénylaminocarbonyle, (C₃-Cs)cycloalkylaminocarbonyle, (C₁-C₆)alkylsulfonylamino, (C₁-C₆)alkylamino, di-(C₁-C₆)alkylamino, aminosulfonyle, (C₁-C₆)alkylaminosulfonyle, di-(C₁-C₆)alkylaminosulfonyle, (C₁-C₆)alkylsulfoximino, aminothiocarbonyle, (C₁-C₆)alkylaminothiocarbonyle, di-(C₁-C₆)alkylaminothiocarbonyle, (C₃-C₈)cycloalkylamino, -NHCO-(C₁-C₆)alkyl((C₁-C₆)alkylcarbonylamino) ou représente à chaque fois aryle ou hétéroaryle, le cas échéant monosubstitué ou polysubstitué, de manière identique ou différente, où (dans le cas de hétéroaryle), le cas échéant au moins un groupe carbonyle peut être contenu et où les substituants qui entrent à chaque fois en ligne de compte sont : cyano, carboxyle, halogéno, nitro, acétyle, hydroxy, amino, SCN, tri-(C₁-C₆)alkylsilyle, (C₁-C₆)alkyle, (C₁-C₆)halogénoalkyle, (C₁-C₆)cyanoalkyle, (C₁-C₆)hydroxyalkyle, hydroxycarbonyl-(C₁-C₆)-alcoxy, (C₁-C₆)alcoxycarbonyl-(C₁-C₆)alkyle, (C₁-C₆)alcoxy-(C₁-C₆)alkyle, (C₂-C₆)alcényle, (C₂-C₆)halogénoalcényle, (C₂-C₆)cyanoalcényle, (C₂-C₆)alcynyle, (C₂-C₆)halogénoalcynyle, (C₂-C₆)cyanoalcynyle, (C₁-C₆)alcoxy, (C₁-C₆)halogénoalcoxy, (C₁-C₆)cyanoalcoxy, (C₁-C₆)alcoxycarbonyl-(C₁-C₆)alcoxy, (C₁-C₆)alcoxy-(C₁-C₆)alcoxy, (C₁-C₆)alkylhydroxyimino, (C₁-C₆)alcoxyimino, (C₁-C₆)alkyl-(C₁-C₆)alcoxyimino, (C₁-C₆)halogénoalkyl-(C₁-C₆)alcoxyimino, (C₁-C₆)alkylthio, (C₁-C₆)halogénoalkylthio, (C₁-C₆)alcoxy-(C₁-C₆)alkylthio, (C₁-C₆)alkylthio-(C₁-C₆)alkyle, (C₁-C₆)alkylsulfinyle, (C₁-C₆)halogénoalkylsulfinyle, (C₁-C₆)alcoxy-(C₁-C₆)alkylsulfinyle, (C₁-C₆)alkylsulfinyl-(C₁-C₆)alkyle, (C₁-C₆)alkylsulfonyle, (C₁-C₆)halogénoalkylsulfonyle, (C₁-C₆)alcoxy-(C₁-C₆)alkylsulfonyle, (C₁-C₆)alkylsulfonyl-(C₁-C₆)alkyle, (C₁-C₆)alkylsulfonyloxy, (C₁-C₆)alkylcarbonyle, (C₁-C₆)halogénoalkylcarbonyle, (C₁-C₆)alkylcarbonyloxy, (C₁-C₆)alcoxycarbonyle, (C₁-C₆)halogénoalcoxycarbonyle, aminocarbonyle, (C₁-C₆)alkylaminocarbonyle, di-(C₁-C₆)alkylaminocarbonyle, (C₂-C₆)alcénylaminocarbonyle, di-(C₂-C₆)-alcénylaminocarbonyle, (C₃-C₈)cycloalkylaminocarbonyle, (C₁-C₆)alkylsulfonylamino, (C₁-C₆)alkylamino, di-(C₁-C₆)alkylamino, aminosulfonyle, (C₁-C₆)alkylaminosulfonyle, di-(C₁-C₆)alkylaminosulfonyle, (C₁-C₆)alkylsulfoximino, aminothiocarbonyle, (C₁-C₆)alkylaminothiocarbonyle, di-(C₁-C₆)alkylaminothiocarbonyle ou (C₃-C₈)cycloalkylamino,
R⁴ représente hydrogène, cyano, halogéno, nitro, acétyle, hydroxy, amino, SCN, tri-(C₁-C₆)alkylsilyle, (C₃-C₈)cycloalkyle, (C₃-C₈)cycloalkyl-(C₃-C₈)cycloalkyle, (C₁-C₆)alkyl-(C₃-C₈)cycloalkyle, halogéno(C₃-C₈)cycloalkyle, cyano(C₃-C₈)cycloalkyle, (C₁-C₆)alkyle, (C₁-C₆)halogénoalkyle, (C₁-C₆)cyanoalkyle, (C₁-C₆)hydroxyalkyle, hydroxycarbonyl-(C₁-C₆)-alcoxy, (C₁-C₆)alcoxycarbonyl-(C₁-C₆)alkyle, (C₁-C₆)alcoxy-(C₁-C₆)alkyle, (C₂-C₆)alcényle, (C₂-C₆)halogénoalcényle, (C₂-C₆)cyanoalcényle, (C₂-C₆)alcynyle, (C₂-C₆)halogénoalcynyle, (C₂-C₆)cyanoalcynyle, (C₁-C₆)alcoxy, (C₁-C₆)halogénoalcoxy, (C₁-C₆)cyanoalcoxy, (C₁-C₆)alcoxycarbonyl-(C₁-C₆)alcoxy, (C₁-C₆)alcoxy-(C₁-C₆)alcoxy, (C₁-C₆)alkylhydroxyimino, (C₁-C₆)alcoxyimino, (C₁-C₆)alkyl-(C₁-C₆)alcoxyimino, (C₁-C₆)halogénoalkyl-(C₁-C₆)alcoxyimino, (C₁-C₆)alkylthio, (C₁-C₆)halogénoalkylthio, (C₁-C₆)alcoxy-(C₁-C₆)alkylthio, (C₁-C₆)alkylthio-(C₁-C₆)alkyle, (C₁-C₆)alkylsulfinyle, (C₁-C₆)halogénoalkylsulfinyle, (C₁-C₆)alcoxy-(C₁-C₆)alkylsulfinyle, (C₁-C₆)alkylsulfinyl-(C₁-C₆)alkyle, (C₁-C₆)alkylsulfonyle, (C₁-C₆)halogénoalkylsulfonyle, (C₁-C₆)alcoxy-(C₁-C₆)alkylsulfonyle, (C₁-C₆)alkylsulfonyl-(C₁-C₆)alkyle, (C₁-C₆)alkylsulfonyloxy, (C₁-C₆)alkylcarbonyle, (C₁-C₆)alkylthiocarbonyle, (C₁-C₆)halogénoalkylcarbonyle, (C₁-C₆)alkylcarbonyloxy, (C₁-C₆)alcoxycarbonyle, (C₁-C₆)halogénoalcoxycarbonyle, aminocarbonyle, (C₁-C₆)alkylaminocarbonyle, (C₁-C₆)alkylaminothiocarbonyle, di-(C₁-C₆)alkylaminocarbonyle, di-(C₁-C₆)alkylaminothiocarbonyle, (C₂-C₆)alcénylaminocarbonyle, di-(C₂-C₆)-alcénylaminocarbonyle, (C₃-C₈)cycloalkylaminocarbonyle, (C₁-C₆)alkylsulfonylamino, (C₁-C₆)alkylamino, di-(C₁-C₆)alkylamino, aminosulfonyle, (C₁-C₆)alkylaminosulfonyle, di-(C₁-C₆)alkylaminosulfonyle, (C₁-C₆)alkylsulfoximino, aminothiocarbonyle, (C₁-C₆)alkylaminothiocarbonyle, di-(C₁-C₆)alkylaminothiocarbonyle, (C₃-C₈)cycloalkylamino, NHCO-(C₁-C₆)alkyl((C₁-C₆)alkylcarbonylamino), ou représente à chaque fois aryle ou hétéroaryle, le cas échéant monosubstitué ou polysubstitué, de manière identique ou différente, où (dans le cas de hétéroaryle), le cas échéant au moins un groupe carbonyle peut être contenu et où les substituants qui entrent à chaque fois en ligne de compte sont : cyano, carboxyle, halogéno, nitro, acétyle, hydroxy, amino, SCN, tri-(C₁-C₆)alkylsilyle, (C₃-C₈)cycloalkyle, (C₃-C₈)cycloalkyl- (C₃-C₈)cycloalkyle, (C₁-C₆)alkyl-(C₃-C₈)cycloalkyle, halogéno(C₃-C₈)cycloalkyle, (C₁-C₆)alkyle, (C₁-C₆)halogénoalkyle, (C₁-C₆)cyanoalkyle, (C₁-C₆)hydroxyalkyle, hydroxycarbonyl-(C₁-C₆)-alcoxy, (C₁-C₆)alcoxycarbonyl-(C₁-C₆)alkyle, (C₁-C₆)alcoxy-(C₁-C₆)alkyle, (C₂-C₆)alcényle, (C₂-C₆)halogénoalcényle, (C₂-C₆)cyanoalcényle, (C₂-C₆)alcynyle, (C₂-C₆)halogénoalcynyle, (C₂-C₆)cyanoalcynyle, (C₁-C₆)alcoxy, (C₁-C₆)halogénoalcoxy, (C₁-C₆)cyanoalcoxy, (C₁-C₆)alcoxycarbonyl-(C₁-C₆)alcoxy, (C₁-C₆)alcoxy-(C₁-C₆)alcoxy, (C₁-C₆)alkylhydroxyimino, (C₁-C₆)alcoxyimino, (C₁-C₆)alkyl-(C₁-C₆)alcoxyimino, (C₁-C₆)halogénoalkyl-(C₁-C₆)alcoxyimino, (C₁-C₆)alkylthio, (C₁-C₆)halogénoalkylthio, (C₁-C₆)alcoxy-(C₁-C₆)alkylthio, (C₁-C₆)alkylthio-(C₁-C₆)alkyle, (C₁-C₆)alkylsulfinyle, (C₁-C₆)halogénoalkylsulfinyle, (C₁-C₆)alcoxy-(C₁-C₆)alkylsulfinyle, (C₁-C₆)alkylsulfinyl-(C₁-C₆)alkyle, (C₁-C₆)alkylsulfonyle, (C₁-C₆)halogénoalkylsulfonyle, (C₁-C₆)alcoxy-(C₁-C₆)alkylsulfonyle, (C₁-C₆)alkylsulfonyl-(C₁-C₆)alkyle, (C₁-C₆)alkylsulfonyloxy, (C₁-C₆)alkylcarbonyle, (C₁-C₆)halogénoalkylcarbonyle, (C₁-C₆)alkylcarbonyloxy, (C₁-C₆)alcoxycarbonyle, (C₁-C₆)halogénoalcoxycarbonyle, aminocarbonyle, (C₁-C₆)alkylaminocarbonyle, di-(C₁-C₆)alkylaminocarbonyle, (C₂-C₆)alcénylaminocarbonyle, di-(C₂-C₆)-alcénylaminocarbonyle, (C₃-Cs)cycloalkylaminocarbonyle, (C₁-C₆)alkylsulfonylamino, (C₁-C₆)alkylamino, di-(C₁-C₆)alkylamino, aminosulfonyle, (C₁-C₆)alkylaminosulfonyle, di-(C₁-C₆)alkylaminosulfonyle, (C₁-C₆)alkylsulfoximino, aminothiocarbonyle, (C₁-C₆)alkylaminothiocarbonyle, di-(C₁-C₆)alkylaminothiocarbonyle, (C₃-C₈)cycloalkylamino, (C₁-C₆)alkylcarbonylamino,
R⁵ représente hydrogène, cyano, halogéno, nitro, acétyle, hydroxy, amino, SCN, tri-(C₁-C₆)alkylsilyle, (C₃-C₈)cycloalkyle, (C₃-C₈)cycloalkyl-(C₃-C₈)cycloalkyle, (C₁-C₆)alkyl-(C₃-C₈)cycloalkyle, halogéno(C₃-C₈)cycloalkyle, (C₁-C₆)alkyle, (C₁-C₆)halogénoalkyle, (C₁-C₆)cyanoalkyle, (C₁-C₆)hydroxyalkyle, hydroxycarbonyl-(C₁-C₆)-alcoxy, (C₁-C₆)alcoxycarbonyl-(C₁-C₆)alkyle, (C₁-C₆)alcoxy-(C₁-C₆)alkyle, (C₂-C₆)alcényle, (C₂-C₆)halogénoalcényle, (C₂-C₆)cyanoalcényle, (C₂-C₆)alcynyle, (C₂-C₆)halogénoalcynyle, (C₂-C₆)cyanoalcynyle, (C₁-C₆)alcoxy, (C₁-C₆)halogénoalcoxy, (C₁-C₆)cyanoalcoxy, (C₁-C₆)alcoxycarbonyl-(C₁-C₆)alcoxy, (C₁-C₆)alcoxy-(C₁-C₆)alcoxy, (C₁-C₆)alkylhydroxyimino, (C₁-C₆)alcoxyimino, (C₁-C₆)alkyl-(C₁-C₆)alcoxyimino, (C₁-C₆)halogénoalkyl-(C₁-C₆)alcoxyimino, (C₁-C₆)alkylthio, (C₁-C₆)halogénoalkylthio, (C₁-C₆)alcoxy-(C₁-C₆)alkylthio, (C₁-C₆)alkylthio-(C₁-C₆)alkyle, (C₁-C₆)alkylsulfinyle, (C₁-C₆)halogénoalkylsulfinyle, (C₁-C₆)alcoxy-(C₁-C₆)alkylsulfinyle, (C₁-C₆)alkylsulfinyl-(C₁-C₆)alkyle, (C₁-C₆)alkylsulfonyle, (C₁-C₆)halogénoalkylsulfonyle, (C₁-C₆)alcoxy-(C₁-C₆)alkylsulfonyle, (C₁-C₆)alkylsulfonyl-(C₁-C₆)alkyle, (C₁-C₆)alkylsulfonyloxy, (C₁-C₆)alkylcarbonyle, (C₁-C₆)alkylthiocarbonyle, (C₁-C₆)halogénoalkylcarbonyle, (C₁-C₆)alkylcarbonyloxy, (C₁-C₆)alcoxycarbonyle, (C₁-C₆)halogénoalcoxycarbonyle, aminocarbonyle, (C₁-C₆)alkylaminocarbonyle, (C₁-C₆)alkylaminothiocarbonyle, di-(C₁-C₆)alkylaminocarbonyle, di-(C₁-C₆)alkylaminothiocarbonyle, (C₂-C₆)alcénylaminocarbonyle, di-(C₂-C₆)-alcénylaminocarbonyle, (C₃-Cs)cycloalkylaminocarbonyle, (C₁-C₆)alkylsulfonylamino, (C₁-C₆)alkylamino, di-(C₁-C₆)alkylamino, aminosulfonyle, (C₁-C₆)alkylaminosulfonyle, di-(C₁-C₆)alkylaminosulfonyle, (C₁-C₆)alkylsulfoximino, aminothiocarbonyle, (C₁-C₆)alkylaminothiocarbonyle, di-(C₁-C₆)alkylaminothiocarbonyle, (C₃-C₈)cycloalkylamino ou -NHCO-(C₁-C₆)alkyl((C₁-C₆)alkylcarbonylamino) et
R⁶ représente (C₁-C₆)alkyle, (C₁-C₆)halogénoalkyle, (C₁-C₆)cyanoalkyle, (C₁-C₆)hydroxyalkyle, (C₁-C₆)alcoxy-(C₁-C₆)alkyle, (C₁-C₆)halogénoalcoxy-(C₁-C₆)alkyle, (C₂-C₆)alcényle, (C₂-C₆)alcényloxy-(C₁-C₆)alkyle, (C₂-C₆)halogénoalcényloxy-(C₁-C₆)alkyle, (C₂-C₆)halogénoalcényle, (C₂-C₆)cyanoalcényle, (C₂-C₆)alcynyle, (C₂-C₆)alcynyloxy- (C₁-C₆)alkyle, (C₂-C₆)halogénoalcynyloxy-(C₁-C₆)alkyle, (C₂-C₆)halogénoalcynyle, (C₂-C₆)cyanoalcynyle, (C₃-C₈)cycloalkyle, (C₃-C₈)cycloalkyl-(C₃-C₈)cycloalkyle, (C₁-C₆)alkyl-(C₃-C₈)cycloalkyle, halogéno(C₃-C₈)cycloalkyle, (C₁-C₆)alkylthio-(C₁-C₆)alkyle, (C₁-C₆)halogénoalkylthio-(C₁-C₆)alkyle, (C₁-C₆)alkylsulfinyl-(C₁-C₆)alkyle, (C₁-C₆)halogénoalkylsulfinyl-(C₁-C₆)alkyle, (C₁-C₆)alkylsulfonyl-(C₁-C₆)alkyle, (C₁-C₆)halogénoalkylsulfonyl-(C₁-C₆)alkyle, (C₁-C₆)alcoxy-(C₁-C₆)alkylthio-(C₁-C₆)alkyle, (C₁-C₆)alcoxy-(C₁-C₆)alkylsulfinyl-(C₁-C₆)alkyle, (C₁-C₆)alcoxy-(C₁-C₆)alkylsulfonyl-(C₁-C₆)alkyle, (C₁-C₆)alkylcarbonyl-(C₁-C₆)alkyle, (C₁-C₆)halogénoalkylcarbonyl-(C₁-C₆)alkyle, (C₁-C₆)alcoxycarbonyl-(C₁-C₆)alkyle, (C₁-C₆)halogénoalcoxycarbonyl-(C₁-C₆)alkyle, aminocarbonyl-(C₁-C₆)alkyle, (C₁-C₆)alkylamino-(C₁-C₆)alkyle, di-(C₁-C₆)alkylamino-(C₁-C₆)alkyle ou (C₃-C₈)cycloalkylamino-(C₁-C₆)alkyle,
**caractérisé en ce que**, dans une première étape de procédé a), un composé de formule (I) 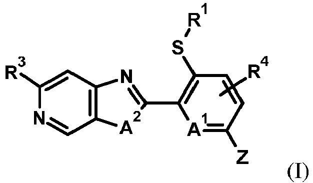 dans laquelle Z, R¹, R³, R⁴, A¹ et A² présentent à chaque fois les significations susmentionnées,
est transformé avec une base métallo-organique de zinc de structure (NR^{a}R^{b})-Zn-R^{c} ou (NR^{a}R^{b})₂-Zn, dans lesquelles
R^{c} représente halogéno ou -O-pivaloyle et
R^{a} et R^{b} représentent, conjointement, un groupe -(CH₂)₄-, -(CH₂)₅- ou -(CH₂)₂O(CH₂)₂-, chacun de ces groupes pouvant éventuellement être substitué par 1, 2, 3 ou 4 radicaux R^{d} et R^{d} étant choisi dans le groupe constitué par méthyle, éthyle, n-propyle et i-propyle,
en un composé de formule (IVa) ou un composé de formule (IVb), 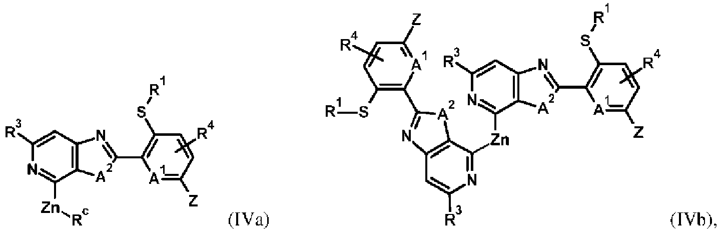 dans lesquelles Z, R¹, R³, R⁴, R^{c}, A¹ et A² présentent à chaque fois les significations susmentionnées,
et ce composé de formule (IVa) ou (IVb) est transformé, dans une deuxième étape de procédé b), avec un composé de structure Q-V, dans laquelle V représente halogéno et Q présente la signification susmentionnée, en composé de formule (II).

2. Procédé selon la revendication 1, **caractérisé en ce que**
Q représente chlore, iode ou brome,
Z représente brome, fluor ou chlore,
R^{c} représente -O-pivaloyle, chlore, brome ou iode,
A¹ représente azote, =N⁺-O⁻ ou =C-R⁵,
A² représente -N-R⁶ ou oxygène,
R¹ représente (C₁-C₄)alkyle, (C₁-C₄)hydroxyalkyle, (C₁-C₄)halogénoalkyle, (C₁-C₄)cyanoalkyle, (C₁-C₄)alcoxy-(C₁-C₄)alkyle, (C₁-C₄)halogénoalcoxy-(C₁-C₄)alkyle, (C₂-C₄)alcényle, (C₂-C₄)alcényloxy-(C₁-C₄)alkyle, (C₂-C₄)halogénoalcényloxy-(C₁-C₄)alkyle, (C₂-C₄)halogénoalcényle, (C₂-C₄)cyanoalcényle, (C₂-C₄)alcynyle, (C₂-C₄)alcynyloxy-(C₁-C₄)alkyle, (C₂-C₄)halogénoalcynyloxy-(C₁-C₄)alkyle, (C₂-C₄)halogénoalcynyle, (C₂-C₄)cyanoalcynyle, (C₃-C₆)cycloalkyle, (C₃-C₆)halogénocycloalkyle, (C₃-C₆)cyanocycloalkyle, (C₃-C₆)cycloalkyl(C₃-C₆)cycloalkyle, (C₁-C₄)alkyl-(C₃-C₆)cycloalkyle, halogéno(C₃-C₆)cycloalkyle, (C₁-C₄)alkylamino, (C₃-C₆)cycloalkylamino, (C₁-C₄)alkylcarbonylamino, (C₁-C₄)alkylthio-(C₁-C₄)alkyle, (C₁-C₄)halogénoalkylthio-(C₁-C₄)alkyle, (C₁-C₄)alkylsulfinyl-(C₁-C₄)alkyle, (C₁-C₄)halogénoalkylsulfinyl-(C₁-C₄)alkyle, (C₁-C₄)alkylsulfonyl-(C₁-C₄)alkyle, (C₁-C₄)alkylcarbonyl-(C₁-C₄)alkyle, (C₁-C₄)halogénoalkylcarbonyl-(C₁-C₄)alkyle, (C₁-C₄)alkylsulfonylamino,
ou représente à chaque fois (C₁-C₄)alkyle, (C₁-C₄)alcoxy, (C₂-C₄)alcényle, (C₂-C₄)alcynyle ou (C₃-C₆)cycloalkyle le cas échéant monosubstitué ou disubstitué, de manière identique ou différente, par aryle, hétéroaryle ou hétérocyclyle, où aryle, hétéroaryle ou hétérocyclyle peuvent à chaque fois le cas échéant être monosubstitués ou disubstitués, de manière identique ou différente, par halogéno, cyano, carbamoyle, aminosulfonyle, (C₁-C₄)alkyle, (C₃-C₄)cycloalkyle, (C₁-C₄)alcoxy, (C₁-C₄)halogénoalkyle, (C₁-C₄)halogénoalcoxy, (C₁-C₄)alkylthio, (C₁-C₄)alkylsulfinyle, (C₁-C₄)alkylsulfonyle ou (C₁-C₄)alkylsulfimino,
R³ représente hydrogène, cyano, halogéno, nitro, acétyle, hydroxy, amino, SCN, tri-(C₁-C₄)alkylsilyle, (C₃-C₆)cycloalkyle, (C₃-C₆)cycloalkyl-(C₃-C₆)cycloalkyle, (C₁-C₄)alkyl-(C₃-C₆)cycloalkyle, halogéno(C₃-C₆)cycloalkyle, cyano(C₃-C₈)cycloalkyle, (C₁-C₄)alkyle, (C₁-C₄)halogénoalkyle, (C₁-C₄)cyanoalkyle, (C₁-C₄)hydroxyalkyle, (C₁-C₄)alcoxy-(C₁-C₄)alkyle, (C₂-C₄)alcényle, (C₂-C₄)halogénoalcényle, (C₂-C₄)cyanoalcényle, (C₂-C₄)alcynyle, (C₂-C₄)halogénoalcynyle, (C₂-C₄)cyanoalcynyle, (C₁-C₄)alcoxy, (C₁-C₄)halogénoalcoxy, (C₁-C₄)cyanoalcoxy, (C₁-C₄)alcoxy-(C₁-C₄)alcoxy, (C₁-C₄)alkylhydroxyimino, (C₁-C₄)alcoxyimino, (C₁-C₄)alkyl-(C₁-C₄)alcoxyimino, (C₁-C₄)halogénoalkyl-(C₁-C₄)alcoxyimino, (C₁-C₄)alkylthio, (C₁-C₄)halogénoalkylthio, (C₁-C₄)alkylthio- (C₁-C₄)alkyle, (C₁-C₄)alkylsulfinyle, (C₁-C₄)halogénoalkylsulfinyle, (C₁-C₄)alkylsulfinyl-(C₁-C₄)alkyle, (C₁-C₄)alkylsulfonyle, (C₁-C₄)halogénoalkylsulfonyle, (C₁-C₄)alkylsulfonyl-(C₁-C₄)alkyle, (C₁-C₄)alkylsulfonyloxy, (C₁-C₄)alkylcarbonyle, (C₁-C₄)halogénoalkylcarbonyle, aminocarbonyle, aminothiocarbonyle, (C₁-C₄)alkylaminocarbonyle, di-(C₁-C₄)alkylaminocarbonyle, (C₁-C₄)alkylsulfonylamino, (C₁-C₄)alkylamino, di-(C₁-C₄)alkylamino, aminosulfonyle, (C₁-C₄)alkylaminosulfonyle, di-(C₁-C₄)alkylaminosulfonyle, aminothiocarbonyle, NHCO-(C₁-C₄)alkyl((C₁-C₄)alkylcarbonylamino) ou représente à chaque fois phényle ou hétéroaryle, le cas échéant monosubstitué ou disubstitué, de manière identique ou différente, où (dans le cas de hétéroaryle), le cas échéant au moins un groupe carbonyle peut être contenu et où les substituants qui entrent à chaque fois en ligne de compte sont : cyano, halogéno, nitro, acétyle, amino, (C₁-C₄)alkyle, (C₁-C₄)halogénoalkyle, (C₁-C₄)cyanoalkyle, (C₁-C₄)hydroxyalkyle, (C₁-C₄)alcoxy-(C₁-C₄)alkyle, (C₂-C₄)alcényle, (C₂-C₄)halogénoalcényle, (C₂-C₄)cyanoalcényle, (C₂-C₄)alcynyle, (C₂-C₄)halogénoalcynyle, (C₂-C₄)cyanoalcynyle, (C₁-C₄)alcoxy, (C₁-C₄)halogénoalcoxy, (C₁-C₄)cyanoalcoxy, (C₁-C₄)alcoxy-(C₁-C₄)alcoxy, (C₁-C₄)alkylhydroxyimino, (C₁-C₄)alcoxyimino, (C₁-C₄)alkyl-(C₁-C₄)alcoxyimino, (C₁-C₄)halogénoalkyl-(C₁-C₄)alcoxyimino, (C₁-C₄)alkylthio, (C₁-C₄)halogénoalkylthio, (C₁-C₄)alkylthio-(C₁-C₄)alkyle, (C₁-C₄)alkylsulfinyle, (C₁-C₄) halogénoalkylsulfinyle, (C₁-C₄)alkylsulfinyl-(C₁-C₄)alkyle, (C₁-C₄)alkylsulfonyle, (C₁-C₄)halogénoalkylsulfonyle, (C₁-C₄)alkylsulfonyl-(C₁-C₄)alkyle, (C₁-C₄)alkylsulfonyloxy, (C₁-C₄)alkylcarbonyle, (C₁-C₄)halogénoalkylcarbonyle, aminocarbonyle, (C₁-C₄)alkylaminocarbonyle, di-(C₁-C₄)alkylaminocarbonyle, (C₁-C₄)alkylsulfonylamino, (C₁-C₄)alkylamino, di-(C₁-C₄)alkylamino, aminosulfonyle, (C₁-C₄)alkylaminosulfonyle et di-(C₁-C₄)alkylaminosulfonyle,
R⁴ représente hydrogène, cyano, halogéno, nitro, acétyle, hydroxy, amino, SCN, tri-(C₁-C₄)alkylsilyle, (C₃-C₆)cycloalkyle, (C₃-C₆)cycloalkyl-(C₃-C₆)cycloalkyle, (C₁-C₄)alkyl-(C₃-C₆)cycloalkyle, halogéno(C₃-C₆)cycloalkyle, cyano(C₃-C₈)cycloalkyle, (C₁-C₄)alkyle, (C₁-C₄)halogénoalkyle, (C₁-C₄)cyanoalkyle, (C₁-C₄)hydroxyalkyle, (C₁-C₄)alcoxy-(C₁-C₄)alkyle, (C₂-C₄)alcényle, (C₂-C₄)halogénoalcényle, (C₂-C₄)cyanoalcényle, (C₂-C₄)alcynyle, (C₂-C₄)halogénoalcynyle, (C₂-C₄)cyanoalcynyle, (C₁-C₄)alcoxy, (C₁-C₄)halogénoalcoxy, (C₁-C₄)cyanoalcoxy, (C₁-C₄)alcoxy-(C₁-C₄)alcoxy, (C₁-C₄)alkylhydroxyimino, (C₁-C₄)alcoxyimino, (C₁-C₄)alkyl-(C₁-C₄)alcoxyimino, (C₁-C₄)halogénoalkyl-(C₁-C₄)alcoxyimino, (C₁-C₄)alkylthio, (C₁-C₄)halogénoalkylthio, (C₁-C₄)alkylthio-(C₁-C₄)alkyle, (C₁-C₄)alkylsulfinyle, (C₁-C₄)halogénoalkylsulfinyle, (C₁-C₄) alkylsulfinyl- (C₁-C₄)alkyle, (C₁-C₄)alkylsulfonyle, (C₁-C₄)halogénoalkylsulfonyle, (C₁-C₄)alkylsulfonyl-(C₁-C₄)alkyle, (C₁-C₄)alkylsulfonyloxy, (C₁-C₄)alkylcarbonyle, (C₁-C₄)halogénoalkylcarbonyle, aminocarbonyle, aminothiocarbonyle, (C₁-C₄)alkylaminocarbonyle, di-(C₁-C₄)alkylaminocarbonyle, (C₁-C₄)alkylsulfonylamino, (C₁-C₄)alkylamino, di-(C₁-C₄)alkylamino, aminosulfonyle, (C₁-C₄)alkylaminosulfonyle, di-(C₁-C₄)alkylaminosulfonyle, aminothiocarbonyle, NHCO-(C₁-C₄)alkyl((C₁-C₄)alkylcarbonylamino), représente en outre à chaque fois phényle ou hétéroaryle, le cas échéant monosubstitué ou disubstitué, de manière identique ou différente, où (dans le cas de hétéroaryle), le cas échéant au moins un groupe carbonyle peut être contenu et où les substituants qui entrent à chaque fois en ligne de compte sont : cyano, halogéno, nitro, acétyle, amino, (C₃-C₆)cycloalkyle, (C₃-C₆)cycloalkyl-(C₃-C₆) cycloalkyle, (C₁-C₄)alkyl-(C₃-C₆)cycloalkyle, halogéno(C₃-C₆)cycloalkyle, (C₁-C₄)alkyle, (C₁-C₄)halogénoalkyle, (C₁-C₄)cyanoalkyle, (C₁-C₄)hydroxyalkyle, (C₁-C₄)alcoxy-(C₁-C₄)alkyle, (C₂-C₄)alcényle, (C₂-C₄)halogénoalcényle, (C₂-C₄)cyanoalcényle, (C₂-C₄)alcynyle, (C₂-C₄)halogénoalcynyle, (C₂-C₄)cyanoalcynyle, (C₁-C₄)alcoxy, (C₁-C₄)halogénoalcoxy, (C₁-C₄)cyanoalcoxy, (C₁-C₄)alcoxy-(C₁-C₄)alcoxy, (C₁-C₄)alkylhydroxyimino, (C₁-C₄)alcoxyimino, (C₁-C₄)alkyl-(C₁-C₄)alcoxyimino, (C₁-C₄)halogénoalkyl-(C₁-C₄)alcoxyimino, (C₁-C₄)alkylthio, (C₁-C₄)halogénoalkylthio, (C₁-C₄)alkylthio-(C₁-C₄)alkyle, (C₁-C₄)alkylsulfinyle, (C₁-C₄)halogénoalkylsulfinyle, (C₁-C₄)alkylsulfinyl- (C₁-C₄)alkyle, (C₁-C₄)alkylsulfonyle, (C₁-C₄)halogénoalkylsulfonyle, (C₁-C₄)alkylsulfonyl-(C₁-C₄)alkyle, (C₁-C₄)alkylsulfonyloxy, (C₁-C₄)alkylcarbonyle, (C₁-C₄)halogénoalkylcarbonyle, aminocarbonyle, (C₁-C₄)alkylaminocarbonyle, di-(C₁-C₄)alkylaminocarbonyle, (C₁-C₄)alkylsulfonylamino, (C₁-C₄)alkylamino, di-(C₁-C₄)alkylamino, aminosulfonyle, (C₁-C₄)alkylaminosulfonyle, di-(C₁-C₄)alkylaminosulfonyle, NHCO-(C₁-C₄)alkyl((C₁-C₄)alkylcarbonylamino),
R⁵ représente hydrogène, cyano, halogéno, nitro, acétyle, hydroxy, amino, SCN, tri-(C₁-C₄)alkylsilyle, (C₃-C₆)cycloalkyle, (C₃-C₆)cycloalkyl-(C₃-C₆)cycloalkyle, (C₁-C₄)alkyl-(C₃-C₆)cycloalkyle, halogéno(C₃-C₆)cycloalkyle, (C₁-C₄)alkyle, (C₁-C₄)halogénoalkyle, (C₁-C₄)cyanoalkyle, (C₁-C₄)hydroxyalkyle, (C₁-C₄)alcoxy-(C₁-C₄)alkyle, (C₂-C₄)alcényle, (C₂-C₄)halogénoalcényle, (C₂-C₄)cyanoalcényle, (C₂-C₄)alcynyle, (C₂-C₄)halogénoalcynyle, (C₂-C₄)cyanoalcynyle, (C₁-C₄)alcoxy, (C₁-C₄)halogénoalcoxy, (C₁-C₄)cyanoalcoxy, (C₁-C₄)alcoxy-(C₁-C₄)alcoxy, (C₁-C₄)alkylhydroxyimino, (C₁-C₄)alcoxyimino, (C₁-C₄)alkyl-(C₁-C₄)alcoxyimino, (C₁-C₄)halogénoalkyl-(C₁-C₄)alcoxyimino, (C₁-C₄)alkylthio, (C₁-C₄)halogénoalkylthio, (C₁-C₄)alkylthio-(C₁-C₄)alkyle, (C₁-C₄)alkylsulfinyle, (C₁-C₄)halogénoalkylsulfinyle, (C₁-C₄)alkylsulfinyl-(C₁-C₄)alkyle, (C₁-C₄)alkylsulfonyle, (C₁-C₄)halogénoalkylsulfonyle, (C₁-C₄)alkylsulfonyl-(C₁-C₄)alkyle, (C₁-C₄)alkylsulfonyloxy, (C₁-C₄)alkylcarbonyle, (C₁-C₄)halogénoalkylcarbonyle, aminocarbonyle, aminothiocarbonyle, (C₁-C₄)alkylaminocarbonyle, di-(C₁-C₄)alkylaminocarbonyle, (C₁-C₄)alkylsulfonylamino, (C₁-C₄)alkylamino, di-(C₁-C₄)alkylamino, aminosulfonyle, (C₁-C₄)alkylaminosulfonyle, di-(C₁-C₄)alkylaminosulfonyle, aminothiocarbonyle ou NHCO-(C₁-C₄)alkyl((C₁-C₄)alkylcarbonylamino) et
R⁶ représente (C₁-C₄)alkyle, (C₁-C₄)halogénoalkyle, (C₁-C₄)cyanoalkyle, (C₁-C₄)hydroxyalkyle, (C₁-C₄)alcoxy-(C₁-C₄)alkyle, (C₁-C₄)halogénoalcoxy- (C₁-C₄)alkyle, (C₂-C₄)alcényle, (C₂-C₄)alcényloxy-(C₁-C₄)alkyle, (C₂-C₄)halogénoalcényloxy-(C₁-C₄)alkyle, (C₂-C₄)halogénoalcényle, (C₂-C₄)cyanoalcényle, (C₂-C₄)alcynyle, (C₂-C₄)alcynyloxy-(C₁-C₄)alkyle, (C₂-C₄)halogénoalcynyle, (C₃-C₆)cycloalkyle, (C₃-C₆)cycloalkyl-(C₃-C₆)cycloalkyle, (C₁-C₄)alkyl-(C₃-C₆)cycloalkyle, halogéno(C₃-C₆)cycloalkyle, (C₁-C₄)alkylthio-(C₁-C₄)alkyle, (C₁-C₄)halogénoalkylthio-(C₁-C₄)alkyle, (C₁-C₄)alkylsulfinyl-(C₁-C₄)alkyle, (C₁-C₄)halogénoalkylsulfinyl-(C₁-C₄)alkyle, (C₁-C₄)alkylsulfonyl-(C₁-C₄)alkyle, (C₁-C₄)halogénoalkylsulfonyl-(C₁-C₄)alkyle, (C₁-C₄)alcoxy-(C₁-C₄)alkylthio-(C₁-C₄)alkyle ou (C₁-C₄)alkylcarbonyl-(C₁-C₄)alkyle.

3. Procédé selon la revendication 1, **caractérisé en ce que**
Q représente chlore, iode ou brome,
Z représente brome, fluor ou chlore,
R^{c} représente -O-pivaloyle, chlore, brome ou iode,
A¹ représente azote ou =C-R⁵,
A² représente -N-R⁶,
R¹ représente (C₁-C₄)alkyle, (C₁-C₄)hydroxyalkyle, (C₁-C₄)halogénoalkyle, (C₂-C₄) alcényle, (C₂-C₄)halogénoalcényle, (C₂-C₄) alcynyle, (C₂-C₄)halogénoalcynyle, (C₃-C₆)cycloalkyle, (C₁-C₄)alkylthio-(C₁-C₄)alkyle, (C₁-C₄)alkylsulfinyl-(C₁-C₄) alkyle ou (C₁-C₄)alkylsulfonyl- (C₁-C₄)alkyle,
R³ représente hydrogène, halogéno, (C₁-C₄)alkyle, (C₁-C₄)halogénoalkyle, (C₁-C₄)halogénoalcoxy, (C₁-C₄)alkylthio, (C₁-C₄)alkylsulfinyle, (C₁-C₄)alkylsulfonyle, (C₁-C₄)halogénoalkylthio, (C₁-C₄)halogénoalkylsulfinyle ou (C₁-C₄)halogénoalkylsulfonyle,
R⁴ représente hydrogène, cyano, halogéno, nitro, hydroxy, amino, SCN, tri-(C₁-C₄)alkylsilyle, (C₃-C₆)cycloalkyle, cyano(C₃-C₈)cycloalkyle, (C₃-C₆)cycloalkyl- (C₃-C₆)cycloalkyle, (C₁-C₄)alkyl- (C₃-C₆)cycloalkyle, halogéno (C₃-C₆)cycloalkyle, (C₁-C₄)alkyle, (C₁-C₄)halogénoalkyle, (C₁-C₄)cyanoalkyle, (C₁-C₄)alcoxy- (C₁-C₄)alkyle, (C₂-C₄)alcényle, (C₂-C₄) halogénoalcényle, (C₂-C₄)cyanoalcényle, (C₂-C₄) alcynyle, (C₂-C₄)halogénoalcynyle, (C₂-C₄) cyanoalcynyle, (C₁-C₄)alcoxy, (C₁-C₄)halogénoalcoxy, (C₁-C₄)cyanoalcoxy, (C₁-C₄)alkylhydroxyimino, (C₁-C₄)alcoxyimino, (C₁-C₄)alkyl- (C₁-C₄)alcoxyimino, (C₁-C₄)alkylthio, (C₁-C₄)halogénoalkylthio, (C₁-C₄)alkylsulfinyle, (C₁-C₄)halogénoalkylsulfinyle, (Cₗ-C₄) alkylsulfonyle, (C₁-C₄)halogénoalkylsulfonyle, (C₁-C₄)alkylsulfonyloxy, (Cₗ-C₄) alkylcarbonyle, (C₁-C₄)halogénoalkylcarbonyle, aminocarbonyle, (C₁-C₄)alkylaminocarbonyle, di-(C₁-C₄)alkylaminocarbonyle, (C₁-C₄)alkylsulfonylamino, (C₁-C₄)alkylamino, di-(C₁-C₄)alkylamino, aminosulfonyle, (C₁-C₄)alkylaminosulfonyle, di-(C₁-C₄)alkylaminosulfonyle ou NHCO- (C₁-C₄)alkyl((C₁-C₄)alkylcarbonylamino), représente en outre à chaque fois phényle ou hétéroaryle, le cas échéant monosubstitué ou disubstitué, de manière identique ou différente, où (dans le cas de hétéroaryle), le cas échéant au moins un groupe carbonyle peut être contenu et où les substituants qui entrent à chaque fois en ligne de compte sont : cyano, halogéno, (C₃-C₆)cycloalkyle, (C₃-C₆)cycloalkyl- (C₃-C₆)cycloalkyle, (C₁-C₄)alkyl-(C₃-C₆)cycloalkyle, halogéno (C₃-C₆)cycloalkyle, (C₁-C₄)alkyle, (C₁-C₄)halogénoalkyle, (C₁-C₄)cyanoalkyle, (C₂-C₄)alcényle, (C₂-C₄) halogénoalcényle, (C₂-C₄)cyanoalcényle, (C₂-C₄)alcynyle, (C₂-C₄)halogénoalcynyle, (C₂-C₄) cyanoalcynyle, (C₁-C₄)alcoxy, (C₁-C₄)halogénoalcoxy, (C₁-C₄)alkylhydroxyimino, (C₁-C₄)alcoxyimino, (C₁-C₄)alkyl- (C₁-C₄)alcoxyimino, (C₁-C₄)alkylthio, (C₁-C₄)halogénoalkylthio, (C₁-C₄)alkylsulfinyle, (C₁-C₄)halogénoalkylsulfinyle, (C₁-C₄)alkylsulfonyle, (C₁-C₄)halogénoalkylsulfonyle, (C₁-C₄)alkylsulfonyloxy, (C₁-C₄)alkylcarbonyle, (C₁-C₄)halogénoalkylcarbonyle, aminocarbonyle, (C₁-C₄)alkylaminocarbonyle, di-(C₁-C₄)alkylaminocarbonyle, (C₁-C₄)alkylsulfonylamino, (C₁-C₄)alkylamino, di-(C₁-C₄)alkylamino, aminosulfonyle, (C₁-C₄)alkylaminosulfonyle, di-(C₁-C₄)alkylaminosulfonyle, NHCO- (C₁-C₄)alkyl((C₁-C₄)alkylcarbonylamino),
R⁵ représente hydrogène, halogéno, cyano ou (C₁-C₄)alkyle et
R⁶ représente (C₁-C₄)alkyle ou (C₁-C₄)alcoxy- (C₁-C₄)alkyle.

4. Procédé selon la revendication 1, **caractérisé en ce que**
Q représente iode ou brome,
Z représente brome ou chlore,
R^{c} représente chlore, brome ou iode,
A¹ représente azote ou =C-R⁵,
A² représente -N-R⁶,
R¹ représente méthyle, éthyle, n-propyle, i-propyle, cyclopropyle, n-butyle, i-butyle, tert-butyle, cyclobutyle, fluorométhyle, difluorométhyle, trifluorométhyle, fluoroéthyle, difluoroéthyle, trifluoroéthyle, tétrafluoroéthyle ou pentafluoroéthyle,
R³ représente fluor, chlore, fluorométhyle, difluorométhyle, trifluorométhyle, fluoroéthyle, difluoroéthyle, trifluoroéthyle, tétrafluoroéthyle, pentafluoroéthyle, trifluorométhoxy, difluorochlorométhoxy, dichlorofluorométhoxy, trifluorométhylthio, trifluorométhylsulfonyle ou trifluorométhylsulfinyle,
R⁴ représente hydrogène, cyano, halogéno, (C₁-C₄)alkyle, (C₁-C₄)halogénoalkyle, (C₁-C₄)halogénoalcoxy, (C₁-C₄)alkylthio, (C₁-C₄)alkylsulfinyle, (C₁-C₄)alkylsulfonyle, (C₁-C₄)halogénoalkylthio, (C₁-C₄)halogénoalkylsulfinyle, (C₁-C₄)halogénoalkylsulfonyle ou NHCO- (C₁-C₄)alkyl((C₁-C₄)alkylcarbonylamino),
R⁵ représente hydrogène, fluor, chlore, brome ou cyano et
R⁶ représente méthyle, éthyle, i-propyle, méthoxyméthyle ou méthoxyéthyle.

5. Procédé selon la revendication 1, **caractérisé en ce que**
Q représente iode ou brome,
Z représente brome ou chlore,
R^{c} représente chlore ou brome,
A¹ représente azote,
A² représente -N-R⁶,
R¹ représente méthyle, éthyle, n-propyle, i-propyle ou cyclopropyle,
R³ représente trifluorométhyle ou pentafluoroéthyle,
R⁴ représente hydrogène et
R⁶ représente méthyle.

6. Procédé selon la revendication 1, **caractérisé en ce que**
Q représente iode ou brome,
Z représente brome ou chlore,
R^{c} représente chlore,
A¹ représente azote,
A² représente -N-R⁶,
R¹ représente éthyle,
R³ représente trifluorométhyle,
R⁴ représente hydrogène et
R⁶ représente méthyle.

7. Procédé selon l'une quelconque des revendications 1 à 6, **caractérisé en ce qu'**il s'agit, pour la base métallo-organique de zinc, d'un composé de formule (VI)
(VI) (TMP) ₓZnCl₂₋ₓ,
dans laquelle x représente le nombre 1 ou 2.

8. Procédé selon l'une quelconque des revendications 1 à 7, **caractérisé en ce que** la base métallo-organique de zinc se trouve en association avec un halogénure de métal alcalin ou alcalino-terreux, de préférence le chlorure de lithium et/ou le chlorure de magnésium.

9. Procédé selon l'une quelconque des revendications 1 à 8, **caractérisé en ce qu'**il s'agit, pour le composé Q-V, d'un halogène élémentaire, en particulier de F₂, Cl₂, Br₂ ou I₂.

10. Procédé selon l'une quelconque des revendications 1 à 9, **caractérisé en ce que** le solvant est le THF ou le N,N-diméthylformamide (DMF).

11. Procédé selon l'une quelconque des revendications 1 à 10, **caractérisé en ce que** l'étape de procédé a) est réalisée à une température entre 0°C et 80°C.

12. Procédé selon l'une quelconque des revendications 1 à 11, **caractérisé en ce que** l'étape de procédé b) est réalisée à une température entre 0°C et 80°C.

13. Procédé selon l'une quelconque des revendications 1 à 12, **caractérisé en ce que** les composés de formule (II) sont transformés davantage dans une troisième étape c) en composés de formule (III) 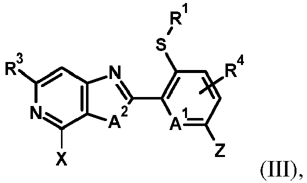 dans laquelle Z, R¹, R³, R⁴, A¹ et A² présentent les significations selon l'une quelconque des revendications 1 à 6 et
X représente cyano, carboxyle, halogéno, nitro, acétyle, hydroxy, amino, SCN, tri-(C₁-C₆)alkylsilyle, (C₁-C₆)alkyle, (C₁-C₆)halogénoalkyle, (C₁-C₆)cyanoalkyle, (C₁-C₆)hydroxyalkyle, hydroxycarbonyl-(C₁-C₆)-alcoxy, (C₁-C₆)alcoxycarbonyl-(C₁-C₆)alkyle, (C₁-C₆)alcoxy-(C₁-C₆)alkyle, (C₂-C₆)alcényle, (C₂-C₆)halogénoalcényle, (C₂-C₆)cyanoalcényle, (C₂-C₆)alcynyle, (C₂-C₆)halogénoalcynyle, (C₂-C₆)cyanoalcynyle, (C₃-C₆)cycloalkyle, (C₃-C₆)halogénocycloalkyle, (C₃-C₆)cyanocycloalkyle, (C₃-C₆)cycloalkyl-(C₃-C₆)cycloalkyle, (C₁-C₄)alkyl-(C₃-C₆)cycloalkyle, (C₁-C₆)alcoxy, (C₁-C₆)halogénoalcoxy, (C₁-C₆)cyanoalcoxy, (C₁-C₆)alcoxycarbonyl-(C₁-C₆)alcoxy, (C₁-C₆)alcoxy- (C₁-C₆)alcoxy, (C₁-C₆)alcoxyimino, -N=C (H)-O(C₁-C₆)alkyle, -C(H)=N-O(C₁-C₆)alkyle, (C₁-C₆)halogénoalkyl-(C₁-C₆)alcoxyimino, (C₁-C₆)alkylthio, (C₁-C₆)halogénoalkylthio, (C₁-C₆)alcoxy-(C₁-C₆)alkylthio, (C₁-C₆)alkylthio-(C₁-C₆)alkyle, (C₁-C₆) alkylsulfinyle, (C₁-C₆)halogénoalkylsulfinyle, (C₁-C₆)alcoxy-(C₁-C₆)alkylsulfinyle, (C₁-C₆)alkylsulfinyl-(C₁-C₆)alkyle, (C₁-C₆) alkylsulfonyle, (C₁-C₆)halogénoalkylsulfonyle, (C₁-C₆)alcoxy-(C₁-C₆)alkylsulfonyle, (C₁-C₆)alkylsulfonyl-(C₁-C₆)alkyle, (C₁-C₆)alkylsulfonyloxy, (C₁-C₆)alkylcarbonyle, (C₁-C₆)halogénoalkylcarbonyle, (C₁-C₆)alkylcarbonyloxy, (C₁-C₆)alcoxycarbonyle, (C₁-C₆)halogénoalcoxycarbonyle, aminocarbonyle, (C₁-C₆)alkylaminocarbonyle, di-(C₁-C₆)alkylaminocarbonyle, (C₂-C₆)alcénylaminocarbonyle, di-(C₂-C₆)-alcénylaminocarbonyle, (C₃-Cs)cycloalkylaminocarbonyle, (C₁-C₆)alkylsulfonylamino, (C₁-C₆)alkylamino, di-(C₁-C₆)alkylamino, aminosulfonyle, (C₁-C₆)alkylaminosulfonyle, di-(C₁-C₆)alkylaminosulfonyle, (C₁-C₆)alkylsulfoximino, aminothiocarbonyle, (C₁-C₆)alkylaminothiocarbonyle, di-(C₁-C₆)alkylaminothiocarbonyle, (C₃-C₈)cycloalkylamino, (C₁-C₆) alkylcarbamoyle (qui comprend -NHCOO(C₁-C₆)alkyle, -N(C₁-C₆)alkylCOO(C₁-C₆)alkyle, -OCONH(C₁-C₆)alkyle ou -OCON(C₁-C₆)dialkyle), (C₁-C₆)alkylcarbonylamino((C₁-C₆)alkylCONH), (C₁-C₆) alkylurée (qui comprend -NHCONH(C₁-C₆)alkyle et -NHCON(C₁-C₆)dialkyle) ou un cycle, le cas échéant monosubstitué ou polysubstitué, de manière identique ou différente, saturé, partiellement saturé ou hétéroaromatique, dans lequel au moins un atome de C est remplacé par un hétéroatome, ou un cycle, monosubstitué ou polysubstitué, de manière identique ou différente, aromatique, où à chaque fois, le cas échéant, au moins un groupe carbonyle peut être contenu et où les substituants qui entrent en ligne de compte sont : cyano, carboxyle, halogéno, nitro, acétyle, hydroxy, amino, SCN, tri-(C₁-C₆)alkylsilyle, (C₁-C₆)alkyle, (C₁-C₆) halogénoalkyle, (C₁-C₆)cyanoalkyle, (C₁-C₆)hydroxyalkyle, hydroxycarbonyl-(C₁-C₆)-alcoxy, (C₁-C₆)alcoxycarbonyl-(C₁-C₆)alkyle, (C₁-C₆)alcoxy- (C₁-C₆)alkyle, (C₂-C₆)alcényle, (C₂-C₆)halogénoalcényle, (C₂-C₆)cyanoalcényle, (C₂-C₆)alcynyle, (C₂-C₆)halogénoalcynyle, (C₂-C₆)cyanoalcynyle, (C₃-C₆)cycloalkyle, (C₃-C₆)halogénocycloalkyle, (C₃-C₆)cyanocycloalkyle, (C₃-C₆)cycloalkyl-(C₃-C₆)cycloalkyle, (C₁-C₄)alkyl-(C₃-C₆)cycloalkyle, (C₁-C₆)alcoxy, (C₁-C₆)halogénoalcoxy, (C₁-C₆)cyanoalcoxy, (C₁-C₆)alcoxycarbonyl-(C₁-C₆)alcoxy, (C₁-C₆)alcoxy-(C₁-C₆) alcoxy, (C₁-C₆)alcoxyimino, -N=C(H)-O(C₁-C₆)alkyle, -C(H)=N-O(C₁-C₆) alkyle, (C₁-C₆)halogénoalkyl-(C₁-C₆)alcoxyimino, (C₁-C₆)alkylthio, (C₁-C₆)halogénoalkylthio, (C₁-C₆) alcoxy- (C₁-C₆)alkylthio, (C₁-C₆)alkylthio- (C₁-C₆) alkyle, (C₁-C₆)alkylsulfinyle, (C₁-C₆)halogénoalkylsulfinyle, (C₁-C₆)alcoxy-(C₁-C₆)alkylsulfinyle, (C₁-C₆)alkylsulfinyl-(C₁-C₆) alkyle, (C₁-C₆)alkylsulfonyle, (C₁-C₆)halogénoalkylsulfonyle, (C₁-C₆)alcoxy- (C₁-C₆)alkylsulfonyle, (C₁-C₆)alkylsulfonyl- (C₁-C₆) alkyle, (C₁-C₆)alkylsulfonyloxy, (C₁-C₆)alkylcarbonyle, (C₁-C₆)halogénoalkylcarbonyle, (C₁-C₆)alkylcarbonyloxy, (C₁-C₆)alcoxycarbonyle, (C₁-C₆)halogénoalcoxycarbonyle, aminocarbonyle, (C₁-C₆)alkylaminocarbonyle, di-(C₁-C₆)alkylaminocarbonyle, (C₂-C₆)alcénylaminocarbonyle, di-(C₂-C₆)-alcénylaminocarbonyle, (C₃-Cs)cycloalkylaminocarbonyle, (C₁-C₆)alkylsulfonylamino, (C₁-C₆)alkylamino, di-(C₁-C₆)alkylamino, aminosulfonyle, (C₁-C₆)alkylaminosulfonyle, di-(C₁-C₆)alkylaminosulfonyle, (C₁-C₆)alkylsulfoximino, aminothiocarbonyle, (C₁-C₆)alkylaminothiocarbonyle, di-(C₁-C₆)alkylaminothiocarbonyle, (C₃-C₈)cycloalkylamino ou (C₁-C₆)alkylcarbonylamino.

14. Procédé selon la revendication 13, **caractérisé en ce que**
X représente cyano, halogéno, nitro, acétyle, amino, (C₁-C₄)alkyle, (C₁-C₄)halogénoalkyle, (C₁-C₄)cyanoalkyle, (C₁-C₄)hydroxyalkyle, (C₁-C₄)alcoxy-(C₁-C₄)alkyle, (C₂-C₄)alcényle, (C₂-C₄)halogénoalcényle, (C₂-C₄)cyanoalcényle, (C₂-C₄)alcynyle, (C₂-C₄)halogénoalcynyle, (C₂-C₄)cyanoalcynyle, (C₃-C₆)cycloalkyle, (C₃-C₆)halogénocycloalkyle, (C₃-C₆)cyanocycloalkyle, (C₃-C₆)cycloalkyl- (C₃-C₆)cycloalkyle, (C₁-C₄)alkyl-(C₃-C₆)cycloalkyle, (C₁-C₄)alcoxy, (C₁-C₄)halogénoalcoxy, (C₁-C₄)cyanoalcoxy, (C₁-C₄)alcoxy-(C₁-C₄)alcoxy, (C₁-C₄)alcoxyimino, -N=C (H)-O(C₁-C₄)alkyle, -C(H)=N-O(C₁-C₄)alkyle, (C₁-C₄)halogénoalkyl- (C₁-C₄)alcoxyimino, (C₁-C₄)alkylthio, (C₁-C₄)halogénoalkylthio, (C₁-C₄)alkylthio-(C₁-C₄)alkyle, (C₁-C₄)alkylsulfinyle, (C₁-C₄)halogénoalkylsulfinyle, (C₁-C₄)alkylsulfinyl-(C₁-C₄)alkyle, (C₁-C₄)alkylsulfonyle, (C₁-C₄)halogénoalkylsulfonyle, (C₁-C₄)alkylsulfonyl-(C₁-C₄)alkyle, (C₁-C₄)alkylsulfonyloxy, (C₁-C₄)alkylcarbonyle, (C₁-C₄)halogénoalkylcarbonyle, aminocarbonyle, (C₁-C₄)alkylaminocarbonyle, di-(C₁-C₄)alkylaminocarbonyle, (C₁-C₄)alkylsulfonylamino, (C₁-C₄)alkylamino, di-(C₁-C₄)alkylamino, aminosulfonyle, (C₁-C₄)alkylaminosulfonyle, di-(C₁-C₄)alkylaminosulfonyle, (C₁-C₄)alkylcarbamoyle (qui comprend -NHCOO(C₁-C₄)alkyle, -N(C₁-C₄)alkylCOO(C₁-C₄)alkyle, -OCONH(C₁-C₄)alkyle ou -OCON(C₁-C₄)dialkyle), (C₁-C₄)alkylcarbonylamino ((C₁-C₄)alkylCONH) ou (C₁-C₄)alkylurée (qui comprend -NHCONH(C₁-C₆)alkyle et -NHCON(C₁-C₆)dialkyle).

15. Procédé selon la revendication 13, **caractérisé en ce que**
X représente cyano, halogéno, nitro, acétyle, amino, (C₁-C₄) alkyle, (C₁-C₄)halogénoalkyle, (C₁-C₄)cyanoalkyle, (C₁-C₄)hydroxyalkyle, (C₁-C₄)alcoxy-(C₁-C₄) alkyle, (C₂-C₄) alcényle, (C₂-C₄)halogénoalcényle, (C₂-C₄) cyanoalcényle, (C₂-C₄)alcynyle, (C₂-C₄) halogénoalcynyle, (C₂-C₄)cyanoalcynyle, (C₃-C₆)cycloalkyle, (C₃-C₆)halogénocycloalkyle, (C₃-C₆)cyanocycloalkyle, (C₃-C₆)cycloalkyl- (C₃-C₆)cycloalkyle, (C₁-C₄)alkyl-(C₃-C₆)cycloalkyle, (C₁-C₄)alcoxy, (C₁-C₄)halogénoalcoxy, (C₁-C₄)cyanoalcoxy ou (C₁-C₄)alcoxy- (C₁-C₄)alcoxy.

16. Procédé selon la revendication 13, **caractérisé en ce que**
X représente cyano, halogéno, amino, (C₁-C₄)alkyle, (C₁-C₄) halogénoalkyle, (C₁-C₄)cyanoalkyle, (C₁-C₄)hydroxyalkyle, (C₁-C₄)alcoxy-(C₁-C₄)alkyle, (C₂-C₄)alcényle, (C₂-C₄)halogénoalcényle, (C₂-C₄)cyanoalcényle, (C₂-C₄)alcynyle, (C₂-C₄)halogénoalcynyle, (C₂-C₄)cyanoalcynyle, (C₃-C₆)cycloalkyle, (C₃-C₆)halogénocycloalkyle, (C₃-C₆)cyanocycloalkyle, (C₃-C₆)cycloalkyl-(C₃-C₆)cycloalkyle, (C₁-C₄)alcoxy ou (C₁-C₄)cyanoalcoxy.

17. Procédé selon l'une quelconque des revendications 13 à 16, **caractérisé en ce que** la transformation dans l'étape de procédé c) est réalisée à une température entre 0°C et 110°C.

18. Procédé selon l'une quelconque des revendications 13 à 17, **caractérisé en ce que** les composés de formule (II) sont transformés avec des réactifs métallo-organiques de zinc en présence d'un catalyseur ou avec des sels métalliques.

19. Composés de formule (IVa) 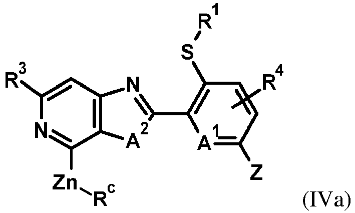 dans laquelle Z, R¹, R³, R⁴, R^{c}, A¹ et A² présentent les significations selon l'une quelconque des revendications 1 à 6.

20. Composés de formule (IVb) 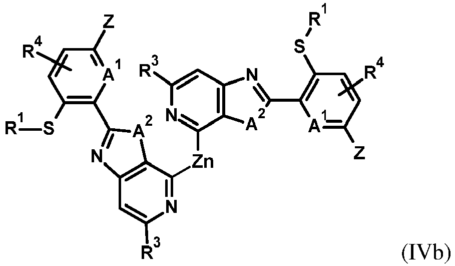 dans laquelle Z, R¹, R³, R⁴, A¹ et A² présentent les significations selon l'une quelconque des revendications 1 à 6.

21. Composés de formule (II) 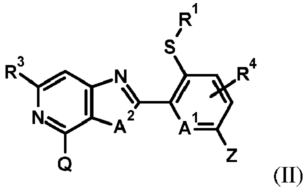 dans laquelle Q, Z, R¹, R³, R⁴, A¹ et A² présentent les significations selon l'une quelconque des revendications 1 à 6.
